# EUROPEAN PATENT APPLICATION

(11) **EP 1 331 010 A1**
(43) Date of publication of application: **30.07.2003**
(21) Application number: 01958447.3
(22) Date of filing: 24.08.2001
(51) Int. Cl.: A61K 45/00, A61K 31/4738, A61K 31/40, A61K 31/55, A61P 25/00

(54) **PREVENTIVES AND REMEDIES FOR CENTRAL NERVOUS SYSTEM DISEASES**

(30) Priority: 25.08.2000 JP 2000255529
(71) Applicant: Takeda Chemical Industries, Ltd., Osaka-shi, Osaka 541-0045 (JP)
(72) Inventor: FUKUMOTO, Hiroaki, Inagawa-cho, Kawabe-gun, Hyogo (JP); MORI, Masaaki, Tsukuba-shi, Ibaraki 305-0821 (JP); MIYAMOTO, Masaomi, Takarazuka-shi, Hyogo 665-0841 (JP)
(74) Representative: Rickard, Timothy Mark Adrian
(86) International application number: JP0107237
(87) International publication number: WO02015934

(57) **Abstract**

A prophylactic or therapeutic agent for central nervous system diseases based on amyloid β40 secretion inhibitory activity of a compound having urotensin II receptor antagonistic activity or a salt thereof.

## Description

### Field of the Invention

The present invention relates to a prophylactic or therapeutic agent for central nervous system diseases and an amyloid β40 secretion inhibitor which comprises as an active component a compound having urotensin II receptor antagonistic activity or a salt thereof.

### Background Art

Urotensin II was discovered as one of peptide hormones having a potent vasoconstrictive activity, and has been proved to have extremely higher vasoconstrictive activity than endothelin, which is the potentest vasoconstrictor among those which are currently known to have vasoconstrictive activity on mammal arteria. The receptor for urotensin II is GPR 14 protein, which is one of orphan receptors [Nature, vol. 401, p. 282 (1999)], its antagonist has not yet been reported.

WO2001/14888 discloses an identification method of a ligand of urotensin II receptor and use of urotensin II receptor agonist and antagonist, but does not suggest amyloid β40 secretion inhibitory activity at all.

JP 3-220189 A discloses a quinoline derivative condensed with pyrrolidine ring or piperidine ring which has acetylcholin esterase inhibitory activity, as a dysmnesia improving agent in senile dementia and Alzheimer's disease, but does not suggest amyloid β40 secretion inhibitory activity at all.

### Objects of the Invention

The present invention provides a prophylactic or therapeutic agent for central nervous system diseases such as neurodegenerative diseases, for example, cerebrovascular amyloid angiopathy, Alzheimer's disease, etc., neuropathy at cerebrovascular disorder, etc., dysmnesia, mental diseases, and the like, which is based on amyloid β40 secretion inhibitory activity.

### Summary of the Invention

The present inventors have found that urotensin II increases the secretion of amyloid β40 for the first time, and have intensively studied compounds having urotensin II receptor antagonistic activity. As a result, the present inventors found that a compound having urotensin II receptor antagonistic activity or a salt thereof suppresses the secretion induction of amyloid β40 by urotensin II. The present invention has been completed based on this finding.

That is, the present invention provides:
(1) A prophylactic or therapeutic agent for central nervous system diseases which comprises a compound having urotensin II receptor antagonistic activity or a salt thereof;
(2) The agent according to the above (1), which is an amyloid β40 secretion inhibitor;
(3) The agent according to the above (1), which is a prophylactic or therapeutic agent for (a) neurodegenerative diseases, (b) neuropathy at cerebrovascular disorder, cephal injury or myelo injury, sequelae of encephalitis or cerebral paralysis, (c) dysmnesia, or (d) mental diseases;
(4) The agent according to the above (1), wherein the compound having urotensin II receptor antagonistic activity or a salt thereof is a non-peptide compound or a salt thereof;
(5) The agent according to the above (1), wherein the compound having urotensin II receptor antagonistic activity is a quinoline derivative;
(6) The agent according to the above (1), wherein the compound having urotensin II receptor antagonistic activity is a 4-aminoquinoline derivative;
(7) The agent according to the above (1), wherein the compound having urotensin II receptor antagonistic activity or a salt thereof is a compound represented by the formula (Ia): 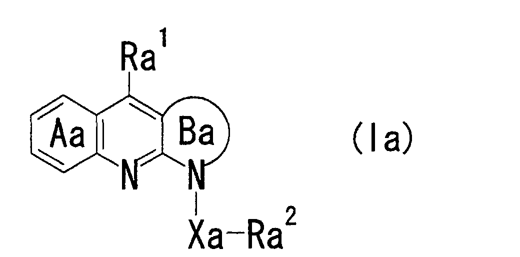 wherein Aa is a benzene ring which may be substituted, Ba is a 5- to 8-membered ring which may be substituted, Xa is a divalent group in which the number of atom(s) in a linear chain portion is 1 to 4, Ra¹ is an amino group which may be substituted, and Ra² is a cyclic group which may be substituted, or a salt thereof;
(8) The agent according to the above (7), wherein Aa is substituted with a hydrocarbon group which may be substituted;
(9) The agent according to the above (7), wherein Aa is substituted with a C₁₋₄ alkyl group which may be substituted;
(10) The agent according to the above (1), wherein the compound having urotensin II receptor antagonistic activity or a salt thereof is a compound represented by the formula (IIa): 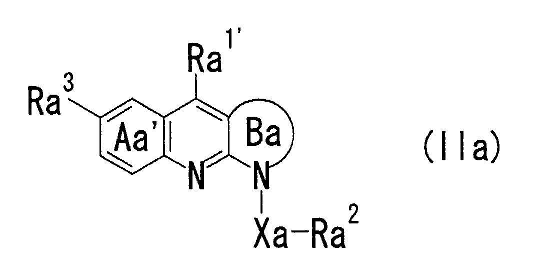 wherein Aa' is a benzene ring which may be further substituted in addition to the substituent Ra³, Ba is a 5-to 8-membered ring which may be substituted, Xa is a divalent group in which the number of atom(s) in a linear chain portion is 1 to 4, Ra^{1'} is a substituted amino group, Ra² is a cyclic group which may be substituted, Ra³ is a hydrocarbon group which may be substituted, a heterocyclic group which may be substituted, nitro group, a halogen atom, amino group which may be substituted, or a group represented by Ra4-Ya- (wherein Ya is oxygen atom or sulfur atom which may be oxidized, and Ra⁴ is a hydrocarbon group which may be substituted, or a heterocyclic group which may be substituted), or a salt thereof;
(11) The agent according to the above (1), wherein the compound having urotensin II receptor antagonistic activity or a salt thereof is a compound represented by the formula (IIa'): 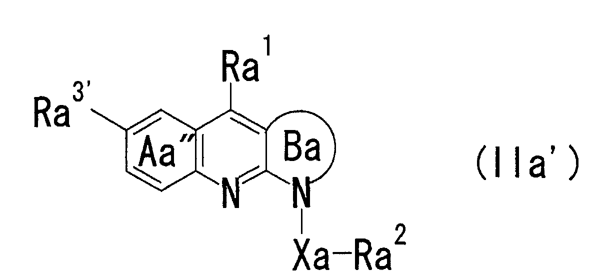 wherein Aa'' is a benzene ring which may be further substituted in addition to the substituent Ra^{3'}, Ba is a 5-to 8-membered ring which may be substituted, Xa is a divalent group in which the number of atom(s) in a linear chain portion is 1 to 4, Ra¹ is an amino group which may be substituted, Ra² is a cyclic group which may be substituted, Ra^{3'} is a hydrocarbon group which may be substituted, a heterocyclic group which may be substituted, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted), or a salt thereof;
(12) The agent according to the above (11), wherein R^{3'} is a hydrocarbon group which may be substituted;
(13) The agent according to the above (12), wherein R^{3'} is alkyl;
(14) The agent according to the above (12), wherein Ra¹ is amino;
(15) The agent according to the above (1), wherein the compound having urotensin II receptor antagonistic activity or a salt thereof is a compound represented by the formula (Ib): 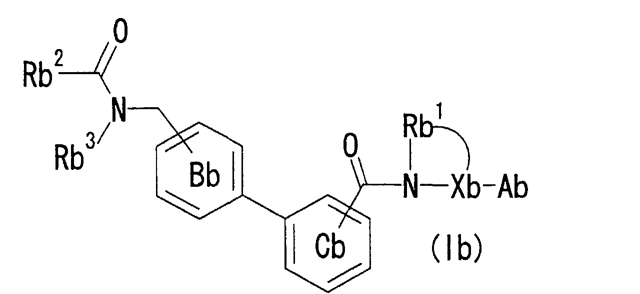 wherein Rb¹ is a hydrogen atom or a hydrocarbon group which may be substituted, Xb is a spacer in which the number of atom(s) constituting a linear chain portion is 1 to 8, Rb¹ and Xb may be bonded to form a ring, Ab is an amino group which may be substituted or a nitrogen-containing heterocyclic group which may be substituted, each of Rb² and Rb³ is a hydrocarbon group which may be substituted, and each of ring Bb and ring Cb is a benzene ring which may be further substituted (provided that 4'-[[(methoxyacetyl)methylamino]methyl]-N-[4-methoxy-3-(4-methyl-1-piperazinyl)phenyl]-2'-methyl-[1,1'-biphenyl]-4-carboxamide is excluded), or a salt thereof;
(16) The agent according to the above (15), wherein Xb is a chain spacer;
(17) The agent according to the above (1), wherein the compound having urotensin II receptor antagonistic activity or a salt thereof is a compound represented by the formula (Ic): 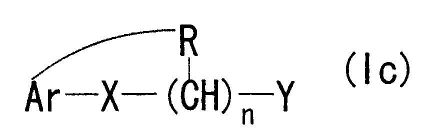 wherein Ar is an aryl group which may be substituted, X is a spacer in which the number of atom(s) constituting a linear chain portion is 1 to 4, n is an integer of 1 to 10, R is a hydrogen atom or a hydrocarbon group which may be substituted, and may be the same or different in the repetition of n, R may be bonded to Ar or the substituent of Ar to form a ring, and Y is an amino group which may be substituted or a nitrogen-containing heterocyclic group which may be substituted, or a salt thereof;
(18) The agent according to the above (17), wherein X is a spacer other than -CO-; and
(19) The agent according to the above (1), wherein the compound having urotensin II receptor antagonistic activity or a salt thereof is a compound represented by the formula (IIc): 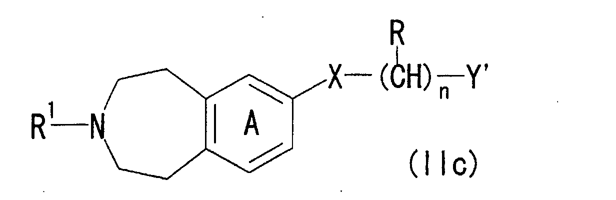 wherein R¹ is a hydrogen atom or a hydrocarbon group which may be substituted or an acyl group which may be substituted, ring A is a benzene ring which may be further substituted, X is a chain spacer in which the number of atom(s) constituting a linear chain portion is 1 to 4 (provided that -CO- is excluded), n is an integer of 1 to 10, R is a hydrogen atom or a hydrocarbon group which may be substituted, and may be the same or different in the repetition of n, R may be bonded to ring A or the substituent of ring A to form a ring, and Y is an amino group which may be substituted, or a salt thereof.
The present invention also provides:
(20) A method for preventing or treating central nervous system diseases in a mammal which comprises administering an effective amount of a compound having urotensin II receptor antagonistic activity or a salt thereof to the mammal in need of the prevention or treatment of central nervous system diseases;
(21) The method according to the above (20), wherein the compound having urotensin II receptor antagonistic activity or a salt thereof is a compound having amyloid β40 secretion inhibitory activity or a salt thereof;
(22) The method according to the above (20), wherein (a) neurodegenerative diseases, (b) neuropathy at cerebrovascular disorder, cephal injury and myelo injury, sequelae of encephalitis or cerebral paralysis, (c) dysmnesia, or (d) mental diseases are prevented or treated;
(23) The method according to the above (20), wherein the compound having urotensin II receptor antagonistic activity or a salt thereof is a non-peptide compound or a salt thereof;
(24) The method according to the above (20), wherein the compound having urotensin II receptor antagonistic activity is a quinoline derivative;
(25) The method according to the above (20), wherein the compound having urotensin II receptor antagonistic activity is a 4-aminoquinoline derivative;
(26) The method according to the above (20), wherein the compound having urotensin II receptor antagonistic activity or a salt thereof is a compound represented by the formula (Ia): 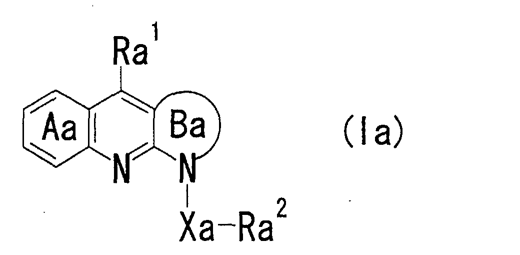 wherein Aa is a benzene ring which may be substituted, Ba is a 5- to 8-membered ring which may be substituted, Xa is a divalent group in which the number of atom(s) in a linear chain portion is 1 to 4, Ra¹ is an amino group which may be substituted, and Ra² is a cyclic group which may be substituted, or a salt thereof;
(27) The method according to the above (26), wherein Aa is substituted with a hydrocarbon group which may be substituted;
(28) The method according to the above (20), wherein the compound having urotensin II receptor antagonistic activity or a salt thereof is a compound represented by the formula (IIa): 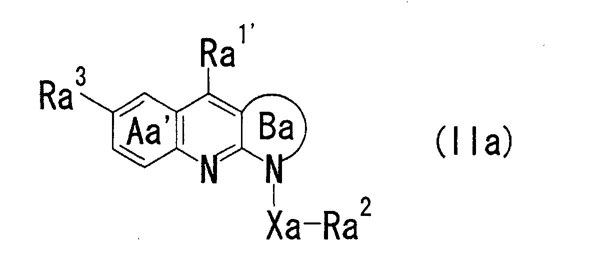 wherein Aa' is a benzene ring which may be further substituted in addition to a substituent Ra³, Ba is a 5- to 8-membered ring which may be substituted, Xa is a divalent group in which the number of atom(s) in a linear chain portion is 1 to 4, Ra^{1'} is a substituted amino group, Ra² is a cyclic group which may be substituted, Ra³ is a hydrocarbon group which may be substituted, a heterocyclic group which may be substituted, nitro group, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted), or a salt thereof;
(29) The method according to the above (20), wherein the compound having urotensin II receptor antagonistic activity or a salt thereof is a compound represented by the formula (IIa'): 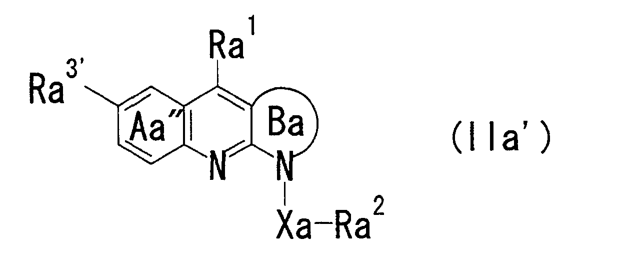 wherein Aa'' is a benzene ring which may be further substituted in addition to the substituent Ra^{3'}, Ba is a 5-to 8-membered ring which may be substituted, Xa is a divalent group in which the number of atom(s) in a linear chain portion is 1 to 4, Ra¹ is an amino group which may be substituted, Ra² is a cyclic group which may be substituted, Ra^{3'} is a hydrocarbon group which may be substituted, a heterocyclic group which may be substituted, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted), or a salt thereof;
(30) The method according to the above (29), wherein R^{3'} is a hydrocarbon group which may be substituted;
(31) The method according to the above (30), wherein R^{3'} is alkyl;
(32) The method according to the above (30), wherein Ra¹ is amino.
(33) The method according to the above (20), wherein the compound having urotensin II receptor antagonistic activity or a salt thereof is a compound represented by the formula (Ib): 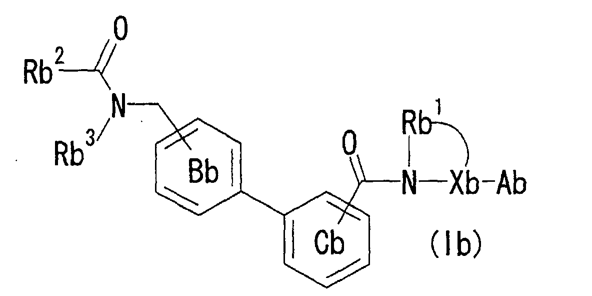 wherein Rb¹ is a hydrogen atom or a hydrocarbon group which may be substituted, Xb is a spacer in which the number of atom(s) constituting a linear chain portion is 1 to 8, Rb¹ and Xb may be bonded to form a ring, Ab is an amino group which may be substituted or a nitrogen-containing heterocyclic group which may be substituted, each of Rb² and Rb³ is a hydrocarbon group which may be substituted, and each of ring Bb and ring Cb is a benzene ring which may be further substituted (provided that 4'-[[(methoxyacetyl)methylamino]methyl]-N-[4-methoxy-3-(4-methyl-1-piperazinyl)phenyl]-2'-methyl-[1,1'-biphenyl]-4-carboxamide is excluded)], or a salt thereof;
(34) The method according to the above (33), wherein Xb is a chain spacer;
(35) The method according to the above (20), wherein the compound having urotensin II receptor antagonistic activity or a salt thereof is a compound represented by the formula (Ic): 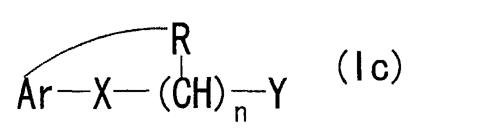 wherein Ar is an aryl group which may be substituted, X is a spacer in which the number of atom(s) constituting a linear chain portion is 1 to 4, n is integer of 1 to 10, R is a hydrogen atom or a hydrocarbon group which may be substituted, and may be the same or different in the repetition of n, R may be bonded to Ar or the substituent of Ar to form a ring, and Y is an amino group which may be substituted or a nitrogen-containing heterocyclic group which may be substituted, or a salt thereof;
(36) The method according to the above (35), wherein X is a spacer other than -CO-; and
(37) The method according to the above (20), wherein the compound having urotensin II receptor antagonistic activity or a salt thereof is a compound represented by the formula (IIc): 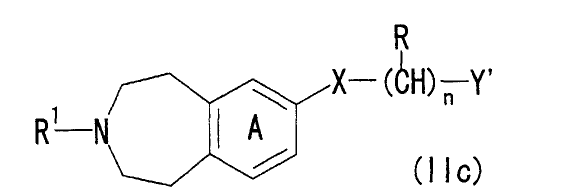 wherein R¹ is a hydrogen atom or a hydrocarbon group which may be substituted or an acyl group which may be substituted, ring A indicates benzene ring which may be further substituted, X is a chain spacer in which the atomic number constituting a linear chain portion is 1 to 4 (provided that -CO- is excluded), n is an integer of 1 to 10, R is a hydrogen atom or a hydrocarbon group which may be substituted, and may be the same or different in the repetition of n, R may be bonded to ring A or the substituent of ring A to form a ring, and Y is an amino group which may be substituted, or a salt thereof.
Further, the present invention provides:
(38) Use of a compound having urotensin II receptor antagonistic activity or a salt thereof for manufacturing a prophylactic or therapeutic agent for central nervous system diseases;
(39) The use according to the above (38), wherein the compound having urotensin II receptor antagonistic activity or a salt thereof is a compound having amyloid β40 secretion inhibitory activity or a salt thereof;
(40) The use according to the above (38) for manufacturing a prophylactic or therapeutic agent of (a) neurodegenerative diseases, (b) neuropathy at cerebrovascular disorder, cephal injury and myelo injury, sequelae of encephalitis or cerebral paralysis, (c) dysmnesia, or (d) mental diseases;
(41) The use according to the above (38), wherein the compound having urotensin II receptor antagonistic activity or a salt thereof is a non-peptide compound or a salt thereof;
(42) The use according to the above (38), wherein the compound having urotensin II receptor antagonistic activity is a quinoline derivative;
(43) The use according to the above (38), wherein the compound having urotensin II receptor antagonistic activity is a 4-aminoquinoline derivative;
(44) The use according to the above (38), wherein the compound having urotensin II receptor antagonistic activity or a salt thereof is a compound represented by the formula (Ia): 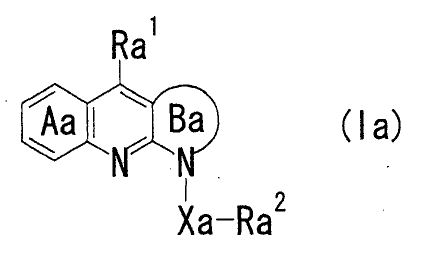 wherein Aa is a benzene ring which may be substituted, Ba is a 5- to 8-membered ring which may be substituted, Xa is a divalent group in which the number of atom(s) in a linear chain portion is 1 to 4, Ra¹ is an amino group which may be substituted, and Ra² is a cyclic group which may be substituted, or a salt thereof;
(45) The use according to the above (44), wherein Aa is substituted with a hydrocarbon group which may be substituted;
(46) The use according to the above (38), wherein the compound having urotensin II receptor antagonistic activity or a salt thereof is a compound represented by the formula (IIa): 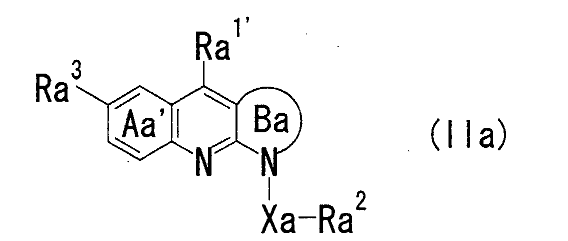 wherein Aa' is a benzene ring which may be further substituted in addition to the substituent Ra³, Ba is a 5-to 8-membered ring which may be substituted, Xa is a divalent group in which the number of atom(s) in a linear chain portion is 1 to 4, Ra^{1'} is a substituted amino group, Ra² is a cyclic group which may be substituted, Ra³ is a hydrocarbon group which may be substituted, a heterocyclic group which may be substituted, nitro group, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted), or a salt thereof;
(47) The use according to the above (38), wherein the compound having urotensin II receptor antagonistic activity or a salt thereof is a compound represented by the formula (IIa'): 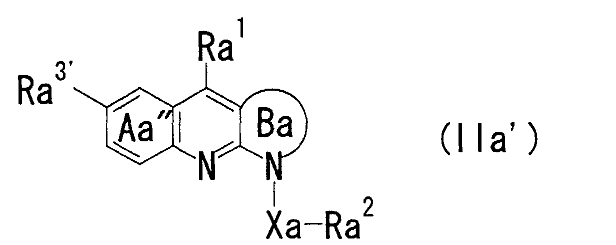 wherein Aa'' is a benzene ring which may be further substituted in addition to the substituent Ra^{3'}, Ba is a 5-to 8-membered ring which may be substituted, Xa is a divalent group in which the number of atom(s) in a linear chain portion is 1 to 4, Ra¹ is an amino group which may be substituted, Ra² is a cyclic group which may be substituted, Ra^{3'} is a hydrocarbon group which may be substituted, a heterocyclic group which may be substituted, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted), or a salt thereof;
(48) The use according to the above (47), wherein R^{3'} is a hydrocarbon group which may be substituted;
(49) The use according to the above (47), wherein R^{3'} is alkyl;
(50) The use according to the above (47), wherein Ra¹ is amino;
(51) The use according to the above (38), wherein the compound having urotensin II receptor antagonistic activity or a salt thereof is a compound represented by the formula (Ib): 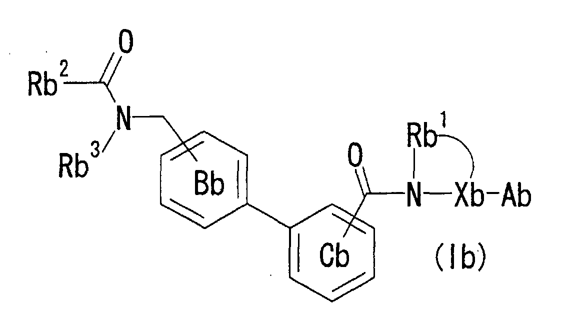 wherein Rb¹ is a hydrogen atom or a hydrocarbon group which may be substituted, Xb is a spacer in which the number of atom(s) constituting a linear chain portion is 1 to 8, Rb¹ and Xb may be bonded to form a ring, Ab is an amino group which may be substituted or a nitrogen-containing heterocyclic group which may be substituted, each of Rb² and Rb³ is a hydrocarbon group which may be substituted, and each of ring Bb and ring Cb is a benzene ring which may be further substituted, (provided that 4'-[(methoxyacetyl)methylamino]methyl]-N-[4-methoxy-3-(4-methyl-1-piperazinyl)phenyl]-2'-methyl-[1,1'-biphenyl]-4-carboxamide is excluded), or a salt thereof;
(52) The use according to the above (51), wherein Xb is a chain spacer;
(53) The use according to the above (38), wherein the compound having urotensin II receptor antagonistic activity or a salt thereof is a compound represented by the formula (Ic): 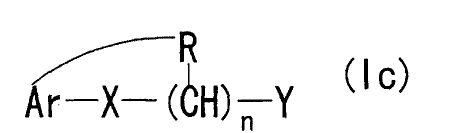 wherein Ar is an aryl group which may be substituted, X is a spacer in which the number of atom(s) constituting a linear chain portion is 1 to 4, n indicates an integer of 1 to 10, R is a hydrogen atom or a hydrocarbon group which may be substituted, and may be the same or different in the repetition of n, R may be bonded to Ar or the substituent of Ar to form a ring, and Y is an amino group which may be substituted or a nitrogen-containing heterocyclic group which may be substituted, or a salt thereof;
(54) The use according to the above (53), wherein X is a spacer other than -CO-; and
(55) The use according to the above (38), wherein the compound having urotensin II receptor antagonistic activity or a salt thereof is a compound represented by the formula (IIc): 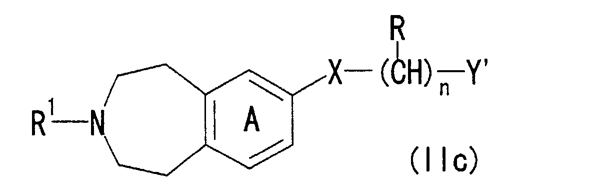 wherein R¹ is a hydrogen atom or a hydrocarbon group which may be substituted or an acyl group which may be substituted, ring A is a benzene ring which may be further substituted, X is a chain spacer in which the number of atom(s) constituting a linear chain portion is 1 to 4 (provided that -CO- is excluded), n indicates an integer of 1 to 10, R is a hydrogen atom or a hydrocarbon group which may be substituted, and may be the same or different in the repetition of n, R may be bonded to ring A or the substituent of ring A to form a ring, and Y is an amino group which may be substituted, or a salt thereof.

### Brief Description of Drawings

Fig. 1 illustrates the inhibitory effect of test compound 1 on suppression of increase of amyloid β40 induced by urotensin II.
Fig. 2 illustrates the inhibitory effect of test compound 2 on suppression of increase of amyloid β40 induced by urotensin II.

### Detailed Description of the Invention

"Urotensin II receptor antagonistic activity" in the present invention means an activity of competitively or non-competitively inhibiting the coupling of urotensin II to a urotensin II receptor on a cell membrane.

In the present invention, "the compound having urotensin II receptor antagonistic activity or a salt thereof" can be used for a prophylactic or therapeutic agent for various central nervous system diseases based on amyloid β40 secretion inhibitory activity. Particularly, it is preferably used as a prophylactic or therapeutic agent for (1) cerebrovascular amyloid angiopathy which is mainly caused by the deposition of amyloid β40, (2) neurodegenerative diseases, (3) neuropathy at cerebrovascular disorder, cephal injury and myelo injury, sequelae of encephalitis or cerebral paralysis, (4) dysmnesia, (5) mental diseases (for example, depression, anxiety disorder, panic disorder, shizophrenia, and the like), or the like.

As the compound having urotensin II receptor antagonistic activity or a salt thereof used in the present invention, a non-peptide compound having urotensin II receptor antagonistic activity or a salt thereof which has an advantage of long duration of action is preferred. In particular, a quinoline derivative is preferred, and a 4-aminoquinoline derivative is preferably used.

As the compound having urotensin II receptor antagonistic activity or a salt thereof used in the present invention, preferably, among others, there are a compound represented by the formula (Ia), 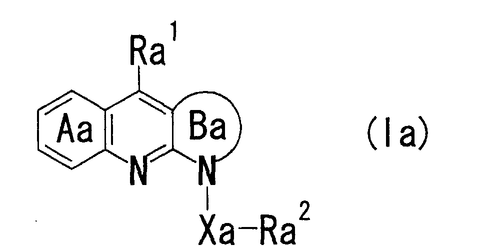 wherein Aa is a benzene ring which may be substituted, Ba is a 5- to 8-membered ring which may be substituted, Xa is a divalent group in which the number of atom(s) in a linear chain portion is 1 to 4, Ra¹ is an amino group which may be substituted, and Ra² is a cyclic group which may be substituted, or a salt thereof; a compound represented by the formula (Ib), 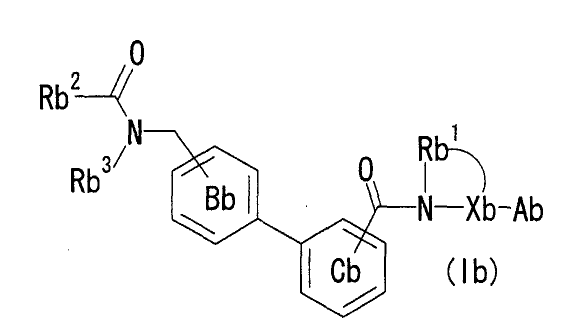 wherein Rb¹ is a hydrogen atom or a hydrocarbon group which may be substituted, Xb is a spacer in which the number of atom(s) constituting a linear chain portion is 1 to 8, Rb¹ and Xb may be bonded to form a ring, Ab is an amino group which may be substituted or a nitrogen-containing heterocyclic group which may be substituted, each of Rb² and Rb³ is a hydrocarbon group which may be substituted, and each of ring Bb and ring Cb is a benzene ring which may be further substituted, (provided that 4'-[[(methoxyacetyl)methylamino]methyl]-N-[4-methoxy-3-(4-methyl-1-piperazinyl)phenyl]-2'-methyl-[1,1'-biphenyl]-4-carboxamide is excluded)], or a salt thereof; and a compound represented by the formula (Ic); 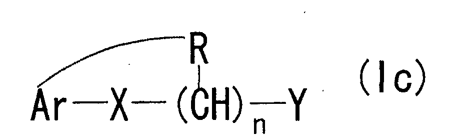 wherein Ar is an aryl group which may be substituted, X is a spacer in which the number of atom(s) constituting a linear chain portion is 1 to 4, n is an integer of 1 to 10, R is a hydrogen atom or a hydrocarbon group which may be substituted, and may be the same or different in the repetition of n, R may be bonded with Ar or the substituent of Ar to form a ring, and Y is an amino group which may be substituted or a nitrogen-containing heterocyclic group which may be substituted, or a salt thereof, and the like.

In the above formula, examples of the substituent of the benzene ring in the "benzene ring which may be substituted" represented by Aa include a hydrocarbon group which may be substituted, a heterocyclic group which may be substituted, nitro group, a halogen atom, amino group which may be substituted, a group represented by Ra⁴-Ya- (wherein Ya is oxygen atom or sulfur atom which may be oxidized, and Ra⁴ is a hydrocarbon group which may be substituted, or a heterocyclic group which may be substituted), cyano group, acyl group which may be substituted, carboxyl group which may be esterified or amidated, and the like.

Examples of the "hydrocarbon group" in the "hydrocarbon group which may be substituted" as the substituent of the benzene ring in the "benzene ring which may be substituted" represented by Aa, and in the "hydrocarbon group which may be substituted" represented by Ra⁴ include:
(1) alkyl (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, and the like, preferably lower (C₁₋₆) alkyl and the like);
(2) cycloalkyl (for example, C₃₋₈ cycloalkyl such as cyclopropyl, cyclobutyl, cyclopentyl, cyclohexyl, cycloheptyl, and the like); further, said cycloalkyl may be condensed with benzene ring to form indan (for example, indan-1-yl, indan-2-yl, and the like), tetrahydronaphthalene (for example, tetrahydronaphthalen-5-yl, tetrahydronaphthalen-6-yl, and the like), and the like (preferably, indan, and the like); further, said cycloalkyl may be crosslinked through a linear atomic chain having 1 to 2 carbons to form a crosslinked cyclic hydrocarbon residual group such as bicyclo[2.2.1]heptyl, bicyclo[2.2.2]octyl, bicyclo[3.2.1]octyl, bicyclo[3.2.2]nonyl, and the like (preferably, cyclohexyl having crosslink through a linear atomic chain having 1 to 2 carbons, and the like, and further preferably, bicyclo[2.2.1]heptyl, and the like);
(3) alkenyl (for example, C₂₋₁₀ alkenyl such as vinyl, allyl, crotyl, 2-pentenyl, 3-hexenyl, and the like, preferably lower (C₂₋₆) alkenyl and the like);
(4) cycloalkenyl (for example, C₃₋₈ cycloalkenyl such as 2-cyclopentenyl, 2-cyclohexenyl, 2-cyclopentenylmethyl, 2-cyclohexenylmethyl, and the like);
(5) alkynyl (for example, C₂₋₁₀ alkynyl such as ethynyl, 1-propynyl, 2-propynyl, 1-butynyl, 2-pentynyl, 3-hexynyl, and the like, preferably lower (C₂₋₆) alkynyl, and the like);
(6) aryl (for example, C₆₋₁₄ aryl such as phenyl, naphthyl, and the like, preferably C₆₋₁₀ aryl, more preferably phenyl, and the like);
(7) aralkyl (for example, C₁₋₆ alkyl having 1 to 3 C₆₋₁₄ aryls, preferably phenyl-C₁₋₄ alkyl (for example, benzyl, phenethyl and the like), and the like. Among these, alkyl is preferred, C₁₋₄ alkyl such as methyl and ethyl are more preferred and, in particular, methyl is preferably used. Said hydrocarbon group may be substituted, and examples of the substituent include halogen (for example, fluorine, chlorine, bromine, iodine and the like), nitro, cyano, hydroxy group, thiol group which may be substituted (for example, thiol, C₁₋₄ alkylthio and the like), amino group which may be substituted (for example, amino, mono-C₁₋₄ alkylamino, di-C₁₋₄ alkylamino, mono-C₂₋₅ alkanoylamino, 5- to 6-membered cyclic amino such as tetrahydropyrrole, piperazine, piperidine, morpholine, thiomorpholine, pyrrole, imidazole, and the like), phenyl-lower (C₁₋₄) alkyl, C₃₋₇ cycloalkyl, carboxyl group which may be esterified or amidated (for example, carboxyl, C₁₋₄ alkoxy-carbonyl, lower (C₇₋₁₀) aralkyloxy-carbonyl, carbamoyl, mono-C₁₋₄ alkylcarbamoyl, di-C₁₋₄ alkylcarbamoyl, and the like), C₁₋₄ alkyl which may be substituted with a halogen atom or C₁₋₄ alkoxy (for example, trifluoromethyl, methyl, ethyl, and the like), C₁₋₄ alkoxy which may be substituted with a halogen atom or C₁₋₄ alkoxy (for example, methoxy, ethoxy, trifluoromethoxy, trifluoroethoxy, and the like), C₁₋₄ alkylenedioxy (for example, -O-CH₂-O-, -O-CH₂-CH₂-O-, and the like), formyl, C₂₋₄ alkanoyl (for example, acetyl, propionyl, and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), and the like, and the number of the substituent(s) is preferably 1 to 3.

Examples of the "heterocyclic group" in the "heterocyclic group which may be substituted" as the substituent of the benzene in the "benzene ring which may be substituted" represented by Aa, and in the "heterocyclic group which may be substituted" represented by Ra⁴ include a group formed by eliminating one hydrogen atom from a 5-to 8-membered aromatic heterocyclic ring which contains at least one (preferably, 1 to 4, and more preferably, 1 to 2) hetero atoms of 1 to 3 kinds (preferably, 1 to 2 kinds) selected from oxygen atom, sulfur atom, nitrogen atom, and the like; a saturated or unsaturated non-aromatic heterocyclic ring (aliphatic heterocyclic ring); and the like.

Examples of the "aromatic heterocyclic ring" used herein include a 5- to 8-membered (preferably, 5- to 6-membered) aromatic monocyclic heterocyclic ring (for example, furan, thiophene, pyrrole, oxazole, isoxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, and the like), and the like. Examples of the "non-aromatic heterocyclic ring" include a 5- to 8-membered (preferably, 5- to 6-membered) saturated or unsaturated monocyclic non-aromatic heterocyclic ring (aliphatic heterocyclic ring) such as pyrrolidine, tetrahydrofuran, tetrahydrothiophene, thiolane, dithiolane, oxathiolane, pyrroline, imidazolidine, imidazoline, pyrazolidine, pyrazoline, oxazine, oxadiazine, thiazine, thiadiazine, piperidine, morpholine, thiomorpholine, tetrahydropyran, piperazine, pyran, oxepine, thiepin, azepine, and the like; a 5- to 8-membered non-aromatic heterocyclic ring in which a part or all of double bonds of the above aromatic monocyclic heterocyclic ring are saturated; and the like.

Further, examples of the "heterocyclic group" in the "heterocyclic group which may be substituted" as the substituent of the benzene ring in the "benzene ring which may be substituted" represented by Aa, and in the "heterocyclic group which may be substituted" represented by Ra⁴ include a group formed by eliminating one hydrogen atom from a condensed ring which is formed by condensing 2 to 3 rings (preferably, 2) selected from the above monocyclic heterocyclic rings (a monocyclic aromatic heterocyclic ring and a monocyclic non-aromatic heterocyclic ring) and 5- to 8-membered cyclic hydrocarbons (5- to 8-membered (preferably, 5- to 6-membered) saturated or unsaturated alicyclic hydrocarbons such as C₅₋₈ cycloalkane, C₅₋₈ cycloalkene and C₅₋₈ cycloalkadiene; 6-membered aromatic hydrocarbons such as benzene; and the like), and the like. These condensed rings may be any one of a saturated condensed ring, a condensed ring partially having unsaturated bond(s), and an aromatic condensed ring. Preferred examples of such condensed ring include a ring in which the same or different two heterocyclic rings (preferably, one heterocyclic ring and one aromatic heterocyclic ring, and more preferably, the same or different two aromatic heterocyclic rings) are condensed; one heterocyclic ring and one homocyclic ring (preferably, one heterocyclic ring and one benzene ring, and more preferably, one aromatic heterocyclic ring and one benzene ring) are condensed; and the like. Specific examples of the condensed ring include indole, benzothiophene, benzofuran, benzimidazole, imidazo[1,2-a]pyridine, quinoline, isoquinoline, cinnoline, and the like.

The "heterocyclic group" in the "heterocyclic group which may be substituted" as the substituent of the benzene ring in the "benzene ring which may be substituted" represented by Aa, and in the "heterocyclic group which may be substituted" represented by Ra⁴ may be substituted, and examples of the substituent include those similar to the substituent of the "hydrocarbon group which may be substituted" as the substituent of the benzene ring in the "benzene ring which may be substituted" represented by Aa.

Examples of the "halogen atom" as the substituent of the benzene ring in the "benzene ring which may be substituted" represented by Aa include fluorine, chlorine, bromine, iodine, and the like.

Examples of the "amino group which may be substituted" as the substituent of the benzene ring in the "benzene ring which may be substituted" represented by Aa include those similar to the "amino group which may be substituted" represented by Ra¹ described hereinafter. Among these, preferred examples thereof include amino group which may have one or two substituents selected from the "hydrocarbon group which may be substituted" (that similar to the above "hydrocarbon group which may be substituted" as the substituent of the benzene ring in the "benzene ring which may be substituted" represented by Aa), the "heterocyclic group which may be substituted" (that similar group to the above "heterocyclic group which may be substituted" as the substituent of the benzene ring in the "benzene ring which may be substituted" represented by Aa), and the "acyl group which may be substituted" (that similar to the "acyl group which may be substituted" as the substituent of the benzene ring in the "benzene ring which may be substituted" represented by Aa hereinafter). Among these, preferred are amino group which may have 1 to 2 alkyls [for example, C₁₋₁₀ alkyls such as methyl, ethyl, propyl, isopropyl, butyl, isobutyl, sec-butyl, tert-butyl, pentyl, isopentyl, neopentyl, hexyl, heptyl, octyl, nonyl, decyl, and the like, preferably lower (C₁₋₆) alkyl, and the like, which may have 1 to 3 substituents selected from, for example, halogen (for example, fluorine, chlorine, bromine, iodine, and the like), nitro, cyano, hydroxy group, thiol group which may be substituted (for example, thiol, C₁₋₄ alkylthio, and the like), amino group which may be substituted (for example, amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, and the like), phenyl-lower (C₁₋₄) alkyl, C₃₋₇ cycloalkyl, carboxyl group which may be esterified or amidated (for example, carboxyl, C₁₋₄ alkoxy-carbonyl, lower (C₇₋₁₀) aralkyloxy-carbonyl, carbamoyl, mono-C₁₋₄ alkyl-carbamoyl, di-C₁₋₄ alkyl-carbamoyl, and the like), C₁₋₄ alkyl which may be substituted with halogen atom or C₁₋₄ alkoxy (for example, trifluoromethyl, methyl, ethyl, and the like), C₁₋₄ alkoxy which may be substituted with halogen atom or C₁₋₄ alkoxy (for example, methoxy, ethoxy, trifluoromethoxy, trifluoroethoxy, and the like), C₁₋₄ alkylenedioxy (for example, -O-CH₂-O-, -O-CH₂-CH₂-O-, and the like), formyl, C₂₋ ₄ alkanoyl (for example, acetyl, propionyl, and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), and the like)].

Further, the substituents of the "amino group which may be substituted" as the substituent of the benzene ring in the "benzene ring which may be substituted" represented by Aa may be bonded to each other to form a cyclic amino group (for example, a cyclic amino group formed by eliminating one hydrogen atom from the ring-constituting nitrogen atom of a 5- to 6-membered ring such as tetrahydropyrrole, piperazine, piperidine, morpholine, thiomorpholine, pyrrole, imidazole, and the like, and has a bonding hand on the nitrogen atom, and the like). Said cyclic amino group may be substituted, and examples of the substituent include halogen (for example, fluorine, chlorine, bromine, iodine, and the like), nitro, cyano, hydroxy group, thiol group, amino group, carboxyl group, C₁₋₄ alkyl which may be halogenated (for example, trifluoromethyl, methyl, ethyl, and the like), C₁₋₄ alkoxy which may be halogenated (for example, methoxy, ethoxy, propoxy, butoxy, trifluoromethoxy, trifluoroethoxy, and the like), formyl, C₂₋₄ alkanoyl (for example, acetyl, propionyl, and the like), C₁₋₄ alkylsulfonyl (for example, methanesulfonyl, ethanesulfonyl, and the like), and the like. The number of the substituent(s) is preferably 1 to 3.

Examples of the "acyl group which may be substituted" as the substituent of the benzene ring in the "benzene ring which may be substituted" represented by Aa include those obtained by bonding hydrogen, the "hydrocarbon group which may be substituted" (that similar to the above "hydrocarbon group which may be substituted" as the substituent of the benzene ring in the "benzene ring which may be substituted" represented by Aa), the "heterocyclic group which may be substituted" (that similar to the above "heterocyclic group which may be substituted" as the substituent of the benzene ring in the "benzene ring which may be substituted" represented Aa), and the like, with carbonyl group, sulfonyl group, or the like. Preferred examples thereof includes those obtained by bonding:
(1) hydrogen,
(2) alkyl which may be substituted, for example, (C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, and the like, preferably lower (C₁₋₆) alkyl, and the like),
(3) cycloalkyl which may be substituted (for example, C₃₋₇ cycloalkyl such as cyclopropyl, cyclobutyl, cyclopentyl, cyclohexyl, cycloheptyl, and the like),
(4) alkenyl which may be substituted (for example, C₂₋₁₀ alkenyl such as allyl, crotyl, 2-pentenyl, 3-hexenyl, and the like, preferably lower (C₂₋₆) alkenyl, and the like);
(5) cycloalkenyl which may be substituted (for example, C₃₋₇ cycloalkenyl such as 2-cyclopentenyl, 2-cyclohexenyl, 2-cyclopentenylmethyl, 2-cyclohexenylmethyl, and the like);
(6) 5- to 6-membered monocyclic aromatic group which may be substituted (for example, phenyl, pyridyl, and the like), and the like,
   with carbonyl group or sulfonyl group (for example, acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl, and the like). Examples of the substituent of the above (2) alkyl which may be substituted, (3) cycloalkyl which may be substituted, (4) alkenyl which may be substituted, (5) cycloalkenyl which may be substituted, and (6) 5- to 6-membered monocyclic aromatic group which may be substituted include halogen (for example, fluorine, chlorine, bromine, iodine, and the like), nitro, cyano, hydroxy group, thiol group which may be substituted (for example, thiol, C₁₋₄ alkylthio, and the like), amino group which may be substituted (for example, amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, and imidazole, and the like), carboxyl group which may be esterified or amidated (for example, carboxyl, C₁₋₄ alkoxy-carbonyl, carbamoyl, mono-C₁₋₄ alkyl-carbamoyl, di-C₁₋₄ alkyl-carbamoyl, and the like), C₁₋₄ alkyl which may be substituted with a halogen atom or C₁₋₄ alkoxy (for example, trifluoromethyl, methyl, ethyl, and the like), C₁₋₄ alkoxy which may be substituted with a halogen atom or C₁₋₄ alkoxy (for example, methoxy, ethoxy, trifluoromethoxy, trifluoroethoxy, and the like), formyl, C₂₋₄ alkanoyl (for example, acetyl, propionyl, and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), and the like, and the number of the substituent(s) is preferably 1 to 3.

Examples of the "carboxyl group which may be esterified" as the substituent of the benzene ring in the "benzene ring which may be substituted" represented by Aa include those obtained by bonding hydrogen, the "hydrocarbon group which may be substituted" (that similar to the above "hydrocarbon group which may be substituted" as the substituent of the benzene ring in the "benzene ring which may be substituted" represented by Aa), and the like, with carbonyloxy group. Preferred examples include those obtained by bonding:
(1) hydrogen,
(2) alkyl which may be substituted (C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, and the like, preferably lower (C₁₋₆) alkyl, and the like),
(3) cycloalkyl which may be substituted (for example, C₃₋₇ cycloalkyl such as cyclopropyl, cyclobutyl, cyclopentyl, cyclohexyl, cycloheptyl, and the like),
(4) alkenyl which may be substituted (for example, C₂₋₁₀ alkenyl such as allyl, crotyl, 2-pentenyl, 3-hexenyl, and the like, preferably lower (C₂₋₆) alkenyl, and the like),
(5) cycloalkenyl which may be substituted (for example, C₃₋₇ cycloalkenyl such as 2-cyclopentenyl, 2-cyclohexenyl, 2-cyclopentenylmethyl, 2-cyclohexenylmethyl, and the like),
(6) aryl which may be substituted (for example, phenyl, naphthyl, and the like),
   with carbonyloxy group. Carboxyl, lower (C₁₋₆) alkoxycarbonyl, aryloxycarbonyl (for example, methoxycarbonyl, ethoxycarbonyl, propoxycarbonyl, phenoxycarbonyl, naphthoxycarbonyl, and the like), and the like are more preferable. Examples of the substituent of the above (2) alkyl which may be substituted, (3) cycloalkyl which may be substituted, (4) alkenyl which may be substituted, (5) cycloalkenyl which may be substituted, and (6) aryl which may be substituted include halogen (for example, fluorine, chlorine, bromine, iodine, and the like), nitro, cyano, hydroxy group, thiol group which may be substituted (for example, thiol, C₁₋₄ alkylthio, and the like), amino group which may be substituted (for example, amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, and the like), carboxyl group which may be esterified or amidated (for example, carboxyl, C₁₋₄ alkoxy-carbonyl, carbamoyl, mono-C₁₋₄ alkyl-carbamoyl, di-C₁₋₄ alkyl-carbamoyl, and the like), C₁₋₄ alkyl which may be substituted with a halogen atom or C₁₋₄ alkoxy (for example, trifluoromethyl, methyl, ethyl, and the like), C₁₋₄ alkoxy which may be substituted with a halogen atom or C₁₋₄ alkoxy (for example, methoxy, ethoxy, trifluoromethoxy, trifluoroethoxy, and the like), formyl, C₂₋₄ alkanoyl (for example, acetyl, propionyl, and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), and the like, and the number of the substituent(s) is preferably 1 to 3.

Examples of the "carboxyl group which may be amidated" as the substituent of the benzene ring in the "benzene ring which may be substituted" represented by Aa include those obtained by bonding:
(1) hydrogen,
(2) the "amino group which may be substituted" (that similar to the above "amino group which may be substituted" as the substituent of the benzene ring in the "benzene ring which may be substituted" represented by A), and the like, with carbonyl group.

The substituent of the benzene ring in the "benzene ring which may be substituted" represented by Aa may be one to four (preferably, 1 to 2) the same or different substituents at any possible position(s) of the ring. Further, when the benzene ring in "the benzene ring which may be substituted" represented by Aa has two or more substituents, two substituents may be bonded to each other to form, for example, lower (C₁₋₆) alkylene (for example, trimethylene, tetramethylene, and the like), lower (C₁₋₆) alkyleneoxy (for example, -O-CH₂-O-, -O-CH₂-CH₂-, and the like), lower (C₁₋₆) alkylenedioxy (for example, -O-CH₂-O-, -O-CH₂-CH₂-O-, and the like) , lower (C₂₋₆) alkenylene (for example, -CH₂-CH=CH-, -CH₂-CH₂-CH=CH-, -CH₂-CH=CH-CH₂-, and the like), lower (C₄₋₆) alkadienylene (for example, -CH=CH-CH=CH-, and the like), and the like.

Preferred examples of the substituent of the benzene ring in the "benzene ring which may be substituted" represented by Aa include a hydrocarbon group which may be substituted, a heterocyclic group which may be substituted, nitro group, a halogen atom, amino group which may be substituted, a group represented by Ra⁴-Ya- (wherein Ya is oxygen atom or sulfur atom which may be oxidized, and Ra⁴ is a hydrocarbon group which may be substituted, or a heterocyclic group which may be substituted), and the like, and more preferably, it is a hydrocarbon group which may be substituted, a heterocyclic group which may be substituted, halogen atom, amino group which may be substituted, a group represented by Ra4-Ya- (wherein Ya is oxygen atom or sulfur atom which may be oxidized, and Ra⁴ is a hydrocarbon group which may be substituted, or a heterocyclic group which may be substituted), and the like. Among these, lower (C₁₋₄) alkyl, a halogen atom, and the like are preferred. In particular, (C₁₋₄) alkyl is preferred.

Further, preferred examples of the "benzene ring which may be substituted" represented by Aa include benzene ring having at least one substituent at the position of "a" on the benzene ring represented by the formula:

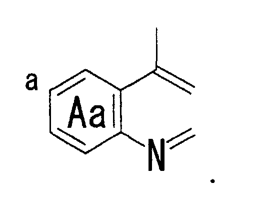

Among these, benzene ring represented by the formula: 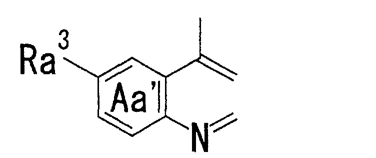 wherein Aa' is a benzene ring which may be further substituted in addition to the substituent Ra³, Ra³ is a hydrocarbon group which may be substituted, a heterocyclic group which may be substituted, nitro group, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted), is preferred.

In particular, benzene ring represented by the formula: 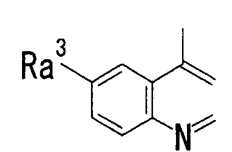 wherein Ra³ is as defined above, is preferred. Preferred examples of Ra³ in the above formula include a hydrocarbon group which may be substituted, a heterocyclic group which may be substituted, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted). Among these, a hydrocarbon group which may be substituted, a heterocyclic group which may be substituted, a halogen atom, and the like are preferred, and in particular, a lower alkyl group which may be substituted or a halogen atom is preferred.

Examples of the "5- to 8-membered ring which may be substituted" represented by Ba in the above formula include a saturated 5- to 8-membered ring represented by the formula: 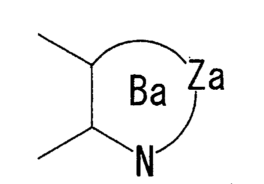 wherein Za is a saturated divalent group in which the ring Ba can form a saturated 5- to 8-membered ring which may be substituted, and may be substituted at any possible position, and the like. The saturated 5- to 8-membered ring may have partially unsaturated bond(s) and may further form an aromatic ring. As ring Ba, a saturated 5- to 8-membered ring which may be substituted is preferred.

In this case, examples of the "saturated 5- to 8-membered ring" in the "saturated 5- to 8-membered ring which may be substituted" as ring Ba means a "5- to 8-membered ring in which all of the bonds constituting ring Ba other than the double bond at the site where the ring Ba and the quinoline ring form the condensed ring are saturated single bonds (single bonds)", and the "unsaturated 5- to 8-membered ring" in the "unsaturated 5-to 8-membered ring which may be substituted" as ring Ba means a "5- to 8-membered ring in which at least one of the bonds constituting ring Ba other than the double bond at a site where the ring Ba and the quinoline ring form the condensed ring is an unsaturated bond".

The saturated divalent group represented by Za in the above formula may be any one of those in which ring Ba can form a saturated 5- to 8-membered ring which may be substituted. Namely, Za may be any one so far as it is a saturated divalent group in which the number of atom(s) in a linear chain portion is 2 to 5 (preferably, a saturated divalent hydrocarbon group in which the number of atom(s) in a linear chain portion is 2 to 5). Specific examples include:
(1) -(CH₂)ₐ₁- (a1 is an integer of 2 to 5),
(2) -(CH₂)_{b1}-Z¹-(CH₂)_{b2}- (b1 and b2 are the same or different and are an integer of 0 to 4, provided that the sum of b1 and b2 is 1 to 4. Z¹ is NH, O, S, SO or SO₂),
(3) -(CH₂)_{d1}-Z¹-(CH₂)_{d2}- Z²-(CH₂)_{d3}- (d1, d2 and d3 are the same or different and are an integer of 0 to 3, provided that the sum of d1, d2 and d3 is 0 to 3. Each of Z¹ and Z² is NH, O, S, SO or SO₂),
(4) -(CH₂)ₑ₁-Z¹-(CH₂)ₑ₂- Z²-(CH₂)ₑ₃- Z³-(CH₂)ₑ₄- (e1, e2, e3 and e4 are the same or different and are an integer of 0 to 2, provided that the sum of d1, d2, d3 is 0 to 2. Each of Z¹, Z² and Z³ is NH, O, S, SO or SO₂) [preferably, -(CH₂)ₐ₁- (a1 is an integer of 2 to 5)]. Specific examples include divalent group such as -O-(CH₂)ₖ₁- (k1 is an integer of 1 to 4), -(CH₂)ₖ₁-O- (k1 is an integer of 1 to 4), -S-(CH₂)ₖ₁- (k1 is an integer of 1 to 4), -(CH₂)ₖ₁-S- (k1 is an integer of 1 to 4), -NH-(CH₂)ₖ₁- (k1 is an integer of 1 to 4), -(CH₂)ₖ₁-NH- (k1 is an integer of 1 to 4), -(CH₂)ₖ₂- (k2 is an integer of 2 to 5), -NH-NH-, -CH₂-NH-NH-, -NH-NH-CH₂- and -NH-CH₂-NH-.

Examples of the "5- to 8-membered ring which may be substituted" represented by Ba in the above formula include not only the "saturated 5- to 8-membered ring which may be substituted" as exemplified above, but also an "unsaturated 5- to 8-membered ring which may be substituted" having partially unsaturated bond(s), or a "5- to 8-membered aromatic ring which may be substituted", and in such a case, in a ring represented by the formula: 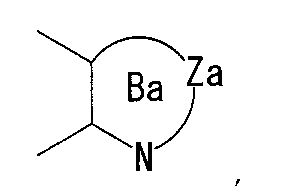 Za may be a divalent group in which a portion of the bonds in the "saturated divalent group in which the number of atom(s) in a linear chain portion is 2 to 5" is converted to an unsaturated bond as exemplified above.

Further, said divalent group may be substituted, and the substituent(s) may be any one so far as it can be bonded with said divalent group. Examples of the "substituent(s)" include those similar to the "substituent" of "the benzene ring which may be substituted" represented by the above Aa, oxo group, and the like. The number of the substituent(s) is 1 to 4 (preferably, 1 to 2), and they are the same or different and may be substituted at any position of said divalent group. Further, when said divalent group has 2 or more substituents, two substituents thereof are bonded to each other to form, for example, lower (C₁₋₆) alkylene (for example, trimethylene, tetramethylene, and the like), lower (C₁₋₆) alkyleneoxy (for example, -CH-O-CH₂-, -O-CH₂-CH₂-, and the like) , lower (C₁₋₆) alkylenedioxy (for example, -O-CH₂-O-, -O-CH₂-CH₂-O-, and the like), lower (C₂₋₆) alkenylene (for example, -CH₂-CH=CH-, -CH₂-CH₂-CH=CH-, -CH₂-CH=CH-CH₂-, and the like), lower (C₄₋₆) alkadienylene (for example, -CH=CH-CH=CH-, and the like), and the like.

In the above formula, examples of the "divalent group in which the number of atom(s) in a linear chain portion is 1 to 4" represented by Xa include saturated divalent groups and divalent groups in which a part of bonds is converted to unsaturated bond(s), such as
(1) -(CH₂)_{f1}- (f1 is an integer of 1 to 4),
(2) -(CH₂)_{g1}-X¹-(CH₂)_{g2}- (g1 and g2 are the same or different and are an integer of 0 to 3, provided that the sum of g1 and g2 is 1 to 3. X¹ indicates NH, O, S, SO or SO₂),
(3) -(CH₂)ₕ₁-X¹-(CH₂)ₕ₂- X²-(CH₂)ₕ₃- (h1, h2 and h3 are the same or different and are an integer of 0 to 2, provided that the sum of h1, h2 and h3 is 0 to 2. Each of X¹ and X² is NH, O, S, SO or SO₂). However, when h2 is 0, at least one of X¹ and X² is preferably NH). Specific examples include divalent groups such as -O-(CH₂)ₖ₃- (k3 is an integer of 1 to 3), -(CH₂)ₖ₃-O- (k3 is an integer of 1 to 3), -S-(CH₂)ₖ₃- (k3 is an integer of 1 to 3), -(CH₂)ₖ₃-S- (k3 is an integer of 1 to 3), NH-(CH₂)ₖ₃- (k3 is an integer of 1 to 3), -(CH₂)ₖ₃-NH- (k3 is an integer of 1 to 3), -(CH₂)ₖ₄-(k4 is an integer of 1 to 4), -CH=CH-, -C≡C-, -CO-NH- and -SO₂-NH-.

As Xa, a divalent group other than -CO-O-CH₂- is preferred, and a divalent group in which the number of carbon atom(s) constituting a linear chain portion is 1 to 4 is more preferred. Among them, C₁₋₄ alkylene, C₂₋₄ alkenylene, and the like are preferred and, in particular, C₂₋₄ alkylene and methylene is preferably used.

The divalent group represented by Xa may be substituted at any possible position (preferably, on a carbon atom), and the substituent may be any one so far as it can be bonded to a divalent chain constituting a linear chain portion. Examples of the substituent include those similar to the "substituent" of "the benzene ring which may be substituted" represented by the above Aa, oxo group, and the like. The number of substituent(s) is 1 to 4 (preferably, 1 to 2). They may be the same or different and may be substituted at any position of said divalent group.

Examples of the preferred substituent of the divalent group represented by Xa include lower (C₁₋₆) alkyl (for example, methyl, ethyl, propyl, isopropyl, butyl, isobutyl, sec-butyl, tert-butyl, pentyl, isopentyl, neopentyl, hexyl, and the like), lower (C₃₋₇) cycloalkyl (for example, cyclopropyl, cyclobutyl, cyclopentyl, cyclohexyl, cycloheptyl, and the like), formyl, lower (C₂₋₇) alkanoyl (for example, acetyl, propionyl, butyryl, and the like), lower (C₁₋₆) alkoxycarbonyl, lower (C₁₋₆) alkoxy, a hydroxy group, oxo, and the like.

In the above formula, examples of the "amino group which may be substituted" represented by Ra¹, amino group, and the like which may have 1 or 2 substituents selected from the "hydrocarbon group which may be substituted" (that similar to the "hydrocarbon group which may be substituted" as the substituent of the benzene ring in "the benzene ring which may be substituted" represented by the above Aa, etc.), the "heterocyclic group which may be substituted" (that similar to the "heterocyclic group which may be substituted" as the substituent of the benzene ring in "the benzene ring which may be substituted" represented by the above Aa, etc.), the "acyl group which may be substituted" (that similar to the "acyl group which may be substituted" as the substituent of the benzene ring in "the benzene ring which may be substituted" represented by the above Aa, etc.), and the like. The substituents of the "amino group which may be substituted" represented by Ra¹ may bond to each other to form a cyclic amino group (for example, a cyclic amino group having a bonding hand on nitrogen atom which is formed by eliminating one hydrogen atom from the nitrogen atom constituting the ring of 5- to 6-membered ring such as tetrahydropyrrole, piperazine, piperidine, morpholine, thiomorpholine, pyrrole and imidazole, and the like). Said cyclic amino group may be substituted, and examples of the substituent include halogen (for example, fluorine, chlorine, bromine, iodine, and the like), nitro, cyano, hydroxy group, thiol group, amino group, carboxyl group, C₁₋₄ alkyl which may be halogenated (for example, trifluoromethyl, methyl, ethyl, and the like), C₁₋₄ alkoxy which may be halogenated (for example, methoxy, ethoxy, propoxy, butoxy, trifluoromethoxy, trifluoroethoxy, and the like), formyl, C₂₋₄ alkanoyl (for example, acetyl, propionyl, and the like), C₁₋₄ alkylsulfonyl (for example, methanesulfonyl, ethanesulfonyl, and the like), and the like, and the number of the substituent(s) is preferably 1 to 3.

Preferred examples of the substituent of the amino group in "the amino group which may be substituted" represented by Ra¹ include
(1) alkyl which may be substituted (for example, C₁₋₁₀ alkyls such as methyl, ethyl, propyl, isopropyl, butyl, isobutyl, sec-butyl, tert-butyl, pentyl, isopentyl, neopentyl, hexyl, heptyl, octyl, nonyl, decyl, and the like, preferably lower (C₁₋₆) alkyl, and the like);
(2) cycloalkyl which may be substituted (for example, C₃₋₈ cycloalkyls such as cyclopropyl, cyclobutyl, cyclopentyl, cyclohexyl, cycloheptyl, and the like); further, said cycloalkyl may be condensed with benzene ring to form indan (for example, indan-1-yl, indan-2-yl, and the like), tetrahydronaphthalene (for example, tetrahydronaphthalen-5-yl, tetrahydronaphthalen-6-yl, and the like), and the like (preferably, indan, and the like); further, said cycloalkyl may be crosslinked through a linear atomic chain having 1 to 2 carbons to form crosslinked cyclic hydrocarbon residual groups such as bicyclo[2.2.1]heptyl, bicyclo[2.2.2]octyl, bicycle[3.2.1]octyl, bicyclo[3.2.2]nonyl, and the like (preferably, cyclohexyl having crosslink through a linear atomic chain having 1 to 2 carbons, and the like, and further preferably, bicyclo[2.2.1]heptyl, and the like);
(3) alkenyl which may be substituted (for example, C₂₋₁₀ alkenyl such as allyl, crotyl, 2-pentenyl and 3-hexenyl, preferably lower (C₂₋₆) alkenyl, and the like);
(4) cycloalkenyl which may be substituted (for example, C₃₋₇ cycloalkenyl such as 2-cyclopentenyl, 2-cyclohexenyl, 2-cyclopentenylmethyl, 2-cyclohexenylmethyl, and the like);
(5) aralkyl which may be substituted (for example, phenyl-C₁₋₄ alkyl) (for example, benzyl, phenethyl, and the like);
(6) formyl or acyl which may be substituted (for example, C₂₋₄ alkanoyl (for example, acetyl, propionyl, butyryl, isobutyryl, and the like), C₁₋₄ alkylsulfonyl (for example, methanesulfonyl, ethanesulfonyl, and the like);
(7) aryl which may be substituted (for example, phenyl, naphthyl, and the like);
(8) a heterocyclic group which may be substituted (for example, a group formed by eliminating one hydrogen atom from a 5- to 6-membered aromatic heterocyclic ring which contains 1 to 4 hetero atoms of 1 to 2 kinds selected from nitrogen atom, sulfur atom and oxygen atom such as furan, thiophene, pyrrole, imidazole, pyrazole, thiazole, oxazole, isothiazole, isoxazole, tetrazole, pyridine, pyrazine, pyrimidine, pyridazine, triazole, and the like), a group formed by eliminating one hydrogen atom from a 5- to 8-membered non-aromatic heterocyclic ring which contains 1 to 4 hetero atoms of 1 to 2 kinds selected from nitrogen atom, sulfur atom and oxygen atom such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, and the like.

Examples of the substituent of the above (1) alkyl which may be substituted, (2) cycloalkyl which may be substituted, (3) alkenyl which may be substituted, (4) cycloalkenyl which may be substituted, (5) aralkyl which may be substituted, (6) acyl which may be substituted, (7) aryl which may be substituted and (8) a heterocyclic group which may be substituted include halogen (for example, fluorine, chlorine, bromine, iodine, and the like), C₁₋₄ alkyl which may be substituted with a halogen atom or C₁₋₄ alkoxy, C₁₋₄ alkoxy which may be substituted with a halogen atom or C₁₋₄ alkoxy (for example, methoxy, ethoxy, propoxy, butoxy, trifluoromethoxy, trifluoroethoxy, and the like), C₁₋₄ alkylenedioxy (for example, -O-CH₂-O-, -O-CH₂-CH₂-O-, and the like), formyl, C₂₋₄ alkanoyl (for example, acetyl, propionyl, and the like), C₁₋₄ alkylsulfonyl (for example, methanesulfonyl, ethanesulfonyl, and the like), phenyl-lower (C₁₋₄ ) alkyl, C₃₋₇ cycloalkyl, cyano, nitro, hydroxy group, thiol group which may be substituted (for example, thiol, C₁₋₄ alkylthio, and the like), amino group which may be substituted (for example, amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, and the like), carboxyl group, lower (C₁₋₄) alkoxy-carbonyl, lower (C₇₋₁₀) aralkyloxy-carbonyl, carbamoyl, mono-C₁₋₄ alkylcarbamoyl, di-C₁₋₄ alkylcarbamoyl (preferably, halogen, lower (C₁₋₄ ) alkyl which may be halogenated, lower (C₁₋₄ ) alkoxy which may be halogenated, phenyl-lower (C₁₋₄ ) alkyl, C₃₋₇ cycloalkyl, cyano, hydroxy group, and the like), and the number of the substituent(s) is preferably 1 to 3.

Preferred examples of the "amino group which may be substituted" represented by Ra¹ include in particular an amino group which may have 1 to 2 alkyls which may be substituted [for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower (C₁₋₆) alkyl, and the like, which may have 1 to 3 substituents selected from halogen (for example, fluorine, chlorine, bromine, iodine, and the like), nitro, cyano, hydroxy group, thiol group which may be substituted (for example, thiol, C₁₋₄ alkylthio, and the like), amino group which may be substituted (for example, amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, and the like), carboxyl group which may be esterified or amidated (for example, carboxyl, C₁₋₄ alkoxy-carbonyl, lower (C₇₋₁₀)aralkyloxy-carbonyl, carbamoyl, mono-C₁₋₄ alkyl-carbamoyl, di-C₁₋₄ alkyl-carbamoyl, and the like) , C₁₋₄ alkyl which may be substituted with a halogen atom or C₁₋₄ alkoxy (for example, trifluoromethyl, methyl, ethyl, and the like), C₁₋₄ alkoxy which may be substituted with a halogen atom or C₁₋₄ alkoxy (for example, methoxy, ethoxy, trifluoromethoxy, trifluoroethoxy, and the like), C₁₋₄ alkylenedioxy (for example, -O-CH₂-O-, -O-CH₂-CH₂-O-, and the like), phenyl-lower (C₁₋₄) alkyl, C₃₋₇ cycloalkyl, formyl, C₂₋₄ alkanoyl (for example, acetyl, propionyl, and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), and the like.

Examples of the "cyclic group" of the "cyclic group which may be substituted" represented by Ra² in the above formula include 5- to 8-membered (preferably, 5- to 6-membered) saturated or unsaturated alicyclic monocyclic hydrocarbon such as C₅₋₈ cycloalkane (for example, cyclopentane, cyclohexane, cycloheptane, and the like), C₅₋₈ cycloalkene (for example, 1-cyclopentene, 2-cyclopentene, 3-cyclopentene, 2-cyclohexene, 3-cyclohexene, and the like) and C₅₋₈ cycloalkadiene (for example, 2,4-cyclopentadiene, 2,4-cyclohexadiene, 2,5-cyclohexadiene, and the like); 6-membered aromatic monocyclic hydrocarbons such as benzene, 5- to 8-membered aromatic monocyclic heterocyclic rings which contain at least one (preferably, 1 to 4 and more preferably, 1 to 2) of 1 to 3 kinds (preferably, 1 to 2 kinds) of hetero atoms selected from oxygen atom, sulfur atom, nitrogen atom, and the like; a saturated or unsaturated non-aromatic monocyclic heterocyclic ring (aliphatic heterocyclic ring), and the like; and a group formed by eliminating one hydrogen atom from a ring in which 2 to 3 the same or different rings selected from these monocyclic rings are condensed, etc.

Examples of the "aromatic monocyclic heterocyclic ring" used herein include a 5- to 8-membered (preferably, 5- to 6-membered) aromatic monocyclic heterocyclic ring (for example, furan, thiophene, pyrrole, oxazole, isoxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, and the like), and the like. Examples of the "non-aromatic monocyclic heterocyclic ring" include a 5- to 8-membered (preferably, 5- to 6-membered) saturated or unsaturated monocyclic non-aromatic heterocyclic ring (aliphatic heterocyclic ring) such as pyrrolidine, tetrahydrofuran, tetrahydrothiophene, thiophene, thiolane, oxathiolane, pyrroline, imidazolidine, imidazoline, pyrazolidine, pyrazoline, oxazine, oxadiazine, thiazine, thiadiazine, piperidine, morpholine, thiomorpholine, tetrahydropyran, piperazine, pyran, oxepine, thiepin, azepine, and the like, or a 5- to 8-membered non-aromatic heterocyclic ring in which a part or all of double bonds of the above aromatic monocyclic heterocyclic ring are saturated, and the like.

Further, "the cyclic group" of "the cyclic group which may be substituted" represented by Ra² may also be a group formed by eliminating one hydrogen atom from a condensed ring formed by condensing 2 to 3 (preferably, 2) the same or different rings selected from the monocyclic homocyclic rings or heterocyclic rings exemplified above, and these condensed rings may be any one of a saturated condensed ring, a condensed ring partially having unsaturated bond(s), and an aromatic condensed ring.

Preferred examples of the condensed ring include a ring obtained by condensing two the same or different rings (preferably, one heterocyclic ring and one aromatic heterocyclic ring, and more preferably, two the same or different aromatic heterocyclic rings); a ring obtained by condensing one heterocyclic ring and one homocyclic ring (preferably, one heterocyclic ring and one benzene ring, and more preferably, one aromatic heterocyclic ring and one benzene ring); and the like. Specific examples of the condensed ring include indole, benzothiophene, benzofuran, benzimidazole, imidazo[1,2-a]pyridine, quinoline, isoquinoline, cinnoline, and the like.

Examples of the substituent of the "cyclic group" of "the cyclic group which may be substituted" represented by Ra² include those similar to the substituents of the "hydrocarbon group which may be substituted" as the substituent of the benzene ring in the "benzene ring which may be substituted" represented by the above A.

The "cyclic group" of the "cyclic group which may be substituted" represented by Ra² is preferably a 5- to 6-membered cyclic group, a 5- to 6-membered aromatic cyclic group is also preferred, and phenyl, furyl, thienyl, pyrrolyl, pyridyl (preferably, 6-membered ring), and the like are further preferred, in particular, phenyl is preferred.

Among the compound represented by the formula (Ia) or a salt thereof, there can be used a compound represented by the formula (IIa); 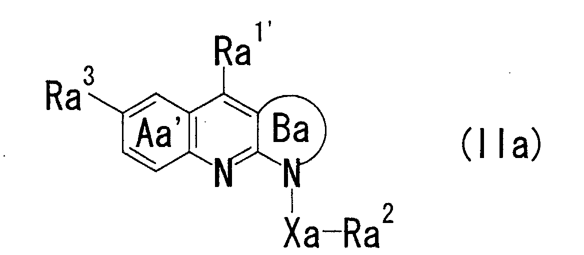 wherein Aa' is a benzene ring which may be further substituted in addition to the substituent Ra³, Ba is a 5-to 8-membered ring which may be substituted, Xa is a divalent group in which the number of atom(s) in a linear chain portion is 1 to 4, Ra^{1'} is an amino group substituted with 1 to 2 lower alkyl groups which may be substituted, Ra² is a cyclic group which may be substituted, Ra³ is a hydrocarbon group which may be substituted, a heterocyclic group which may be substituted, nitro group, a halogen atom, amino group which may be substituted, or a group represented by Ra⁴-Ya- (wherein Y is oxygen atom or sulfur atom which may be oxidized, and Ra⁴ is a hydrocarbon group which may be substituted, or a heterocyclic group which may be substituted), or a salt thereof, and
a compound represented by the formula (IIa'), 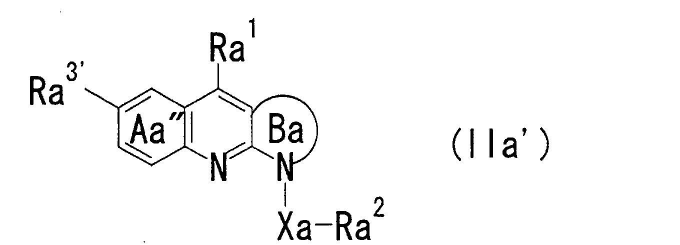 wherein Aa'' is a benzene ring which may be further substituted in addition to the substituent Ra^{3'}, Ba is a 5-to 8-membered ring which may be substituted, Xa is a divalent group in which the number of atom(s) in a linear chain portion is 1 to 4, Ra¹ is an amino group which may be substituted, Ra² is a cyclic group which may be substituted, Ra^{3'} is a hydrocarbon group which may be substituted, a heterocyclic group which may be substituted, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted), or a salt thereof.

In the above formulae, examples of the substituent of the "benzene ring" in the "benzene ring which may be further substituted in addition to the substituent Ra³" represented by Aa' and in the "benzene ring which may be further substituted in addition to the substituent Ra^{3'}" represented by Aa'' include those similar to the substituent of "the benzene ring" in "the benzene ring which may be substituted" represented by above Aa.

In the above formula, examples of the "substituted amino group" represented by Ra^{1'} include the "amino group which may be substituted" represented by the above Ra¹ except the unsubstituted amino group, namely amino group having 1 to 2 the same or different substituents similar to the substituent of the "amino group" in the "amino group which may be substituted" represented by the above Ra¹, etc. Among these, the "amino group substituted with 1 to 2 lower alkyl groups which may be substituted" is preferred.

Examples of the amino group substituted with 1 to 2 of lower alkyl groups which may be substituted include amino group substituted with 1 to 2 lower (C₁₋₆) alkyls (for example, methyl, ethyl, propyl, isopropyl, butyl, isobutyl, sec-butyl, tert-butyl, pentyl, isopentyl, neopentyl and hexyl) which may have 1 to 3 substituents selected from
(1) halogen (for example, fluorine, chlorine, bromine, iodine, and the like),
(2) nitro,
(3) cyano,
(4) hydroxy group,
(5) thiol group which may be substituted (for example, thiol, C₁₋₄ alkylthio, and the like),
(6) amino group which may be substituted (for example, amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, and the like),
(7) carboxyl group which may be esterified or amidated (for example, carboxyl, C₁₋₄ alkoxy-carbonyl, lower (C₇₋ ₁₀)aralkyloxy-carbonyl, carbamoyl, mono-C₁₋₄ alkylcarbamoyl, di-C₁₋₄ alkylcarbamoyl, and the like),
(8) C₁₋₄ alkyl which may be substituted with a halogen atom or C₁₋₄ alkoxy (for example, trifluoromethyl, methyl, ethyl, and the like),
(9) C₁₋₄ alkoxy which may be substituted with a halogen atom or C₁₋₄ alkoxy (for example, methoxy, ethoxy, trifluoromethoxy, trifluoroethoxy, and the like),
(10) C₁₋₄ alkylenedioxy (for example, -O-CH₂-O-, -O-CH₂-CH₂-O-, and the like),
(11) phenyl-lower (C₁₋₄) alkyl,
(12) C₃₋₇ cycloalkyl, formyl, C₂₋₄ alkanoyl (for example, acetyl, propionyl, and the like),
(13) C₁₋₄ alkylsulfonyl (for example, methanesulfonyl, ethanesulfonyl, and the like),
(14) C₁₋₄ alkylsulfinyl (for example, methanesulfinyl, ethanesulfinyl, and the like), and the like. When the number of substituents of the amino group is two, they may be the same or different. In the above formula, examples of "the hydrocarbon group which may be substituted" represented by Ra³ and Ra^{3'} include those similar to the "hydrocarbon group which may be substituted" as the substituent of the "benzene ring" in the "benzene ring which may be substituted" represented by the above A.

In the above formula, examples of the "heterocyclic group which may be substituted" represented by Ra³ and Ra^{3,} include those similar to the "heterocyclic group which may be substituted" as the substituent of the "benzene ring" in the "benzene ring which may be substituted" represented by the above Aa.

In the above formula, examples of the "amino group which may be substituted" represented by Ra³ and Ra^{3'} include those similar to the "the amino group which may be substituted" as the substituent of the "benzene ring" in the "benzene ring which may be substituted" represented by the above Aa.

In the above formula, examples of the "hydrocarbon group which may be substituted" and the "heterocyclic group which may be substituted" represented by Ra⁴ in the group represented by the formula Ra⁴-Ya- include those similar to the "hydrocarbon group which may be substituted" and the "heterocyclic group which may be substituted" as the substituent of the "benzene ring" in "the benzene ring which may be substituted" represented by the above Aa.

In the above formula, examples of the "sulfur atom which may be oxidized" represented by Ya in the group represented by the formula Ra⁴-Ya- include S, S(O), S(O)₂, and the like.

As Ra^{3'}, the "hydrocarbon group which may be substituted", in particular, alkyl is preferred and as Ra¹, amino is preferred.

In the above formula, the "benzene ring which may be further substituted" represented by Bb or Cb is a benzene ring which may be further substituted in addition to the substituent specifically shown in the formula (Ib), and examples of the substituent (the substituent other than the substituent specifically shown in the formula (Ib)) include a hydrocarbon group which may be substituted, a heterocyclic group which may be substituted, nitro group, a halogen atom, amino group which may be substituted, a group represented by the formula Rb⁶-Yb- (wherein Yb is oxygen atom or sulfur atom which may be oxidized (for example, S, S(O), S(O)₂, and the like), and Rb⁶ indicates a hydrocarbon group which may be substituted, or a heterocyclic group which may be substituted), cyano group, an acyl group which may be substituted, carboxylic group which may be esterified or amidated, and the like.

Examples of the "hydrocarbon group which may be substituted" as the substituent of the benzene ring in the "benzene ring which may be further substituted" represented by Bb or Cb and the "hydrocarbon group" in "the hydrocarbon group which may be substituted" represented by Rb⁶ include:
(1) alkyl (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, and the like, preferably lower (C₁₋₆) alkyl, and the like);
(2) cycloalkyl (for example, C₃₋₈ cycloalkyl such as cyclopropyl, cyclobutyl, cyclopentyl, cyclohexyl, cycloheptyl, and the like); further, said cycloalkyl may be condensed with benzene ring to form indan (for example, indan-1-yl, indan-2-yl, and the like), tetrahydronaphthalene (for example, tetrahydronaphthalen-5-yl, tetrahydronaphthalen-6-yl, and the like), and the like (preferably, indan, and the like); further, said cycloalkyl may be crosslinked through a linear atomic chain having 1 to 2 carbons to form crosslinked cyclic hydrocarbon residual groups such as bicyclo[2.2.1]heptyl, bicyclo[2.2.2]octyl, bicyclo[3.2.1]octyl, bicyclo[3.2.2]nonyl, and the like (preferably, cyclohexyl having crosslink through a linear atomic chain having 1 to 2 carbons, and the like, and further preferably, bicyclo[2.2.1]heptyl, and the like);
(3) alkenyl (for example, C₂₋₁₀ alkenyl such as vinyl, allyl, crotyl, 2-pentenyl, 3-hexenyl, and the like, preferably lower (C₂₋₆) alkenyl, and the like);
(4) cycloalkenyl (for example, C₃₋₈ cycloalkenyls such as 2-cyclopentenyl, 2-cyclohexenyl, 2-cyclopentenylmethyl, 2-cyclohexenylmethyl, and the like);
(5) alkynyl (for example, C₂₋₁₀ alkynyl such as ethynyl, 1-propynyl, 2-propynyl, 1-butynyl, 2-pentynyl, 3-hexynyl, and the like, preferably lower (C₂₋₆) alkynyl, and the like);
(6) aryl (for example, C₆₋₁₄ aryls such as phenyl, naphthyl, and the like, preferably C₆₋₁₀ aryl, more preferably phenyl, and the like);
(7) aralkyl (for example, C₁₋₆ alkyl having 1 to 3 C₆₋₁₄ aryls, preferably phenyl-C₁₋₄ alkyl (for example, benzyl, phenethyl, and the like), and the like. Among these, alkyl is preferred, C₁₋₄ alkyl such as methyl, ethyl, and the like are more preferred and, in particular, methyl is preferably used.

The hydrocarbon group may be substituted, and examples of the substituent include halogen (for example, fluorine, chlorine, bromine, iodine, and the like), nitro, cyano, oxo, hydroxy group, thiol. group which may be substituted (for example, thiol, C₁₋₄ alkylthio, and the like), amino group which may be substituted (for example, amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole and imidazole, and the like), phenyl-lower (C₁₋₄) alkyl, C₃₋₇ cycloalkyl, carboxyl group which may be esterified or amidated (for example, carboxyl, C₁₋₄ alkoxy-carbonyl, lower (C₇₋₁₀)aralkyloxy-carbonyl, carbamoyl, mono-C₁₋₄ alkylcarbamoyl, di-C₁₋₄ alkylcarbamoyl, and the like), C₁₋₄ alkyl which may be substituted with a halogen atom or C₁₋₄ alkoxy (for example, trifluoromethyl, methyl, ethyl, and the like), C₁₋₄ alkoxy which may be substituted with a halogen atom or C₁₋₄ alkoxy (for example, methoxy, ethoxy, trifluoromethoxy, trifluoroethoxy, and the like), C₁₋₄ alkylenedioxy (for example, -O-CH₂-O-, -O-CH₂-CH₂-O-, and the like) , formyl, C₂₋ ₄ alkanoyl (for example, acetyl, propionyl, and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), and the like. The number of the substituent(s) is preferably 1 to 3.

Examples of the "heterocyclic group which may be substituted" as the substituent of the "benzene ring" in the "benzene ring which may be further substituted" represented by Bb and Cb and the "heterocyclic group" in the "heterocyclic group which may be substituted" represented by Rb⁶ include a group formed by eliminating one hydrogen atom from a 5- to 8-membered aromatic heterocyclic ring which contains at least one (preferably, 1 to 4, and more preferably, 1 to 2) of 1 to 3 kinds (preferably, 1 to 2 kinds) of hetero atoms selected from oxygen atom, sulfur atom, nitrogen atom, and the like, a saturated or unsaturated non-aromatic heterocyclic ring (aliphatic heterocyclic ring), and the like.

Examples of the "aromatic heterocyclic ring" used herein include a 5- to 8-membered (preferably, 5- to 6-membered) aromatic monocyclic heterocyclic ring (for example, furan, thiophene, pyrrole, oxazole, isoxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, and the like), and the like. Examples of the "non-aromatic heterocyclic ring" include a 5- to 8-membered (preferably, 5- to 6-membered) saturated or unsaturated monocyclic non-aromatic heterocyclic ring (aliphatic heterocyclic ring) such as pyrrolidine, tetrahydrofuran, tetrahydrothiophene, thiolane, dithiolane, oxathiolane, pyrroline, imidazolidine, imidazoline, pyrazolidine, pyrazoline, oxazine, oxadiazine, thiazine, thiadiazine, piperidine, morpholine, thiomorpholine, tetrahydropyran, piperazine, pyran, oxepine, thiepin and azepine, or a 5- to 8-membered non-aromatic heterocyclic ring in which a part or all of double bonds of the above aromatic monocyclic heterocyclic ring are saturated, and the like.

Further, examples of the "heterocyclic group which may be substituted" as the substituent of the benzene ring in the "benzene ring which may be further substituted" represented by Bb or Cb and "the heterocyclic group" in the "heterocyclic group which may be substituted" represented by Rb⁶ include a group formed by eliminating one hydrogen atom from a condensed ring which is formed by condensing 2 to 3 rings (preferably, 2) selected from the above monocyclic heterocyclic rings (a monocyclic aromatic heterocyclic ring and a monocyclic non-aromatic heterocyclic ring) and 5- to 8-membered cyclic hydrocarbons (5- to 8-membered (preferably, 5- to 6-membered) saturated or unsaturated alicyclic hydrocarbons such as C₅₋₈ cycloalkane, C₅₋₈ cycloalkene and C₅₋₈ cycloalkadiene; 6-membered aromatic hydrocarbons such as benzene; and the like). These condensed rings may be any one of a saturated condensed ring, a condensed ring partially having unsaturated bond(s), and an aromatic condensed ring.

Preferred examples of such condensed ring include a ring in which two the same or different heterocyclic rings (preferably, one heterocyclic ring and one aromatic heterocyclic ring, and more preferably, the same or different two aromatic heterocyclic rings) are condensed; one heterocyclic ring and one homocyclic ring (preferably, one heterocyclic ring and one benzene ring, and more preferably, one aromatic heterocyclic ring and one benzene ring): and the like. Specific examples of the condensed ring include indole, benzothiophene, benzofuran, benzimidazole, imidazo[1,2-a]pyridine, quinoline, isoquinoline cinnoline, and the like.

The "heterocyclic group which may be substituted" as the substituent of the benzene ring in the "benzene ring which may be further substituted" represented by Bb or Cb and the "heterocyclic group" in the "heterocyclic group which may be substituted" represented by Rb⁶ may be substituted, and examples thereof include those similar to the substituent of "the hydrocarbon group which may be substituted" as the substituent of the benzene ring in "the benzene ring which may be further substituted" represented by the above Bb or Cb.

Examples of the "halogen atom" as the substituent of the benzene ring in the "benzene ring which may be substituted" represented by Bb or Cb include fluorine, chlorine, bromine, iodine, and the like.

Examples of the "amino group which may be substituted" as the substituent of the benzene ring in the "benzene ring which may be further substituted" represented by Bb or Cb include those similar to the "amino group which may be substituted" represented by Ab described hereinafter. Among these, preferred are amino group which may have one to two substituents selected from the "hydrocarbon group which may be substituted" (that similar to the "hydrocarbon group which may be substituted" as the substituent of the benzene ring in the "benzene ring which may be further substituted" represented by the above Bb or Cb), the "heterocyclic group which may be substituted" (that similar to the "heterocyclic group which may be substituted" as the substituent of the benzene ring in the "benzene ring which may be further substituted" represented by the above Bb or Cb), and the "acyl group which may be substituted" (that similar to the "acyl group which may be substituted" as the substituent of the benzene ring in the "benzene ring which may be further substituted" represented by Bb or Cb hereinafter). Among these, preferred is an amino group which may have 1 to 2 alkyls which may be substituted [for example, C₁₋₁₀ alkyls such as methyl, ethyl, propyl, isopropyl, butyl, isobutyl, sec-butyl, tert-butyl, pentyl, isopentyl, neopentyl, hexyl, heptyl, octyl, nonyl, decyl, and the like, preferably lower (C₁₋₆) alkyl, and the like which may have 1 to 3 substituents selected, for example, halogen (for example, fluorine, chlorine, bromine, iodine, and the like), nitro, cyano, hydroxy group, thiol group which may be substituted (for example, thiol, C₁₋₄ alkylthio, and the like), amino group which may be substituted (for example, amino, mono-C₁₋₄ alkylamino, di-C₁₋₄ alkylamino, 5-to 6-membered cyclic amino such as tetrahydropyrrole, piperazine, piperidine, morpholine, thiomorpholine, pyrrole, imidazole, and the like), phenyl-lower (C₁₋₄) alkyl, C₃₋₇ cycloalkyl, carboxyl group which may be esterified or amidated (for example, carboxyl, C₁₋₄ alkoxy-carbonyl, lower (C₇₋₁₀) aralkyloxy-carbonyl, carbamoyl, mono-C₁₋₄ alkylcarbamoyl, di-C₁₋₄ alkylcarbamoyl, and the like), C₁₋₄ alkyl which may be substituted with a halogen atom or C₁₋₄ alkoxy (for example, trifluoromethyl, methyl, ethyl, and the like), C₁₋₄ alkoxy which may be substituted with a halogen atom or C₁₋₄ alkoxy (for example, methoxy, ethoxy, trifluoromethoxy, trifluoroethoxy, and the like), C₁₋₄ alkylenedioxy (for example, -O-CH₂-O-, -O-CH₂-CH₂-O-, and the like), formyl, C₂₋₄ alkanoyl (for example, acetyl, propionyl, and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), and the like].

Further, the substituents of the "amino group which may be substituted" as the substituent of the benzene ring in the "benzene ring which may be further substituted" represented by Bb or Cb may be bonded to each other to form a cyclic amino group (for example, a cyclic amino group formed by eliminating one hydrogen atom from the ring-constituting nitrogen atom of a 5- to 6-membered ring such as tetrahydropyrrole, piperazine, piperidine, morpholine, thiomorpholine, pyrrole, imidazole, and the like and has a bonding hand on the nitrogen atom, and the like). Said cyclic amino group may be substituted and examples of the substituent include halogen (for example, . fluorine, chlorine, bromine, iodine, and the like), nitro, cyano, hydroxy group, thiol group, amino group, carboxyl group, C₁₋₄ alkyl which may be halogenated (for example, trifluoromethyl, methyl, ethyl, and the like), C₁₋₄ alkoxy which may be halogenated (for example, methoxy, ethoxy, propoxy, butoxy, trifluoromethoxy, trifluoroethoxy, and the like), formyl, C₂₋₄ alkanoyl (for example, acetyl, propionyl and the like), C₁₋₄ alkylsulfonyl (for example, methanesulfonyl, ethanesulfonyl, and the like), and the like. The number of the substituent(s) is preferably 1 to 3.

Examples of the "acyl group which may be substituted" as the substituent of the benzene ring in the "benzene ring which may be further substituted" represented by Bb or Cb include those obtained by bonding hydrogen, the "hydrocarbon group which may be substituted" (that similar to the "hydrocarbon group which may be substituted" as the substituent of the benzene ring in the "benzene ring which may be further substituted" represented by the above Bb or Cb), the "heterocyclic group which may be substituted" (that similar to the "heterocyclic group which may be substituted" as the substituent of the benzene ring in the "benzene ring which may be substituted" represented by the above Bb or Cb), and the like, with carbonyl group or sulfonyl group. Preferred examples include those obtained by bonding
(1) hydrogen,
(2) alkyl which may be substituted (C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, and the like, preferably lower (C₁₋₆) alkyl, and the like);
(3) cycloalkyl which may be substituted (for example, C₃₋₇ cycloalkyl such as cyclopropyl, cyclobutyl, cyclopentyl, cyclohexyl, cycloheptyl, and the like);
(4) alkenyl which may be substituted (for example, C₂₋₁₀ akenyl such as allyl, crotyl, 2-pentenyl, 3-hexenyl, and the like, preferably lower (C₂₋₆) alkenyl, and the like);
(5) cycloalkenyl which may be substituted (for example, C₃₋₇ cycloalkenyl such as 2-cyclopentenyl, 2-cyclohexenyl, 2-cyclopentenylmethyl, 2-cyclohexenylmethyl, and the like);
(6) 5- to 6-membered monocyclic aromatic group which may be substituted (for example, phenyl, pyridyl, and the like), and the like, with carbonyl group or sulfonyl group (for example, acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl, and the like). Examples of the substituent of (2) alkyl which may be substituted, (3) cycloalkyl which may be substituted, (4) alkenyl which may be substituted,
(5) cycloalkenyl which may be substituted, and (6) 5- to 6-membered monocyclic aromatic group which may be substituted include halogen (for example, fluorine, chlorine, bromine, iodine, and the like), nitro, cyano, hydroxy group, thiol group which may be substituted (for example, thiol, C₁₋₄ alkylthio, and the like), amino group which may be substituted (for example, amino, mono-C₁₋₄ alkylamino, di-C₁₋ ₄ alkylamino, 5- to 6-membered cyclic amino such as tetrahydropyrrole, piperazine, piperidine, morpholine, thiomorpholine, pyrrole, imidazole, and the like), carboxyl group which may be esterified or amidated (for example, carboxyl, C₁₋₄ alkoxy-carbonyl, carbamoyl, mono-C₁₋₄ alkylcarbamoyl, di-C₁₋₄ alkylcarbamoyl, and the like), C₁₋₄ alkyl which may be substituted with a halogen atom or C₁₋₄ alkoxy (for example, trifluoromethyl, methyl, ethyl, and the like), C₁₋₄ alkoxy which may be substituted with a halogen atom or C₁₋₄ alkoxy (for example, methoxy, ethoxy, trifluoromethoxy, trifluoroethoxy, and the like), formyl, C₂₋₄ alkanoyl (for example, acetyl, propionyl, and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), and the like, and the number of the substituent(s) is preferably 1 to 3.

Examples of the "carboxyl group which may be esterified" as the substituent of the benzene ring in the "benzene ring which may be further substituted" represented by Bb or Cb include those obtained by bonding hydrogen, the " hydrocarbon group which may be substituted" (that similar to the "hydrocarbon group which may be substituted" as the substituent of the benzene ring in "the benzene ring which may be further substituted" represented by the above Bb or Cb), and the like, with carbonyloxy group. Preferred examples include those obtained by bonding:
(1) hydrogen,
(2) alkyl which may be substituted (C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, and the like, preferably lower (C₁₋₆) alkyl, and the like);
(3) cycloalkyl which may be substituted (for example, C₃₋₇ cycloalkyl such as cyclopropyl, cyclobutyl, cyclopentyl, cyclohexyl, cycloheptyl, and the like);
(4) alkenyl. which may be substituted (for example, C₂₋₁₀ alkenyl such as allyl, crotyl, 2-pentenyl, 3-hexenyl, and the like, preferably lower (C₂₋₆) alkenyl, and the like);
(5) cycloalkenyl which may be substituted(for example, C₃₋₇ cycloalkenyl such as 2-cyclopentenyl, 2-cyclohexenyl, 2-cyclopentenylmethyl, 2-cyclohexenylmethyl, and the like);
(6) aryl which may be substituted (for example, phenyl, naphthyl, and the like), with carbonyloxy group. Preferred are carboxyl, lower (C₁₋₆) alkoxycarbonyl, aryloxycarbonyl (for example, methoxycarbonyl, ethoxycarbonyl, propoxycarbonyl, phenoxycarbonyl, naphthoxycarbonyl, and the like), and the like. Examples of the substituent of (2) alkyl which may be substituted, (3) cycloalkyl which may be substituted, (4) alkenyl which may be substituted, (5) cycloalkenyl which may be substituted, and (6) aryl which may be substituted include halogen (for example, fluorine, chlorine, bromine, iodine, and the like), nitro, cyano, hydroxy group, thiol group which may be substituted (for example, thiol, C₁₋₄ alkylthio, and the like), amino group which may be substituted (for example, amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, and the like), carboxyl group which may be esterified or amidated (for example, carboxyl, C₁₋₄ alkoxy-carbonyl, carbamoyl, mono-C₁₋₄ alkylcarbamoyl, di-C₁₋₄ alkylcarbamoyl, and the like), C₁₋₄ alkyl which may be substituted with a halogen atom or C₁₋₄ alkoxy (for example, trifluoromethyl, methyl, ethyl, and the like), C₁₋₄ alkoxy which may be substituted with a halogen atom or C₁₋₄ alkoxy (for example, methoxy, ethoxy, trifluoromethoxy, trifluoroethoxy, and the like), formyl, C₂₋₄ alkanoyl (for example, acetyl, propionyl, and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), and the like, and the number of the substituent(s) is preferably 1 to 3.

Examples of the "carboxyl group which may be amidated" as the substituent of the benzene ring in the "benzene ring which may be further substituted" represented by Bb or Cb include those obtained by bonding
(1)hydroxy group,
(2) "the amino group which may be substituted" (that similar to the "amino group which may be substituted" as the optional substituent of the benzene ring in the "benzene ring which may be further substituted" represented by the above Bb or Cb), and the like, with carbonyl group.

One to four (preferably, 1 to 2) of the substituent(s) of the benzene ring in the "benzene ring which may be further substituted" represented by Bb or Cb may be the same or different and may be substituted at any position of the ring. Further, when the benzene ring in the "benzene ring which may be further substituted" represented by Bb or Cb has 2 or more substituents, two substituents among these are bonded to each other, for example, to form lower (C₁₋₆) alkylene (for example, trimethylene, tetramethylene, and the like), lower (C₁₋₆) alkyleneoxy (for example, -CH₂-O-CH₂-O-, -O-CH₂-CH₂-, and the like) , lower (C₁₋₆) alkylenedioxy (for example, -O-CH₂-O-, -O-CH₂-CH₂-O-, and the like), lower (C₂₋₆) alkenylene (for example, -CH₂-CH=CH-, -CH₂-CH₂-CH=CH-, -CH₂-CH=CH-CH₂-, and the like), lower (C₄₋₆) alkadienylene (for example, -CH=CH-CH=CH-, and the like), and the like.

As the substituent of the benzene ring in the "benzene ring which may be further substituted" represented by Bb or Cb, preferred are a hydrocarbon group which may be substituted, a heterocyclic group which may be substituted, nitro group, halogen atom, amino group which may be substituted, a group represented by Rb⁶-Yb- (wherein Yb is oxygen atom or sulfur atom which may be oxidized, and Rb⁶ is a hydrocarbon group which may be substituted, or a heterocyclic group which may be substituted), and the like. More preferred are a hydrocarbon group which may be substituted, a heterocyclic group which may be substituted, a halogen atom, an amino group which may be substituted, a group represented by Rb⁶-Yb- (wherein Yb indicates oxygen atom or sulfur atom which may be oxidized, and Rb⁶ indicates a hydrocarbon group which may be substituted, or a heterocyclic group which may be substituted), and the like. Among these, in particular, lower (C₁₋₄) alkyl, a halogen atom, and the like are preferred.

As the " benzene ring " in the " benzene ring which may be further substituted " represented by Bb or Cb, preferred is a benzene ring which does not have a substituent other than the each substituent represented explicitly.

Examples of the "hydrocarbon group" in the "hydrocarbon group which may be substituted" represented by Rb¹, Rb² and Rb³ include
(1) alkyl (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, and the like, preferably lower (C₁₋₆) alkyl, and the like);
(2) cycloalkyl (for example, C₃₋₈ cycloalkyls such as cyclopropyl, cyclobutyl, cyclopentyl, cyclohexyl, cycloheptyl, and the like); further, said cycloalkyl may be condensed with benzene ring to form indan (for example, indan-1-yl, indan-2-yl, and the like), tetrahydronaphthalene (for example, tetrahydronaphthalen-5-yl, tetrahydronaphthalen-6-yl, and the like), and the like (preferably, indan, and the like); further, said cycloalkyl may be crosslinked through a linear atomic chain having 1 to 2 carbons to form crosslinked cyclic hydrocarbon residual groups such as bicyclo[2,2,1]heptyl, bicyclo[2,2,2]octyl, bicyclo[3,2,1]octyl, bicyclo[3,2,2]nonyl, and the like (preferably, cyclohexyl having crosslink through a linear atomic chain having 1 to 2 carbons, and the like, and further preferably, bicyclo[2,2,1]heptyl, and the like);
(3) alkenyl (for example, C₂₋₁₀ alkenyl such as vinyl, allyl, crotyl, 2-pentenyl, 3-hexenyl, and the like, preferably lower (C₂₋₆) alkenyl, and the like);
(4) cycloalkenyl (for example, C₃₋₈ cycloalkenyl such as 2-cyclopentenyl, 2-cyclohexenyl, 2-cyclopentenylmethyl, 2-cyclohexenylmethyl, and the like);
(5) alkynyl (for example, C₂₋₁₀ alkynyl such as ethynyl, 1-propynyl, 2-propynyl, 1-butynyl, 2-pentynyl, 3-hexynyl, preferably lower (C₂₋₆) alkynyl, and the like);
(6) aryl (for example, C₆₋₁₄ aryl such as phenyl, naphthyl, and the like, preferably C₆₋₁₀ aryl, more preferably phenyl, and the like);
(7) aralkyl (for example, C₁₋₆ alkyl having 1 to 3 C₆₋₁₄ aryl, preferably phenyl-C₁₋₄ alkyl (for example, benzyl, phenethyl, and the like), and the like.
(8) a group represented by the formula

   X'''-G-(CH₂)ₙ-J

   wherein X''' is a C₁₋₄ alkylene group or a C₁₋₄ alkenylene group, G is a bonding hand, -O-, -S-, -CO-NH- or -NH-CO-, n is an integer of 0 to 3, and J is an aromatic cyclic group which may be substituted, and the like.

In the above formula, examples the aromatic cyclic group which may be substituted represented by J include an aryl group which may be substituted, an aromatic heterocyclic group which may be substituted, and the like.

Examples of the "aryl group" in the "aryl group which may be substituted" represented by J include C₆₋₁₄ aryl such as phenyl, naphthyl, and the like, preferably C₆₋₁₀ aryl, more preferably phenyl, and the like.

Examples of the "aromatic heterocyclic group" in the "aromatic heterocyclic group which may be substituted" represented by J include those similar to the "aromatic heterocyclic group which may be substituted" in the "heterocyclic group which may be substituted" exemplified with respect to Ra⁶. Among these, preferred is a 5- to 6-membered aromatic monocyclic heterocyclic group which may be substituted. Examples of the 5- to 6-membered aromatic monocyclic heterocyclic group include furan, thiophene, pyrrole, oxazole, isoxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, and the like

The "aromatic cyclic group" in the "aromatic cyclic group which may be substituted" represented by J may be substituted, and examples of the substituent include halogen (for example, fluorine, chlorine, bromine, iodine, and the like), nitro, cyano, hydroxy group, thiol group which may be substituted (for example, thiol, C₁₋₄ alkylthio, and the like), amino group which may be substituted (for example, amino, mono-C₁₋₄ alkylamino, di-C₁₋₄ alkylamino, 5-to 6-membered cyclic amino such as tetrahydropyrrole, piperazine, piperidine, morpholine, thiomorpholine, pyrrole, imidazole, 2-oxo-1-pyrrolidinyl, 2-oxo-1-piperidinyl, and the like), phenyl-lower (C₁₋₄) alkyl, C₃₋₇ cycloalkyl, carboxyl group which may be esterified or amidated (for example, carboxyl, C₁₋₄ alkoxy-carbonyl, lower (C₇₋₁₀) aralkyloxy-carbonyl, carbamoyl, mono-C₁₋₄ alkylcarbamoyl, di-C₁₋₄ alkylcarbamoyl, and the like) , C₁₋₄ alkyl which may be substituted with a halogen atom or C₁₋₄ alkoxy (for example, trifluoromethyl, methyl, ethyl, and the like), C₁₋₄ alkoxy which may be substituted with a halogen atom or C₁₋₄ alkoxy (for example, methoxy, ethoxy, trifluoromethoxy, trifluoroethoxy, and the like), C₁₋₄ alkylenedioxy (for example, -O-CH₂-O-, -O-CH₂-CH₂-O-, and the like), formyl, C₂₋ ₄ alkanoyl (for example, acetyl, propionyl, and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), sulfamoyl group which may be substituted (for example, sulfamoyl, mond-C₁₋₄ alkylsulfamoyl, di-C₁₋₄ alkylsulfamoyl, and the like), an aryl group which may be substituted, a heterocyclic group which may be substituted, and the like. The number of the substituent(s) is preferably 1 to 3.

The "hydrocarbon group" in the "hydrocarbon group which may be substituted" represented by Rb¹, Rb² and Rb³ may be substituted, and examples of the substituent include halogen (for example, fluorine, chlorine, bromine, iodine, and the like), nitro, cyano, oxo, hydroxy group, thiol group which may be substituted (for example, thiol, C₁₋₄ alkylthio, and the like), amino group which may be substituted (for example, amino, mono-C₁₋₄ alkylamino, di-C₁₋ ₄ alkylamino, 5- to 6-membered cyclic amino such as tetrahydropyrrole, piperazine, piperidine, morpholine, thiomorpholine, pyrrole, imidazole, 2-oxo-1-pyrrolidinyl, 2-oxo-1-piperidinyl, and the like), phenyl-lower (C₁₋₄) alkyl, C₃₋₇ cycloalkyl, carboxyl group which may be esterified or amidated (for example, carboxyl, C₁₋₄ alkoxy-carbonyl, lower (C₇₋₁₀) aralkyloxy-carbonyl, carbamoyl, mono-C₁₋₄ alkylcarbamoyl, di-C₁₋₄ alkylcarbamoyl, and the like), C₁₋₄ alkyl which may be substituted with a halogen atom or C₁₋₄ alkoxy (for example, trifluoromethyl, methyl, ethyl, and the like), C₁₋₄ alkoxy which may be substituted with a halogen atom or C₁₋₄ alkoxy (for example, methoxy, ethoxy, trifluoromethoxy, trifluoroethoxy, and the like), C₁₋₄ alkylenedioxy (for example, -O-CH₂-O-, -O-CH₂-CH₂-O-, and the like), formyl, C₂₋₄ alkanoyl (for example, acetyl, propionyl, and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), a sulfamoyl group which may be substituted (for example, sulfamoyl, mono-C₁₋₄ alkylsulfamoyl, di-C₁₋₄ alkylsulfamoyl, and the like), an aryl group which may be substituted, a heterocyclic group which may be substituted, and the like. The number of the substituent(s) is preferably 1 to 3.

Examples of the "aryl group" in the "aryl group which may be substituted" as the substituent of the "hydrocarbon group which may be substituted" represented by Rb¹, Rb² and Rb³ include C₆₋₁₄ aryls such as phenyl, naphthyl, and the like, preferably C₆₋₁₀ aryl, more preferably phenyl, and the like.

Examples of the substituent of said "aryl group" include halogen (for example, fluorine, chlorine, bromine, iodine, and the like), nitro, cyano, hydroxy group, thiol group which may be substituted (for example, thiol, C₁₋₄ alkylthio, and the like), amino group which may be substituted (for example, amino, mono-C₁₋₄ alkylamino, di-C₁₋ ₄ alkylamino, 5- to 6-membered cyclic amino such as tetrahydropyrrole, piperazine, piperidine, morpholine, thiomorpholine, pyrrole, imidazole, and the like), phenyl-lower (C₁₋₄) alkyl, C₃₋₇ cycloalkyl, a carboxyl group which may be esterified or amidated (for example, carboxyl, C₁₋₄ alkoxy-carbonyl, lower (C₇₋₁₀) aralkyloxy-carbonyl, carbamoyl, mono-C₁₋₄ alkylcarbamoyl, di-C₁₋₄ alkylcarbamoyl, and the like), C₁₋₄ alkyl which may be substituted with a halogen atom or C₁₋₄ alkoxy (for example, trifluoromethyl, methyl, ethyl, and the like), C₁₋₄ alkoxy which may be substituted with a halogen atom or C₁₋₄ alkoxy (for example, methoxy, ethoxy, trifluoromethoxy, trifluoroethoxy, and the like), C₁₋₄ alkylenedioxy (for example, -O-CH₂-O-, -O-CH₂-CH₂-O-, and the like), formyl, C₂₋₄ alkanoyl (for example, acetyl, propionyl, and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), a sulfamoyl group which may be substituted (for example, sulfamoyl, mono-C₁₋₄ alkylsulfamoyl, di-C₁₋₄ alkylsulfamoyl, and the like), a 5-to 6-membered aromatic monocyclic heterocyclic group (furan, thiophene, pyrrole, oxazole, isoxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, and the like). The number of the substituent(s) is preferably 1 to 3.

Examples of the "heterocyclic group which may be substituted" as the substituent of the "hydrocarbon group which may be substituted" represented by Rb¹, Rb² and Rb³, include those similar to the "heterocyclic group which may be substituted" represented by the above Rb⁶.

In the above formula, Rb¹ is preferably hydrogen atom or C₁₋₆ alkyl which may be substituted, more preferably, hydrogen atom or C₁₋₄ alkyl, and in particular, hydrogen atom.

In the above formula, the "hydrocarbon group which may be substituted" represented by Rb² is preferably a group represented by the formula X'''-G-(CH₂)ₙ-J, wherein X''' is a C₁₋₄ alkylene group or a C₁₋₄ alkenylene group, G is a bonding hand -O-, -S-, -CO-NH- or -NH-CO-, n is an integer of 0 to 3, and J is an aromatic cyclic group which may be substituted. Preferred examples of the aromatic cyclic group which may be substituted represented by J include phenyl which may be substituted, a 5- to 6-membered aromatic monocyclic heterocyclic group which may be substituted, and the like.

In the above formula, the "hydrocarbon group which may be substituted" represented by Rb³ is preferably C₁₋₆ alkyl which may be substituted, and in particular, a group represented by the formula -(CH₂)ₚ-K, wherein p is an integer of 1 to 6, and K is an aromatic cyclic group which may be substituted.

Examples of the "aromatic cyclic group which may be substituted" represented by K include those similar to the "aromatic cyclic group which may be substituted" represented by the fore-mentioned J, and the "aromatic cyclic group" in the "aromatic cyclic group which may be substituted" represented by K is preferably phenyl group. The substituent of the "aromatic cyclic group" in the "aromatic cyclic group which may be substituted" represented by K is preferably hydroxy group, sulfamoyl group which may be substituted, and the like.

Further, in the above formula, when Rb¹ and Xb are bonded to form a ring, "the ring" may be either of a saturated ring or an unsaturated ring so far as it is a nitrogen-containing heterocyclic ring, and the size of the ring is not specifically limited, but among these, preferred is a 3- to 8-membered nitrogen-containing heterocyclic ring, in particular, a 3- to 8-membered saturated nitrogen-containing heterocyclic ring, namely a ring represented by the formula: 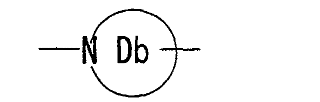 wherein ring Db is a 3- to 8-membered saturated nitrogen-containing heterocyclic ring.

Examples of the "3- to 8-membered nitrogen-containing heterocyclic ring" include a 3- to 8-membered nitrogen-containing heterocyclic ring which contains one nitrogen atom and may contain 1 to 4 (preferably 1 to 2) of one to three kinds (preferably, one to two kinds) hetero atoms selected from oxygen atom, sulfur atom and nitrogen atom, and the like. More specifically, examples thereof include a 3- to 8-membered (preferably, 5- to 6-membered) saturated or unsaturated (preferably, saturated) monocyclic non-aromatic heterocyclic ring (aliphatic heterocyclic ring) such as pyrrolidine, pyrroline, imidazolidine, imidazoline, pyrazolidine, pyrazoline, oxazine, oxadiazine, thiazine, thiadiazine, piperidine, morpholine, thiomorpholine, piperazine, azepine, and the like.

Further, said "3- to 8-membered nitrogen-containing heterocyclic ring" may be substituted, and examples of the substituent include those similar to the substituent of the "hydrocarbon group which may be substituted" as the substituent of the benzene ring in the "benzene ring which may be further substituted" represented by the above B or C.

Further, in the above formula, Rb¹ may be bonded to the "amino group which may be substituted" represented by Ab to form a ring, and the "ring" may be either of a saturated ring and an unsaturated ring so far as it is a heterocyclic ring containing at least two nitrogen atoms, and the size of the ring is not limited. Among these, a 3-to 8-membered nitrogen-containing heterocyclic ring is preferred, and in particular, a 3- to 8-membered saturated nitrogen-containing heterocyclic ring, namely a ring represented by the formula: 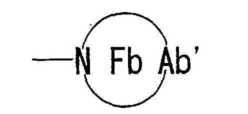 wherein Ab' is nitrogen atom which may be substituted, and ring Fb is a 3- to 8-membered saturated nitrogen-containing heterocyclic ring, is preferred.

In the above formula, examples of the substituent of the "nitrogen atom" in the "nitrogen atom which may be substituted" represented by Ab' include those similar to the substituent of the "amino group" in the "amino group which may be substituted" represented by Ab hereinafter.

Examples of the "3- to 8-membered nitrogen-containing heterocyclic ring" include a 3- to 8-membered nitrogen-containing heterocyclic ring which contains two nitrogen atoms and further 1 to 4 (preferably 1 to 2) of one to three kinds (preferably, one to two kinds) of hetero atoms selected from oxygen atom, sulfur atom and nitrogen atom, and the like. More specifically, examples thereof include a 3- to 8-membered (preferably, 5- to 6-membered) saturated or unsaturated (preferably, saturated) monocyclic non-aromatic heterocyclic ring (aliphatic heterocyclic ring) such as imidazolidine, imidazoline, pyrazolidine, pyrazoline, oxadiazine, thiadiazine, piperazine, diazepine, and the like.

Further, said "3- to 8-membered nitrogen-containing heterocyclic ring" may be substituted, and examples of the substituent include those similar to the substituent of the "hydrocarbon group which may be substituted" as the substituent which the benzene ring in the "benzene ring which may be further substituted" represented by the above Bb or Cb.

In the above formula, "the spacer in which the number of atom(s) constituting a linear chain portion is 1 to 8" represented by Xb may be any one so far as it is "the divalent group in which the number of atom(s) in a linear chain portion is 1 to 8", and examples thereof include a saturated divalent group and a divalent group in which a part of bonds is converted to unsaturated bond(s), such as
(1) -(CH₂)_{f2}- (f2 is an integer of 1 to 8, preferably an integer of 1 to 6 and more preferably an integer of 1 to 4),
(2) -(CH₂)_{g3}-X³-(CH₂)_{g4}- (g3 and g4 are the same or different and are an integer of 0 to 7, provided that the sum of g3 and g4 is 0 to 8. X³ is NH, O, S, SO or SO₂),
(3) -(CH₂)ₕ₄-X³-(CH₂)ₕ₅- X⁴-(CH₂)ₕ₆- (h4, h5 and h6 are the same or different and are an integer of 0 to 6, provided that the sum of h4, h5 and h6 is 0 to 6. Each of X³ and X⁴ is NH, O, S, SO or SO₂). However, when h5 is 0, at least one of X³ and X⁴ is preferably NH). Specific examples include a divalent group such as -O-(CH₂)ₖ₅- (k5 is an integer of 0 to 7), -(CH₂)ₖ₅-O- (k5 is an integer of 0 to 7), -S-(CH₂)ₖ₅- (k5 is an integer of 0 to 7), -(CH₂)ₖ₅-S- (k5 is an integer of 0 to 7), -NH-(CH₂)ₖ₅- (k5 is an integer of 0 to 7), -(CH₂)ₖ₅-NH- (k5 is an integer of 0 to 7), -(CH₂)ₖ₆-(k6 is an integer of 1 to 8), -CH=CH-, -C≡C-, -CO-NH-, -SO₂-NH-, and the like.

As Xb, more preferred one is a divalent group in which the number of carbon atom(s) constituting a linear chain portion is 1 to 4. Among these, C₁₋₄ alkylene, C₂₋₄ alkenylene, and the like are preferred and, in particular, C₁₋₄ alkylene is preferably used.

The divalent group as Xb may be substituted at an arbitrary position (preferably, on a carbon atom), and the substituent may be any one so far as it can be bonded to a divalent chain constituting a linear chin portion. Examples thereof include those similar to the substituent of the "benzene ring" in the "benzene ring which may be further substituted" represented by the above Bb and Cb may, oxo group, and the like. The substituent(s) are the same or different and the number thereof is 1 to 4 (preferably, 1 to 2). They may be substituted at any position of said divalent group. Further, the substituents of the divalent group as Xb may be bonded to each other to form a ring, and examples of "the ring" include C₅₋₇ cycloalkanes such as cyclopentane, cyclohexane, cycloheptane, and the like; benzene; and the like.

Examples of the preferred substituent of the divalent group as Xb include lower (C₁₋₆) alkyl (for example, methyl, ethyl, propyl, isopropyl, butyl, isobutyl, sec-butyl, tert-butyl, pentyl, isopentyl, neopentyl, hexyl, and the like), lower (C₃₋₇) cycloalkyl (for example, cyclopropyl, cyclobutyl, cyclopentyl, cyclohexyl and cycloheptyl, and the like), formyl, lower (C₂₋₇) alkanoyl (for example, acetyl, propionyl, butyryl, and the like), lower (C₁₋₆) alkoxy-carbonyl, lower (C₁₋₆) alkoxy, a hydroxy group, oxo, and the like.

As Xb, in particular, a chain spacer is preferred.

In the above formula, examples of the "amino group which may be substituted" represented by Ab include amino group, and the like which may have 1 or 2 substituents selected from the "hydrocarbon group which may be substituted" (that similar to "the hydrocarbon group which may be substituted" as the substituent of the benzene ring in the "benzene ring which may be further substituted" represented by the above Bb and Cb, and the like), the "heterocyclic group which may be substituted" (that similar to the "heterocyclic group which may be substituted" as the substituent of the benzene ring in the "benzene ring which may be further substituted" represented by the above Bb and Cb, and the like), and the "acyl group which may be substituted" (that similar to the "acyl group which may be substituted" as the substituent of the benzene ring in the "benzene ring which may be further substituted" represented by the above Bb and Cb, and the like). The substituents of the "amino group which may be substituted" represented by Ab may bond to each other to form a cyclic amino group (for example, a cyclic amino group having a bonding hand on nitrogen atom which is formed by eliminating one hydrogen atom from the nitrogen atom constituting the ring of 5- to 6-membered rings such as tetrahydropyrrole, piperazine, piperidine, morpholine, thiomorpholine, pyrrole and imidazole, and the like). Said cyclic amino group may be substituted and examples of the substituent include halogen (for example, fluorine, chlorine, bromine, iodine, and the like), nitro, cyano, hydroxy group, thiol group, amino group, carboxyl group, C₁₋₄ alkyl which may be halogenated (for example, trifluoromethyl, methyl, ethyl, and the like), C₁₋₄ alkoxy which may be halogenated (for example, methoxy, ethoxy, propoxy, butoxy, trifluoromethoxy, trifluoroethoxy, and the like), formyl, C₂₋₄ alkanoyl (for example, acetyl, propionyl, and the like), C₁₋₄ alkylsulfonyl (for example, methanesulfonyl, ethanesulfonyl, and the like), and the like, and the number of the substituent(s) is preferably 1 to 3.

Preferred examples of the substituent of the amino group in the "amino group which may be substituted" represented by Ab include
(1) alkyl which may be substituted (for example, C₁₋₁₀ alkyls such as methyl, ethyl, propyl, isopropyl, butyl, isobutyl, sec-butyl, tert-butyl, pentyl, isopentyl, neopentyl, hexyl, heptyl, octyl, nonyl, decyl, and the like preferably lower (C₁₋₆) alkyl, and the like);
(2) cycloalkyl which may be substituted (for example, C₃₋₈ cycloalkyls such as cyclopropyl, cyclobutyl, cyclopentyl, cyclohexyl, cycloheptyl, cyclooctyl, and the like); further, said cycloalkyl may be condensed with benzene ring to form indan (for example, indan-1-yl, indan-2-yl, and the like), tetrahydronaphthalene (for example, tetrahydronaphthalen-5-yl, tetrahydronaphthalen-6-yl, and the like), and the like (preferably, indan, and the like); further, said cycloalkyl may be crosslinked through a linear atomic chain having 1 to 2 carbons to form crosslinked cyclic hydrocarbon residual groups such as bicyclo[2,2,1]heptyl, bicyclo[2,2,2]octyl, bicyclo[3,2,1]octyl, and bicyclo[3,2,2]nonyl (preferably, cyclohexyl having crosslink through a linear atomic chain having 1 to 2 carbons, and the like, and further preferably, bicyclo[2,2,1]heptyl, and the like);
(3) alkenyl which may be substituted (for example, C₂₋₁₀ alkenyl such as allyl, crotyl, 2-pentenyl, 3-hexenyl, and the like, preferably lower (C₂₋₆) alkenyl, and the like);
(4) cycloalkenyl which may be substituted (for example, C₃₋₇ cycloalkenyl such as 2-cyclopentenyl, 2-cyclohexenyl, 2-cyclopentenylmethyl, 2-cyclohexenylmethyl, and the like);
(5) aralkyl which may be substituted (for example, phenyl-C₁₋₄ alkyl (for example, benzyl, phenethyl, and the like), and the like);
(6) formyl or acyl which may be substituted (for example, C₂₋₄ alkanoyl (for example, acetyl, propionyl, butyryl, isobutyryl, and the like), C₁₋₄ alkylsulfonyl having (for example, methanesulfonyl, ethanesulfonyl, and the like);
(7) aryl which may be substituted (for example, phenyl, naphthyl, and the like);
(8) a heterocyclic group which may be substituted (for example, a group formed by eliminating one hydrogen atom from a 5- to 6-membered aromatic heterocyclic ring which contains 1 to 4 hetero atoms of 1 to 2 kinds selected from nitrogen atom, sulfur atom and oxygen atom such as furan, thiophene, pyrrole, imidazole, pyrazole, thiazole, oxazole, isothiazole, isoxazole, tetrazole, pyridine, pyrazine, pyrimidine, pyridazine, triazole, and the like, a group formed by eliminating one hydrogen atom from a 5- to 6-membered non-aromatic heterocyclic ring which contains 1 to 4 hetero atoms of 1 to 2 kinds selected from nitrogen atom, sulfur atom and oxygen atom such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, and the like).

Preferred examples of the substituent of the above (1) alkyl which may be substituted, (2) cycloalkyl which may be substituted, (3) alkenyl which may be substituted, (4) cycloalkenyl which may be substituted, (5) aralkyl which may be substituted, (6) acyl which may be substituted, (7) aryl which may be substituted and (8) a heterocyclic group which may be substituted include halogen (for example, fluorine, chlorine, bromine, iodine, and the like), C₁₋₄ alkyl which may be substituted with a halogen atom or C₁₋₄ alkoxy, C₁₋₄ alkoxy which may be substituted with a halogen atom or C₁₋₄ alkoxy (for example, methoxy, ethoxy, propoxy, butoxy, trifluoromethoxy, trifluoroethoxy, and the like), C₁₋₄ alkylenedioxy (for example, -O-CH₂-O-, -O-CH₂-CH₂-O-, and the like), formyl, C₂₋₄ alkanoyl (for example, acetyl, propionyl, and the like), C₁₋₄ alkylsulfonyl (for example, methanesulfonyl, ethanesulfonyl, and the like), phenyl-lower (C₁₋₄) alkyl, C₃₋₇ cycloalkyl, cyano, nitro, a hydroxy group, a thiol group which may be substituted (for example, thiol, C₁₋₄ alkylthio, and the like), amino group which may be substituted (for example, amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, and the like), a carboxyl group, lower (C₁₋₄) alkoxy-carbonyl, lower (C₇₋ ₁₀)aralkyloxy-carbonyl, carbamoyl, mono-C₁₋₄ alkylcarbamoyl, di-C₁₋₄ alkylcarbamoyl (preferably, halogen, lower (C₁₋₄ ) alkyl which may be halogenated, lower (C₁₋₄ ) alkoxy which may be halogenated, phenyl-lower (C₁₋₄ ) alkyl, C₃₋₇ cycloalkyl, cyano, hydroxy group, and the like), and the number of the substituent(s) is preferably 1 to 3.

Preferred examples of the "amino group which may be substituted" represented by Ab include, in particular, amino group which may have 1 to 2 alkyls which may be substituted [C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower (C₁₋₆) alkyl, and the like, each of which may have 1 to 3 substituents selected from, for example, halogen (for example, fluorine, chlorine, bromine, iodine, and the like), nitro, cyano, a hydroxy group, a thiol group which may be substituted (for example, thiol, C₁₋₄ alkylthio, and the like), an amino group which may be substituted (for example, amino, mono-C₁₋₄ alkylamino, di-C₁₋₄ alkylamino, 5-to 6-membered cyclic amino such as tetrahydropyrrole, piperazine, piperidine, morpholine, thiomorpholine, pyrrole, imidazole, and the like), a carboxyl group which may be esterified or amidated (for example, carboxyl, C₁₋₄ alkoxycarbonyl, lower (C₇₋₁₀)aralkyloxy-carbonyl, carbamoyl, mono-C₁₋₄ alkylcarbamoyl, di-C₁₋₄ alkylcarbamoyl, and the like), C₁₋₄ alkyl which may be substituted with a halogen atom or C₁₋₄ alkoxy (for example, trifluoromethyl, methyl, ethyl, and the like), C₁₋₄ alkoxy which may be substituted with a halogen atom or C₁₋₄ alkoxy (for example, methoxy, ethoxy, trifluoromethoxy, trifluoroethoxy, and the like), C₁₋₄ alkylenedioxy (for example, -O-CH₂-O-, -O-CH₂-CH₂-O-, and the like), phenyl-lower (C₁₋₄ ) alkyl, C₃₋₇ cycloalkyl, formyl, C₂₋₄ alkanoyl (for example, acetyl, propionyl, and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), and the like.

In the above formula, examples of the "nitrogen-containing heterocyclic group" in the "nitrogen-containing heterocyclic group which may be substituted" represented by Ab include a 5- to 8-membered aromatic monocyclic heterocyclic ring which contains one nitrogen atom and may contain 1 to 4 (preferably 1 to 2) of one to three kinds (preferably one to two kinds) of hetero atoms selected from oxygen atom, sulfur atom and nitrogen atom; a saturated or unsaturated non-aromatic monocyclic heterocyclic ring (aliphatic heterocyclic ring), and the like; and a group formed by eliminating one hydrogen atom from a ring in which 2 to 3 the same or different rings selected from these monocyclic rings are condensed. Further, "the nitrogen-containing heterocyclic group which may be substituted" represented by Ab may be bonded to Xb through either of nitrogen atom and carbon atom. Preferably, it is bonded to Xb through carbon atom.

Examples of the "aromatic monocyclic heterocyclic ring" used herein include a 5- to 8-membered (preferably, 5- to 6-membered) aromatic monocyclic heterocyclic ring (for example, pyrrole, oxazole, isoxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, and the like), and the like. Examples of the "non-aromatic monocyclic heterocyclic ring" include a 5- to 8-membered (preferably, 5- to 6-membered) saturated or unsaturated monocyclic non-aromatic heterocyclic ring (aliphatic heterocyclic ring) such as pyrrolidine, pyrroline, imidazolidine, imidazoline, pyrazolidine, pyrazoline, oxazine, oxadiazine, thiazine, thiadiazine, piperidine, morpholine, thiomorpholine, piperazine, and azepine, or 5- to 8-membered non-aromatic heterocyclic rings in which a part or all of double bonds of the above aromatic monocyclic heterocyclic ring are saturated, and the like.

Examples of the substituent of the "nitrogen-containing heterocyclic group" in the "nitrogen-containing heterocyclic group which may be substituted" represented by Ab include those similar to the substituent of the " hydrocarbon group which may be substituted" as the substituent of the benzene ring in the "benzene ring which may be further substituted" represented by the above Bb or Cb.

The "nitrogen-containing heterocyclic group" in the "nitrogen-containing heterocyclic group which may be substituted" represented by Ab is preferably a 5- to 6-membered nitrogen-containing heterocyclic group, more preferably a 5- to 6-membered saturated nitrogen-containing heterocyclic group, in particular, pyrrolidine, piperidine, piperazine (preferably, a 5- to 6-membered saturated nitrogen-containing heterocyclic group having one nitrogen atom), and the like.

In the above formula, preferred examples of the group represented by the formula: 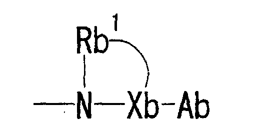 include a group represented by the formula: 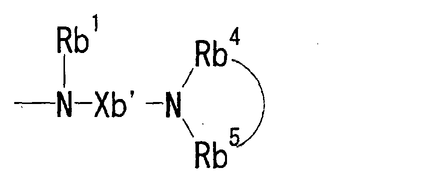 wherein Rb¹ is as defined above, Xb¹ is a C₁₋₆ alkylene group which may be substituted, each of Rb⁴ and Rb⁵ is a hydrogen atom or a C₁₋₆ alkyl group which may be substituted, and Rb⁴ and Rb⁵ may be bonded to each other to form a ring, and
a group represented by the formula: 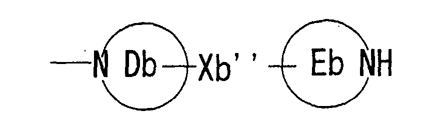 wherein Xb'' is a bonding hand or a C₁₋₄ alkylene group which may be substituted, each of ring Db and ring Eb is a 3- to 8-membered saturated nitrogen-containing heterocyclic group], and the like.

In the above formula, examples of the substituent of the "C₁₋₆ alkylene group (preferably, a C₁₋₄ alkylene group)" in the "C₁₋₆ alkylene group which may be substituted" represented by Xb' include those similar to the substituent of the divalent bond as Xb.

In the above formula, examples of the "C₁₋₆ alkyl group which may be substituted" represented by Rb⁴ and Rb⁵ include lower (C₁₋₆) alkyl such as methyl, ethyl, propyl, isopropyl, butyl, isobutyl, sec-butyl, tert-butyl, pentyl, isopentyl, neopentyl, hexyl, and the like each of which may have 1 to 3 substituents selected from, for example, halogen (for example, fluorine, chlorine, bromine, iodine, and the like), nitro, cyano, hydroxy group, thiol group which may be substituted (for example, thiol, C₁₋₄ alkylthio, and the like), an amino group which may be substituted (for example, amino, mono-C₁₋₄ alkylamino, di-C₁₋₄ alkylamino, 5-to 6-membered cyclic amino such as tetrahydropyrrole, piperazine, piperidine, morpholine, thiomorpholine, pyrrole, imidazole, and the like), carboxyl group which may be esterified or amidated (for example, carboxyl, C₁₋₄ alkoxycarbonyl, lower (C₇₋₁₀)aralkyloxy-carbonyl, carbamoyl, mono-C₁₋₄ alkylcarbamoyl, di-C₁₋₄ alkylcarbamoyl, and the like), C₁₋₄ alkyl which may be substituted with a halogen atom or C₁₋₄ alkoxy (for example, trifluoromethyl, methyl, ethyl, and the like), C₁₋₄ alkoxy which may be substituted with a halogen atom or C₁₋₄ alkoxy (for example, methoxy, ethoxy, trifluoromethoxy, trifluoroethoxy, and the like), C₁₋₄ alkylenedioxy (for example, -O-CH₂-O-, -O-CH₂-CH₂-O-, and the like), phenyl-lower (C₁₋₄ ) alkyl, C₃₋₇ cycloalkyl, formyl, C₂₋₄ alkanoyl (for example, acetyl, propionyl and the like), C₁₋₄ alkylsulfonyl (for example, methanesulfonyl, ethanesulfonyl, and the like), C₁₋₄ alkylsulfinyl (for example, methanesulfinyl, ethanesulfinyl, and the like), and the like.

In the above formula, Rb⁴ and Rb⁵ may be bonded to each other and together with the adjacent nitrogen atom to form a cyclic amino group (for example, a cyclic amino group formed by eliminating one hydrogen atom from the ring-constituting nitrogen atom of a 5- to 6-membered ring such as tetrahydropyrrole, piperazine, piperidine, morpholine, thiomorpholine, pyrrole and imidazole, and which has a bonding hand on nitrogen atom, and the like; preferably 5- to 6-membered saturated cyclic amino groups such as pyrrolidino, piperazino, piperidino, and the like; and more preferably pyrrolidino, and the like). Said cyclic amino group may be substituted, and examples of the substituent include halogen (for example, fluorine, chlorine, bromine, iodine, and the like), nitro, cyano, hydroxy group, thiol group, amino group, carboxyl group, C₁₋₄ alkyl which may be halogenated (for example, trifluoromethyl, methyl, ethyl, and the like), C₁₋₄ alkoxy which may be halogenated (for example, methoxy, ethoxy, propoxy, butoxy, trifluoromethoxy, trifluoroethoxy, and the like), formyl, C₂₋₄ alkanoyl (for example, acetyl, propionyl, and the like), C₁₋₄ alkylsulfonyl (for example, methanesulfonyl, ethanesulfonyl, and the like), and the like. The number of the substituent(s) is preferably 1 to 3.

In the above formula, examples of the substituent of the "C₁₋₄ alkylene group" in the "C₁₋₄ alkylene group which may be substituted" represented by Xb'' include those similar to the substituent of the divalent group as Xb.

In the above formula, examples of the "3- to 8-membered saturated nitrogen-containing heterocyclic ring" represented by ring Db and ring Eb include a 3- to 8-membered nitrogen-containing heterocyclic ring which contains one nitrogen atom and may contain 1 to 4 (preferably 1 to 2) of one to three kinds (preferably one to two kinds) of hetero atoms selected from oxygen atom, sulfur atom and nitrogen atom, and the like. More specifically, examples thereof include a 3- to 8-membered (preferably, 5- to 6-membered) saturated or unsaturated (preferably, saturated) monocyclic non-aromatic heterocyclic ring (aliphatic heterocyclic ring) such as pyrrolidine, pyrroline, imidazolidine, imidazoline, pyrazolidine, pyrazoline, oxazine, oxadiazine, thiazine, thiadiazine, piperidine, morpholine, thiomorpholine, piperazine, azepine, etc.

Further, said "3- to 8-membered nitrogen-containing heterocyclic ring" may be substituted, and examples of the substituent include those similar to the substituent of the "hydrocarbon group which may be substituted" as the substituent of the benzene ring in the "benzene ring which may be further substituted" represented by the above Bb or Cb.

Further, the "3- to 8-membered nitrogen-containing heterocyclic ring" represented by ring Db and ring Eb may be bonded to Xb'' through either of nitrogen atom or carbon atom, with bonding to Xb'' through carbon atom being preferred.

In the above formula, the group specifically shown as the substituent of ring Bb and ring Cb may be substituted at any possible position. However, preferably, the compound represented by the formula (Ib) or a salt thereof may have any one of structures represented by the formula: 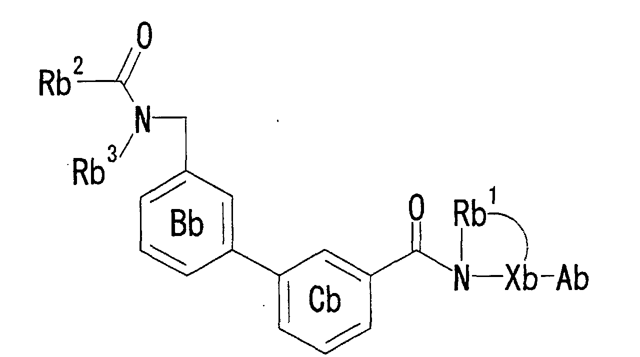 wherein each symbol is as defined above,
the formula: 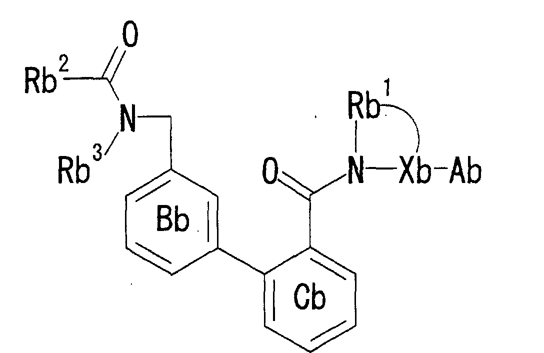 wherein each symbol is as defined above, or
the formula 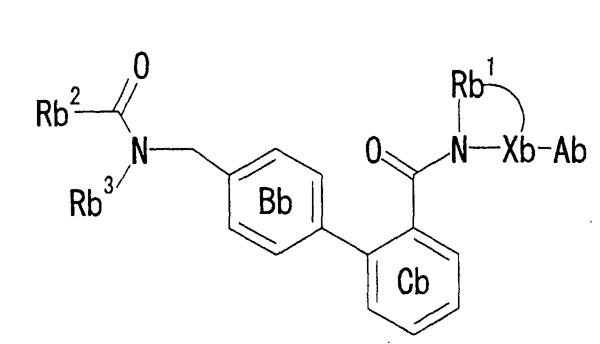 wherein each symbol is as defined above.

Among the compound represented by the formula (Ib), 3'-{[{2-[4-(aminosulfonyl)phenyl]ethyl}(4-phenylbutanoyl)amino]methyl}-N-[2-(1-pyrrolidinyl)ethyl][1,1'-biphenyl]-3-carboxamide, 3'-({{2-[4-(aminosulfonyl)phenyl]ethyl}[(benzyloxy)acetyl] amino}methyl)-N-[2-(1-pyrrolidinyl)ethyl][1,1'-biphenyl]-3-carboxamide, and the like are preferably used.

In the above formula, Ar is the "aryl group which may be substituted".

Examples of the "substituent" of said "aryl group which may be substituted" include (i) lower alkyl which may be halogenated, (ii) a halogen atom (for example, fluorine, chlorine, bromine, iodine, and the like), (iii) a lower alkylenedioxy group (for example, C₁₋₃ alkylenedioxy group such as methylenedioxy, ethylenedioxy, and the like), (iv) nitro group, (v) cyano group, (vi) hydroxy group, (vii) a lower alkoxy group which may be halogenated, (viii) a lower cycloalkyl group (for example, C₃₋₆ cycloalkyl group such as cyclopropyl, cyclobutyl, cyclopentyl, cyclohexyl, and the like), (ix) a lower alkylthio group which may be halogenated, (x) amino group, (xi) a mono-lower alkylamino group (for example, mono-C₁₋₆ alkylamino group such as methylamino, ethylamino, propylamino, and the like), (xii) a di-lower alkylamino group (for example, di-C₁₋₆ alkylamino group such as dimethylamino, diethylamino, and the like), (xiii) a 5- to 7-membered cyclic amino group which may have 1 to 3 hetero atoms selected from, for example, nitrogen atom, oxygen atom or sulfur atom, and the like in addition to one nitrogen atom (for example, pyrrolidino, piperidino, piperazino, morpholino, thiomorpholino, and the like), (xiv) a lower alkyl-carbonylamino group (for example, C₁₋₆ alkyl-carbonylamino group such as acetylamino, propionylamino, butyrylamino, and the like), (xv) aminocarbonyloxy group, (xvi) a mono-lower alkylamino-carbonyloxy group (for example, mono-C₁₋₆ alkylamino-carbonyloxy group such as methylaminocarbonyloxy, ethylaminocarbonyloxy, and the like), (xvii) a di-lower alkylamino-carbonyloxy group (for example, di-C₁₋₆ alkylamino-carbonyloxy group such as dimethylaminocarbonyloxy, diethylaminocarbonyloxy, and the like), (xviii) a lower alkylsulfonylamino group (for example, C₁₋₆ alkylsulfonylamino groups such as methylsulfonylamino, ethylsulfonylamino, propylsulfonylamino, and the like), (xix) a lower alkoxy-carbonyl group (for example, C₁₋₆ alkoxy-carbonyl group such as methoxycarbonyl, ethoxycarbonyl, propoxycarbonyl and isobutoxycarbonyl, and the like), (xx) carboxyl group, (xxi) a lower alkyl-carbonyl group (for example, C₁₋₆ alkyl-carbonyl groups such as methylcarbonyl, ethylcarbonyl, butylcarbonyl, and the like), (xxii) a lower cycloalkyl-carbonyl group (for example, C₃₋₆ cycloalkyl-carbonyl group such as cyclopropylcarbonyl, cyclobutylcarbonyl, cyclopentylcarbonyl, cyclohexylcarbonyl, and the like), (xxiii) carbamoyl group, (xxiv) a mono-lower alkyl-carbamoyl group (for example, mono-C₁₋₆ alkyl-carbamoyl group such as methylcarbamoyl, ethylcarbamoyl, propylcarbamoyl, butylcarbamoyl, and the like), (xxv) a di-lower alkyl-carbamoyl group (for example, di-C₁₋₆ alkyl-carbamoyl group such as diethylcarbamoyl, dibutylcarbamoyl, and the like), (xxvi) a lower alkylsulfonyl group (for example, C₁₋₆ alkylsulfonyl group such as methylsulfonyl, ethylsulfonyl, propylsulfonyl, and the like), (xxvii) a lower cycloalkylsulfonyl group (for example, C₃₋₆ cycloalkylsulfonyl group such as cyclopentylsulfonyl, cyclohexylsulfonyl, and the like), (xxviii) phenyl group, (xxix) naphthyl group, (xxx) a mono-phenyl-lower alkyl group (for example, mono-phenyl-C₁₋₆ alkyl group such as benzyl, phenylethyl, and the like), (xxxi) a di-phenyl-lower alkyl group (for example, di-phenyl-C₁₋₆ alkyl group such as diphenylmethyl, diphenylethyl, and the like), (xxxii) a mono-phenyl-lower alkyl-carbonyloxy group (for example, mono-phenyl-C₁₋₆ alkyl-carbonyloxy group such as phenylmethylcarbonyloxy, phenylethylcarbonyloxy, and the like), (xxxiii) a di-phenyl-lower alkyl-carbonyloxy group (for example, di-phenyl-C₁₋₆ alkyl-carbonyloxy group such as diphenylmethylcarbonyloxy, diphenylethylcarbonyloxy, and the like), (xxxiv) phenoxy group, (xxxv) a mono-phenyl-lower alkyl-carbonyl group (for example, mono-phenyl-C₁₋₆ alkyl-carbonyl group such as phenylmethylcarbonyl, phenylethylcarbonyl, and the like), (xxxvi) a di-phenyl-lower alkyl-carbonyl group (for example, di-phenyl-C₁₋₆ alkyl-carbonyl group such as diphenylmethylcarbonyl, diphenylethylcarbonyl, and the like), (xxxvii) benzoyl group, (xxxviii) phenoxycarbonyl group, (xxxix) a phenyl-lower alkyl-carbamoyl group (for example, phenyl-C₁₋₆ alkyl-carbamoyl group such as phenyl-methylcarbamoyl, phenyl-ethylcarbamoyl, and the like), (xxxx) phenylcarbamoyl group, (xxxxi) a phenyl-lower alkyl-carbonylamino group (for example, phenyl-C₁₋₆ alkyl-carbonylamino group such as phenyl-methylcarbonylamino, phenyl-ethylcarbonylamino, and the like), (xxxxii) a phenyl-lower alkylamino group (for example, phenyl-C₁₋₆ alkylamino group such as phenyl-methylamino, phenyl-ethylamino, and the like), (xxxxiii) a phenyl-lower alkylsulfonyl group (for example, phenyl-C₁₋₆ alkylsulfonyl group such as phenyl-methylsulfonyl, phenyl-ethylsulfonyl, and the like), (xxxxiv) phenylsulfonyl group, (xxxxv) a phenyl-lower alkylsulfinyl group (for example, phenyl-C₁₋₆ alkylsulfinyl group such as phenyl-methylsulfinyl, phenyl-ethylsulfinyl, and the like), (xxxxvi) a phenyl-lower alkylsulfonylamino group (for example, phenyl-C₁₋₆ alkylsulfonylamino group such as phenyl-methylsulfonylamino, phenyl-ethylsulfonylamino, and the like), (xxxxvii) a phenylsulfonylamino group [said (xxviii) phenyl group, (xxix) naphthyl group, (xxx) a mono-phenyl-lower alkyl group, (xxxi) a di-phenyl-lower alkyl group, (xxxii) a mono-phenyl-lower alkyl-carbonyloxy group, (xxxiii) a di-phenyl-lower alkyl-carbonyloxy group, (xxxiv) a phenoxy group, (xxxv) a mono-phenyl-lower alkyl-carbonyl group, (xxxvi) a di-phenyl-lower alkyl-carbonyl group, (xxxvii) a benzoyl group, (xxxviii) a phenoxycarbonyl group, (xxxix) a phenyl-lower alkyl-carbamoyl group, (xxxx) a phenylcarbamoyl group, (xxxxi) a phenyl-lower alkyl-carbonylamino group, (xxxxii) a phenyl-lower alkylamino group, (xxxxiii) a phenyl-lower alkylsulfonyl group, (xxxxiv) a phenylsulfonyl group, (xxxxv) a phenyl-lower alkylsulfinyl group, (xxxxvi) a phenyl-lower alkylsulfonylamino group, (xxxxvii) and a phenylsulfonylamino group may further have 1 to 4 substituent(s) selected from, for example, lower alkyl (for example, C₁₋₆ alkyl such as methyl, ethyl, propyl, isopropyl, butyl, sec-butyl, tert-butyl, pentyl, hexyl, and the like), lower alkoxy (for example, C₁₋₆ alkoxy such as methoxy, ethoxy, propoxy, isopropoxy, n-butoxy, isobutoxy, sec-butoxy, tert-butoxy, and the like), a halogen atom (for example, chlorine, bromine, iodine, and the like), hydroxy, benzyloxy, amino, a mono-lower alkylamino group (for example, mono-C₁₋₆ alkylamino such as methylamino, ethylamino, propylamino, and the like), a di-lower alkylamino group (for example, di-C₁₋₆ alkylamino such as. dimethylamino, diethylamino, and the like), nitro, lower alkyl-carbonyl (for example, C₁₋₆ alkyl-carbonyl such as methylcarbonyl, ethylcarbonyl, butylcarbonyl, and the like), benzoyl, and the like], and the like.

Examples of the "lower alkyl group which may be halogenated" include a lower alkyl group (for example, C₁₋₆ alkyl group such as methyl, ethyl, propyl, isopropyl, butyl, sec-butyl, tert-butyl, pentyl, hexyl, and the like) which may have, for example, 1 to 3 halogen atoms (for example, chlorine, bromine, iodine, and the like). Specific examples thereof include methyl, chloromethyl, difluoromethyl, trichloromethyl, trifluoromethyl, ethyl, 2-bromoethyl, 2,2,2-trifluorethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl, etc.

Examples of the "lower alkoxy group which may be halogenated" include a lower alkoxy group (for example, C₁₋₆ alkoxy group such as methoxy, ethoxy, propoxy, isopropoxy, n-butoxy, isobutoxy, sec-butoxy, tert-butoxy, and the like) which may have, for example, 1 to 3 halogen atoms (for example, chlorine, bromine, iodine, and the like). Specific examples thereof include methoxy, difluoromethoxy, trifluoromethoxy, ethoxy, 2,2,2-trifluorethoxy, n-propoxy, isopropoxy, n-butoxy, 4,4,4-trifluorobutoxy, isobutoxy, sec-butoxy, pentyloxy, hexyloxy, etc.

Examples of the "lower alkylthio group which may be halogenated" include a lower alkylthio group (for example, C₁₋₆ alkylthio group such as methylthio, ethylthio, n-propylthio, isopropylthio, n-butylthio, isobutylthio, sec-butylthio, tert-butylthio, and the like) which may have, for example, 1 to 3 halogen atoms (for example, chlorine, bromine, iodine, and the like). Specific examples thereof include methylthio, difluoromethylthio, trifluoromethylthio, ethylthio, n-propylthio, isopropylthio, n-butylthio, 4,4,4-trifluorobutylthio, isobutylthio, sec-butylthio, tert-butylthio, pentylthio, hexylthio, etc.

Preferred examples of the "substituent" of the "aryl group which may be substituted" include (i) amino group, (ii) a mono-lower alkylamino group (for example, mono-C₁₋₆ alkylamino group such as methylamino, ethylamino, propylamino, and the like), (iii) a di-lower alkylamino group (for example, di-C₁₋₆ alkylamino group such as dimethylamino, diethylamino, and the like), (iv) a 5- to 7-membered cyclic amino group which may have 1 to 3 of hetero atoms selected from, for example, nitrogen atom, oxygen atom or sulfur atom, and the like in addition to one nitrogen atom (for example, pyrrolidino, piperidino, piperazino, morpholino, thiomorpholino, and the like), (v) a lower alkyl-carbonylamino group (for example, C₁₋₆ alkyl-carbonylamino group such as acetylamino, propionylamino, butyrylamino, and the like), (vi) aminocarbonyloxy group, (vii) a mono-lower alkylamino-carbonyloxy group (for example, mono-C₁₋₆ alkylamino-carbonyloxy group such as methylaminocarbonyloxy, ethylaminocarbonyloxy, and the like), (viii) a di-lower alkylamino-carbonyloxy group (for example, di-C₁₋₆ alkylamino-carbonyloxy group such as dimethylaminocarbonyloxy, diethylaminocarbonyloxy, and the like), (ix) a lower alkylsulfonylamino group (for example, C₁₋₆ alkylsulfonylamino group such as methylsulfonylamino, ethylsulfonylamino, propylsulfonylamino, and the like), (x) a phenyl-lower alkylamino group (for example, phenyl-C₁₋₆ alkylamino group such as phenyl-methylamino, phenyl-ethylamino, and the like), (xi) a phenyl-lower alkylsulfonylamino group (for example, phenyl-C₁₋₆ alkyl-sulfonylamino group such as phenyl-methylsulfonylamino, phenyl-ethylsulfonylamino, and the like), (xii) phenylsulfonylamino group, (xiii) a halogen atom (for example, fluorine, chlorine, and the like), (xiv) a lower alkyl group which may be halogenated (for example, methyl, ethyl, isopropyl, tert-butyl, trifluoromethyl, and the like), (xv) a lower alkoxy group which may be halogenated (for example, methoxy, ethoxy, isopropoxy, tert-butoxy, trifluoromethoxy, and the like), etc. In particular, preferred are a 5- to 7-membered cyclic amino group which may have 1 to 3 hetero atoms selected from, for example, nitrogen atom, oxygen atom or sulfur atom, and the like in addition to one nitrogen atom (for example, pyrrolidino, piperidino, piperazino, morpholino, thiomorpholino, and the like), etc.

In the above formula, examples of the "aryl group" in the "aryl group which may be substituted" represented by Ar include C₆₋₁₄ aryl such as phenyl, naphthyl, etc., preferably C₆₋₁₀ aryl, and more preferably phenyl, etc. Said "aryl group which may be substituted" may form a condensed ring by bonding the substituents on the "aryl group" to each other, and examples of formation of a condensed ring by the aryl group (preferably, phenyl group) as Ar include
(1) a case of being condensed with a monocyclic heterocyclic ring which may be substituted;
(2) a case of being condensed with a dicyclic heterocyclic ring which may be substituted, or a case of being condensed with 2 the same or different monocyclic rings (provided that at least one ring is a monocyclic heterocyclic ring);
(3) a case of being condensed with a tricyclic heterocyclic ring which may be substituted; and the like.

Specific examples of a case that the "aryl group" in the "aryl group which may be substituted" is condensed with a monocyclic heterocyclic ring which may be substituted, include a group represented by the formula: 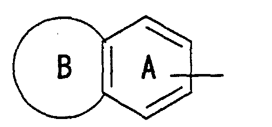 wherein ring B is a heterocyclic ring which may be substituted, and ring A is a benzene ring which may be substituted, etc.

Examples of the substituent of ring A include those similar to the above "aryl group which may be substituted", and the like.

Examples of the "heterocyclic ring" of the " heterocyclic ring which may be substituted" represented by ring B include a 4- to 14-membered ring, preferably a 5- to 9-membered ring, and the like, and they may be aromatic or non-aromatic. As the hetero atom(s), there can be used 1 to 3 or 4 atoms selected from nitrogen atom, oxygen atom or sulfur atom, and the like. Specific examples thereof include pyridine, pyrazine, pyrimidine, imidazole, furan, thiophene, dihydropyridine, azepine, diazepine, oxazepine, pyrrolidine, piperidine, hexamethyleneimine, heptamethyleneimine, tetrahydrofuran, piperazine, homopiperazine, tetrahydrooxazepine, morpholine, thiomorpholine, pyrrole, pyrazole, 1,2,3-triazole, oxazole, oxazolidine, thiazole, thiazolidine, isoxazole, imidazoline, and the like. In particular, preferred are a 5- to 9-membered non-aromatic heterocyclic ring which contains one hetero atom or two the same or different hetero atoms (for example, pyrrolidine, piperidine, hexamethyleneimine, heptamethyleneimine, tetrahydrofuran, piperazine, homopiperazine, tetrahydrooxazepine, morpholine, thiomorpholine, and the like), etc. In particular, for example, a non-aromatic heterocyclic ring which contains one hetero atom selected from nitrogen atom, oxygen atom or sulfur atom, and a non-aromatic heterocyclic ring which contains one nitrogen atom and one hetero atom selected from nitrogen atom, oxygen atom or sulfur atom, etc are often used.

The "substituent" of the "heterocyclic ring which may be substituted" represented by ring B may be substituted on the arbitrary carbon atom of ring B. Examples of the substituent(s) on the arbitrary carbon atom of ring B include 1 to 5 substituents selected from (i) a halogen atom (for example, fluorine, chlorine, bromine, iodine, and the like), (ii) nitro group, (iii) cyano group, (iv) oxo group, (v) hydroxy group, (vi) a lower alkyl group (for example, C₁₋₆ alkyl group such as methyl, ethyl, propyl, isopropyl, butyl, isobutyl, tert-butyl, sec-butyl, and the like), (vii) a lower alkoxy group (for example, C₁₋₆ alkoxy group such as methoxy, ethoxy, n-propyloxy, isopropyloxy, n-butyloxy and the like), (viii) a lower alkylthio group (for example, C₁₋₆ alkylthio group such as methylthio, ethylthio, propylthio, and the like), (ix) amino group, (x) a mono-lower alkylamino group (for example, mono-C₁₋₆ alkylamino group such as methylamino, ethylamino, propylamino, and the like), (xi) a di-lower alkylamino group (for example, di-C₁₋₆ alkylamino group such as dimethylamino, diethylamino, and the like), (xii) a 5- to 7-membered cyclic amino group which may have 1 to 3 hetero atoms selected from, for example, nitrogen atom, oxygen atom or sulfur atom, and the like in addition to carbon atom(s) and one nitrogen atom (for example, pyrrolidino, piperidino, piperazino, morpholino, thiomorpholino, and the like), (xiii) a lower alkyl-carbonylamino group (for example, C₁₋₆ alkyl-carbonylamino group such as acetylamino, propionylamino, butyrylamino, and the like), (xiv) a lower alkylsulfonylamino group (for example, C₁₋₆ alkyl-carbonylamino group such as methylsulfonylamino, ethylsulfonylamino, and the like), (xv) a lower alkoxy-carbonyl group (for example, C₁₋₆ alkoxy-carbonyl group such as methoxycarbonyl, ethoxycarbonyl, propoxycarbonyl and the like), (xvi) carboxyl group, (xvii) a lower alkyl-carbonyl group (for example, C₁₋₆ alkyl-carbonyl group such as methylcarbonyl, ethylcarbonyl, propylcarbonyl, and the like), (xviii) carbamoyl group, (xix) a mono-lower alkylcarbamoyl group (for example, mono-C₁₋₆ alkylcarbamoyl group such as methylcarbamoyl, ethylcarbamoyl, and the like), (xx) a di-lower alkylcarbamoyl group (for example, di-C₁₋₆ alkylcarbamoyl group such as dimethylcarbamoyl, diethylcarbamoyl, and the like), (xxi) a lower alkylsulfonyl group (for example, C₁₋₆ alkylsulfonyl group such as methylsulfonyl, ethylsulfonyl, propylsulfonyl, and the like), and the like.

Among these, oxo group, a lower alkyl group (for example, C₁₋₆ alkyl group such as methyl, ethyl, propyl, isopropyl, butyl, isobutyl, tert-butyl, sec-butyl, and the like) and the like are preferred, and oxo group is widely used.

Further, when the ring B has nitrogen atom in the ring, it may be substituted on the nitrogen atom. Namely, ring B may have

>N-R¹

wherein R¹ is a hydrogen atom, a hydrocarbon group which may be substituted, an acyl group which may be substituted, or a heterocyclic group which may be substituted.

The "hydrocarbon group" of the "hydrocarbon group which may be substituted" represented by the above R¹ is a group formed by eliminating one hydrogen atom from a hydrocarbon compound, and examples thereof include linear chain or cyclic hydrocarbon groups such as an alkyl group, an alkenyl group, an alkynyl group, a cycloalkyl group, an aryl group, an aralkyl group, and the like. Among these, a C₁₋₁₆ hydrocarbon group comprising a linear chain or cyclic hydrocarbon group or a combination thereof is preferably used.

Preferred examples of the linear chain or cyclic hydrocarbon group include
(1) a straight or branched chain lower alkyl group (for example, C₁₋₆ alkyl group such as methyl, ethyl, propyl, isopropyl, butyl, isobutyl, tert-butyl, sec-butyl, pentyl, hexyl, and the like),
(2) a straight or branched chain lower alkenyl group (for example, C₂₋₆ alkenyl group such as vinyl, allyl, isopropenyl, butenyl, isobutenyl, sec-butenyl, and the like),
(3) a straight or branched chain lower alkynyl group (for example, C₂₋₆ alkynyl such as propargyl, ethynyl, butynyl, 1-hexynyl, and the like),
(4) a monocyclic lower cycloalkyl group (for example, C₃₋₆ cycloalkyl such as cyclopropyl, cyclobutyl, cyclopentyl, cyclohexyl, and the like),
(5) a crosslinking ring type lower saturated hydrocarbon group (for example, C₈₋₁₄ crosslinking ring type saturated hydrocarbon group such as bicyclo[3,2,1]oct-2-yl, bicyclo[3,3,1]non-2-yl and adamanthan-1-yl, and the like), or
(6) an aryl group (for example, C₆₋₁₄ aryl group such as phenyl, 1-naphthyl, 2-naphthyl, biphenyl, 2-indenyl, 2-anthryl, and the like), etc.

Further, preferred examples of the hydrocarbon group comprising a combination of a linear chain hydrocarbon group and a cyclic hydrocarbon group include
(1) a lower aralkyl group (for example, C₇₋₁₆ aralkyl group such as phenyl-C₁₋₁₀ alkyl (for example, benzyl, phenylethyl, phenylpropyl, phenylbutyl, phenylpentyl, phenylhexyl, and the like), naphthyl-C₁₋₆ alkyl (for example, α-naphthylmethyl, and the like) or diphenyl-C₁₋₃ alkyl (for example, diphenylmethyl, diphenylethyl, and the like), and the like),
(2) an aryl-alkenyl group (for example, C₆₋₁₄ aryl-C₂₋₁₂ alkenyl group such as styryl, cinnamyl, phenyl-C₂₋₁₂ alkenyl such as 4-phenyl-2-butenyl, 4-phenyl-3-butenyl, and the like),
(3) an aryl-C₂₋₁₂ alkynyl group (for example, C₆₋₁₄ aryl-C₂₋₁₂ alkynyl group such as phenyl-C₂₋₁₂ alkynyl such as phenylethynyl, 3-phenyl-2-propynyl, 3-phenyl-1-propynyl, and the like),
(4) a lower cycloalkyl-lower alkyl group (for example, C₃₋₇ cycloalkyl-C₁₋₆ alkyl group such as cyclopropylmethyl, cyclobutylmethyl, cyclopentylmethyl, cyclohexylmethyl, cycloheptylmethyl, cyclopropylethyl, cyclobutylethyl, cyclopentylethyl, cyclohexylethyl, cycloheptylethyl, cyclopropylpropyl, cyclobutylpropyl, cyclopentylpropyl, cyclohexylpropyl, cycloheptylpropyl, cyclopropylbutyl, cyclobutylbutyl, cyclopentylbutyl, cyclohexylbutyl, cycloheptylbutyl, cyclopropylpentyl, cyclobutylpentyl, cyclopentylpentyl, cyclohexylpentyl, cycloheptylpentyl, cyclopropylhexyl, cyclobutylhexyl, cyclopentylhexyl, cyclohexylhexyl, and the like),
(5) an aryl-aryl group (for example, biphenyl, and the like),
(6) an aryl-aryl-C₁₋₁₀ alkyl group (for example, biphenylmethyl, biphenylethyl, and the like), etc.

Preferred examples of the "hydrocarbon group" of the "hydrocarbon group which may be substituted" represented by R¹, which is often used, include
(1) a straight chain, branched chain or cyclic alkyl group, preferably, a straight or branched chain C₁₋₆ alkyl group (for example, C₁₋₆ alkyl group such as methyl, ethyl, propyl, isopropyl, butyl, isobutyl, tert-butyl, sec-butyl, pentyl, hexyl, and the like), a cyclic C₃₋₈ alkyl group (for example, cyclopropyl, cyclobutyl, cyclopentyl, cyclohexyl, and the like), or a C₄₋₁₂ alkyl group consisting of a combination of a straight chain, branched chain or cyclic alkyl group (for example, cyclopropylmethyl, cyclopentylmethyl, cyclohexylmethyl, cyclohexylethyl, (4-methylcyclohexyl)methyl, and the like),
(2) a C₇₋₁₆ aralkyl group (for example, phenyl-C₁₋₁₀ alkyl (for example, benzyl, phenylethyl, phenylpropyl, phenylbutyl, phenylpentyl, phenylhexyl and the like), naphthyl-C₁₋₆ alkyl (for example, α-naphthylmethyl, and the like) or diphenyl-C₁₋₃ alkyl (for example, diphenylmethyl, diphenylethyl, and the like), and more preferably a C₇₋₁₀ aralkyl group (for example, phenyl-C₁₋₄ alkyl such as benzyl, phenylethyl, phenylpropyl, and the like), etc.

The "hydrocarbon group" represented by R¹ may be substituted and as the substituent, that usually used as a substituent of a hydrocarbon group can be appropriately used. Specifically, examples of the substituent(s) include 1 to 5 (preferably, 1 to 3) substituent(s) selected from (i) a halogen atom (for example, fluorine, chlorine, bromine, iodine, and the like), (ii) nitro group, (iii) cyano group, (iv) oxo group, (v) hydroxy group, (vi) a lower alkyl group which may be substituted with halogen or phenyl (for example, C₁₋₆ alkyl group such as methyl, ethyl, propyl, isopropyl, butyl, isobutyl, tert-butyl, sec-butyl, and the like), (vii) a lower alkoxy group which may be substituted with halogen or phenyl (for example, C₁₋₆ alkoxy group such as methoxy, ethoxy, n-propyloxy, isopropyloxy, n-butyloxy, and the like), (viii) a lower alkylthio group which may be substituted with halogen or phenyl (for example, C₁₋₆ alkylthio group such as methylthio, ethylthio, propylthio, and the like), (ix) amino group, (x) a mono-lower alkylamino group (for example, mono-C₁₋₆ alkylamino group such as methylamino, ethylamino, propylamino, and the like), (xi) a di-lower alkylamino group (for example, di-C₁₋₆ alkylamino group such as dimethylamino, diethylamino, and the like), (xii) a 5- to 7-membered cyclic amino group which may have 1 to 3 hetero atoms selected from, for example, nitrogen atom, oxygen atom or sulfur atom, and the like in addition to carbon atom(s) and one nitrogen atom (for example, pyrrolidino, piperidino, piperazino, morpholino, thiomorpholino, and the like), (xiii) a lower alkyl-carbonylamino group (for example, C₁₋₆ alkyl-carbonylamino group such as acetylamino, propionylamino, butyrylamino, and the like), (xiv) a lower alkylsulfonylamino group (for example, C₁₋₆ alkylsulfonylamino groups such as methylsulfonylamino, ethylsulfonylamino, and the like), (xv) a lower alkoxy-carbonyl group (for example, C₁₋₆ alkoxy-carbonyl group such as methoxycarbonyl, ethoxycarbonyl, propoxycarbonyl, and the like), (xvi) carboxyl group, (xvii) formyl, a lower alkyl-carbonyl group (for example, C₁₋₆ alkyl-carbonyl group such as methylcarbonyl, ethylcarbonyl, butylcarbonyl, and the like), (xviii) carbamoyl group, (xix) a mono-lower alkylcarbamoyl group (for example, mono-C₁₋₆ alkylcarbamoyl group such as methylcarbamoyl, ethylcarbamoyl, and the like), (xx) a di-lower alkylcarbamoyl group (for example, di-C₁₋₆ alkylcarbamoyl groups such as dimethylcarbamoyl, diethylcarbamoyl, and the like), (xxi) a lower alkylsulfonyl group (for example, C₁₋₆ alkylsulfonyl group such as methylsulfonyl, ethylsulfonyl, propylsulfonyl, and the like), (xxii) a lower alkoxy-carbonyl-lower alkyl group (for example, C₁₋₆ alkoxy-carbonyl-C₁₋₆ alkyl group such as methoxycarbonylmethyl, ethoxycarbonylmethyl, tert-butoxycarbonylmethyl, methoxycarbonylethyl, methoxycarbonylmethyl, methoxycarbonyl(dimethyl)methyl, ethoxycarbonyl(dimethyl)methyl, tert-butoxycarbonyl(dimethyl)methyl, and the like), (xxiii) a carboxyl-lower alkyl group (for example, carboxyl-C₁₋₆ alkyl group such as carboxylmethyl, carboxylethyl, carboxyl(dimethyl)methyl, and the like), (xxiv) a heterocyclic group which may be substituted, (xxv) an alkyl group which may be substituted, (xxvi) an alkoxy group which may be substituted, (xxvii) ureido group which may be substituted (for example, ureido, 3-methylureido, 3-ethylureido, 3-phenylureido, 3-(4-fluorophenyl)ureido, 3-(2-methylphenyl)ureido, 3-(4-methoxyphenyl)ureido, 3-(2,4-difluorophenyl)ureido, 3-[3,5-bis(trifluoromethyl)phenyl]ureido, 3-benzylureido, 3-(1-naphthyl)ureido, 3-(2-biphenyl-yl)ureido, and the like), (xxviii) thioureido group which may be substituted (for example, thioureido, 3-methylthioureido, 3-ethylthioureido, 3-phenylthioureido, 3-(4-fluorophenyl)thioureido, 3-(4-methylphenyl)thioureido, 3-(4-methoxyphenyl)thioureido, 3-(2,4-dichlorophenyl)thioureido, 3-benzylthioureido, 3-(1-naphthyl)thioureido, and the like), (xxix) amidino group which may be substituted (for example, amidino, N¹-methylamidino, N¹-ethylamidino, N¹-phenylamidino, N¹,N¹-dimethylamidino, N¹,N²-dimethylamidino, N¹-methyl-N¹-ethylamidino, N¹,N¹-diethylamidino, N¹-methyl-N¹-phenylamidino, N¹,N¹-di(4-nitrophenyl)amidino, and the like), (xxx) guanidino group which may be substituted (for example, guanidino, 3-methylguanidino, 3,3-dimethylguanidino, 3,3-diethylguanidino, and the like), (xxxi) a cyclic aminocarbonyl group which may be substituted (for example, pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl)piperidino]carbonyl, (4-methylpiperazino)carbonyl, (4-phenylpiperazino)carbonyl, [4-(4-nitrophenyl)piperazino]carbonyl, (4-benzylpiperazino)carbonyl, morpholinocarbonyl, thiomorpholinocarbonyl, and the like), (xxxii) aminothiocarbonyl group which may be substituted (for example, aminothiocarbonyl, methylaminothiocarbonyl, dimethylaminothiocarbonyl, and the like), (xxxiii) aminosulfonyl which may be substituted (for example, aminosulfonyl, methylaminosulfonyl, dimethylaminosulfonyl, and the like), (xxxiv) phenylsulfonylamino which may be substituted (for example, phenylsulfonylamino, (4-methylphenyl)sulfonylamino, (4-chlorophenyl)sulfonylamino, (2,5-dichlorophenyl)sulfonylamino, (4-methoxyphenyl)sulfonylamino, (4-acetylaminophenyl)sulfonylamino, (4-nitrophenyl)phenylsulfonylamino, and the like), (xxxv) sulfo group, (xxxvi) sulfino group, (xxxvii) sulfeno group, (xxxviii) a C₁₋₆ alkylsulfo group (for example, methylsulfo, ethylsulfo, propylsulfo, and the like), (xxxix) a C₁₋₆ alkylsulfino group (for example, methylsulfino, ethylsulfino, propylsulfino, and the like), (xxxx) a C₁₋₆ alkylsulfeno group (for example, methylsulfeno, ethylsulfeno, propylsulfeno, and the like), (xxxxi) a phosphono group, (xxxxii) a di-C₁₋₆ alkoxyphosphoryl group (for example, dimethoxyphosphoryl, diethoxyphosphoryl, dipropoxyphosphoryl, and the like), (xxxxiii) C₁₋₄ alkylenedioxy (for example, -O-CH₂-O-, -O-CH₂-CH₂-O-, and the like), (xxxxiv) phenylthio which may be substituted with halogen, (xxxxv) phenoxy which may be substituted with halogen, etc.

Preferred examples of the "substituent" of the "hydrocarbon group which may be substituted" include a halogen atom, an alkyl group which may be substituted, an alkoxy group which may be substituted, hydroxy group, nitro group, cyano group, carboxyl group, a C₁₋₆ alkoxy-carbonyl group, a carbamoyl group, aminothiocarbonyl group, a mono-lower alkylcarbamoyl group, a di-lower alkylcarbamoyl group, a cyclic aminocarbonyl group which may be substituted, amino group, a mono-lower alkylamino group, a di-lower alkylamino group, a 5- to 7-membered cyclic amino group which may have 1 to 3 hetero atoms selected from nitrogen atom, oxygen atom or sulfur atom and the like in addition to carbon atom(s) and one nitrogen atom, a C₁₋₆ alkylcarbonylamino group, phenylsulfonylamino group which may be substituted, a C₁₋₆ alkylsulfonylamino group, an amidino group which may be substituted, ureido group which may be substituted, a heterocyclic group which may be substituted, etc.

Examples of the "heterocyclic group" of the "heterocyclic group which may be substituted" include a group obtained by eliminating one hydrogen atom from a monocyclic heterocyclic ring, a dicyclic heterocyclic ring, and a polycyclic heterocyclic ring such as tricyclic or tetracyclic, etc. Said heterocyclic ring may be either of aromatic or non-aromatic. The hetero atom(s) are, for example, 1 to 6 atom(s) selected from nitrogen atom, oxygen atom, sulfur atom, and the like. Specific examples of the monocyclic heterocyclic group include a group obtained by eliminating one hydrogen atom from the "heterocyclic ring" of the "heterocyclic ring which may be substituted", etc. In addition to those, examples thereof further include a group obtained by eliminating one hydrogen atom from monocyclic heterocyclic rings such as triazole, thiadiazole, oxadiazole, oxathiazole, triazine, tetrazole, etc. Examples of the dicyclic heterocyclic group include a group obtained by eliminating one hydrogen atom from dicyclic heterocyclic ring such as indole, dihydroindole, isoindole, dihydroisoindole, benzofuran, dihydrobenzofuran, benzimidazole, benzoxazole, benzisoxazole, benzothiazole, indazole, quinoline, tetrahydroquinoline, isoquinoline, tetrahydroisoquinoline, tetrahydro-lH-1-benzazepin, tetrahydro-1H-2-benzazepin, tetrahydro-1H-3-benzazepin, tetrahydrobenzoxazepin, quinazoline, tetrahydroquinazoline, quinoxaline, tetrahydroquinoxaline, benzodioxane, benzodioxole, benzothiazine, imidazopyridine, and the like. Examples of the polycyclic heterocyclic ring such as tricyclic, tetracyclic, etc., include acridine, tetrahydroacridine, pyrroloquinoline, pyrroloindole, cyclopentoindole, isoindolobenzazepin, and the like.

In particular, examples of the "heterocyclic ring" of the "heterocyclic ring which may be substituted", which is often used, include a group obtained by eliminating one hydrogen atom from the above monocyclic heterocyclic ring or dicyclic heterocyclic ring, etc.

Further, examples of the "substituent" of the "heterocyclic ring which may be substituted" include the "substituent" of the "heterocyclic ring which may be substituted" represented by the above ring B (provided that "the heterocyclic ring which may be substituted" is excluded), etc.

Examples of the "substituent" of the "alkyl which may be substituted (preferably, C₁₋₆ alkyl which may be substituted)" or the "alkoxy which may be substituted (preferably, C₁₋₆ alkoxy which may be substituted)" include the "substituent" shown in (i) to (xxiv) or (xxvii) to (xxxxii) as the "substituent" of the "hydrocarbon group which may be substituted" represented by the above R¹, etc.

Examples of the "substituent" of the "ureido group which may be substituted", the "thioureido group which may be substituted", the "amidino group which may be substituted", the "guanidino group which may be substituted", the "cyclic aminocarbonyl group which may be substituted", the "aminothiocarbonyl group which may be substituted", the "aminosufonyl which may be substituted", or the "phenylsufonylamino which may be substituted" include the "substituent" shown in (i) to (xxvi) or (xxxv) to (xxxxii) as the "substituent" of the "hydrocarbon group which may be substituted" represented by the above R¹, etc.

Preferred examples of the "hydrocarbon group which may be substituted" represented by R¹ include (i) a C₁₋₆ alkyl group or (ii) a phenyl-C₁₋₆ alkyl group, which may be substituted with a halogen atom, nitro, C₁₋₆ alkyl, C₁₋₆ alkoxy, and the like. More preferred is benzyl group which may be substituted with C₁₋₄ alkyl (methyl, and the like), trihalogeno C₁₋₄ alkyl (methyl, and the like), a halogen atom (fluoro, chloro, and the like), nitro, cyano, C₁₋₄ alkoxy (methoxy, and the like), trihalogen C₁₋₄ alkoxy (methoxy, and the like), hydroxy, carbamoyl, (4-C₁₋₄ alkyl (methyl, etc,)-1-piperadinyl)carbonyl, amniothiocarbonyl, morpholinocarbonyl, carboxyl, C₁₋₄ alkoxy (methoxy, etc.)-carbonyl, C₁₋₄ alkoxy (ethoxy, etc.)-carbonyl-C₁₋₄ alkoxy (methoxy, etc.), carboxyl-C₁₋₄ alkoxy (methoxy, etc.), C₁₋₄ alkoxy (ethoxy, etc.)-carbonyl-C₁₋₆ alkyl (isopropyl, etc.), carboxyl-C₁₋₆ alkyl (isopropyl, etc.), amino, acetylamino, C₁₋₄ alkyl (methyl, etc.)-sulfonylamino, (4-C₁₋₄ alkyl (methyl, etc.)-phenyl)sulfonylamino, ureido, 3-C₁₋₄ alkyl (methyl, etc.)-ureido, amidino, dihydrothiazolyl, dihydroimidazolyl, etc.

Among these, preferred R¹ is benzyl group which may be substituted with C₁₋₄ alkyl (methyl, etc.), trihalogeno (fluoro, etc.)-C₁₋₄ alkyl (methyl, etc.), a halogen atom (fluoro, chloro, etc.), nitro, cyano, carbamoyl, C₁₋₄ alkoxy (methoxy, etc.)-carbonyl, C₁₋₄ alkoxy (ethoxy, etc.)-carbonyl-C₁₋₄ alkoxy (methoxy, etc.), amino, acetylamino, C₁₋ ₄ alkyl (methyl, etc.)-sulfonylamino, 3-C₁₋₄ alkyl (methyl, etc.)-ureido, amidino, or dihydroimidazolyl. Among these, benzyl group which may be substituted with C₁₋₄ alkyl is preferred, and in particular, benzyl group which may be substituted with methyl is preferred.

Examples of the "acyl group which may be substituted" represented by the above R¹ include -(C=O)-R^{2c}, -SO₂-R^{2c}, -SO-R^{2c}, -(C=O)NR^{3c}R^{2c}, -(C=O)O-R^{2c}, -(C=S)O-R^{2c}, -(C=S)NR^{3c}R^{2c}, [R^{2c} and R^{3c} are the same or different and each is (i) hydrogen atom, (ii) a hydrocarbon group which may be substituted or (iii) a heterocyclic group which may be substituted, or R^{2c} and R^{3c} may be bonded to each other to form a nitrogen-containing saturated heterocyclic group which may be substituted, together with the adjacent nitrogen atom], and the like.

Among these, preferred is -(C=O)-R^{2c}, -SO₂-R^{2c}, -SO-R^{2c}, -(C=O)NR^{3c}R^{2c} or -(C=O)O-R^{2c} (R^{2c} and R^{3c} are as defined above), and among these, -(C=O)-R^{2c} or -(C=O)NR^{3c}R^{2c} (R^{2c} and R^{3c} are as defined above) is often used.

The "hydrocarbon group" of the "hydrocarbon group which may be substituted" represented by R^{2c} and R^{3c} is a group obtained by eliminating one hydrogen atom from a hydrocarbon compound, and examples thereof include a linear chain or cyclic hydrocarbon group such as an alkyl group, an alkenyl group, an alkynyl group, a cycloalkyl group, an aryl group, an aralkyl group, and the like. Specific examples thereof include those similar to the "hydrocarbon group" of the "hydrocarbon group which may be substituted" represented by the above R¹, and among these, a liner chain or cyclic C₁₋₁₆ hydrocarbon group is preferred, and in particular, a lower (C₁₋₆) alkyl group, a lower (C₂₋₆) alkenyl group, a C₇₋₁₆ aralkyl group or a C₆₋₁₄ aryl group is preferred. Among these, a lower (C₁₋₆)alkyl group, a C₇₋₁₆ aralkyl group or a C₆₋₁₄ aryl group is widely used.

Examples of the "heterocyclic group" of the "heterocyclic group which may be substituted" represented by R^{2c} and R^{3c} include a group obtained by eliminating one hydrogen atom from a monocyclic heterocyclic ring, a dicyclic heterocyclic ring, and a polycyclic heterocyclic ring such as tricyclic or tetracyclic, etc. The heterocyclic ring, may be either of aromatic or non aromatic. The hetero atom(s) are, for example, 1 to 6 atom(s) selected from nitrogen atom, oxygen atom or sulfur atom and the like. Specifically, as the monocyclic heterocyclic group, a group obtained by eliminating one hydrogen atom from the "heterocyclic ring" of the "heterocyclic ring which may be substituted" represented by the above b ring, etc., can be used. Further, in addition to those, for example, a group obtained by eliminating one hydrogen atom from monocyclic heterocyclic rings such as triazole, thiadiazole, oxadiazole, oxathiazole, triazine, tetrazole, etc., can be used. Examples of the dicyclic heterocyclic group include a group obtained by eliminating one hydrogen atom from dicyclic heterocyclic ring such as indole, dihydroindole, isoindole, dihydroisoindole, benzofuran, dihydrobenzofuran, benzimidazole, benzoxazole, benzisoxazole, benzothiazole, indazole, quinoline, tetrahydroquinoline, isoquinoline, tetrahydroisoquinoline, tetrahydro-1H-1-benzazepin, tetrahydro-1H-2-benzazepin, tetrahydro-1H-3-benzazepin, tetrahydrobenzoxazepin, quinazoline, tetrahydroquinazoline, quinoxaline, tetrahydroquinoxaline, benzodioxane, benzodioxole, benzothiazine, imidazopyridine, and the like. Examples of the polycyclic heterocyclic ring such as tricyclic or tetracyclic ring include a group obtained by eliminating one hydrogen atom from polycyclic heterocyclic rings such as acridine, tetrahydroacridine, pyrroloquinoline, pyrroloindole, cyclopentoindole, isoindolobenzazepin, and the like.

In particular, examples of the "heterocyclic ring" of the "heterocyclic ring which may be substituted", which are often used, include a group obtained by eliminating one hydrogen atom from the above monocyclic heterocyclic ring or dicyclic heterocyclic ring, etc.

Examples of the "nitrogen-containing saturated heterocyclic group which may be substituted" formed by R^{2c} and R^{3c} together with the adjacent nitrogen atom include a 5- to 9-membered nitrogen-containing saturated heterocyclic group which may contain 1 to 3 hetero atoms selected from nitrogen atom, oxygen atom, sulfur atom, and the like in addition to carbon atom(s) and one nitrogen atom, etc. Preferred examples thereof include a nitrogen-containing saturated heterocyclic group having a bonding hand on a ring-constituting nitrogen atom. As the group having a bonding hand on a ring-constituting nitrogen atom, for example, a group represented by the formula: 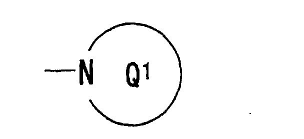 wherein ring Q¹ is a 5- to 9-membered nitrogen-containing saturated heterocyclic group which may contain 1 to 2 hetero atom(s) selected from nitrogen atom, oxygen atom, sulfur atom, and the like, in addition to carbon atom(s) and one nitrogen atom], etc., is used. More specifically, examples thereof. which are often used, include 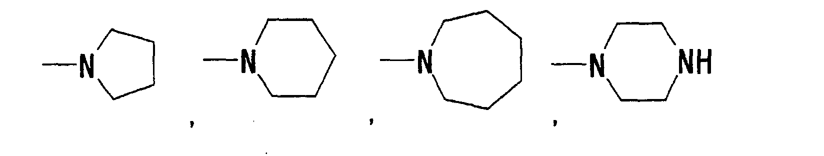 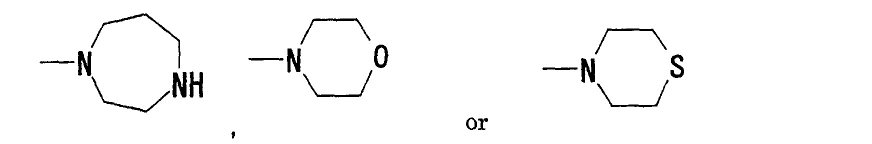 and the like.

Preferred examples of the substituent(s) of the "hydrocarbon group" or the "heterocyclic group" represented by R^{2c} and R^{3c}, and the "nitrogen-containing saturated heterocyclic group" represented by NR^{2c}R^{3c} include 1 to 5 (preferably, 1 to 3) substituent(s) selected from (i) a halogen atom (for example, fluorine, chlorine, bromine, iodine, and the like), (ii) nitro group, (iii) cyano group, (iv) oxo group, (v) hydroxy group, (vi) a hydrocarbon group which may be substituted, (vii) a lower alkoxy group (for example, C₁₋₆ alkoxy group such as methoxy, ethoxy, n-propyloxy, isopropyloxy, n-butyloxy, and the like) which may be substituted with phenyl group, (viii) a lower alkylthio group (for example, C₁₋₆ alkylthio group such as methylthio, ethylthio, propylthio, and the like) which may be substituted with phenyl group, (ix) amino group, (x) a mono-lower alkylamino group (for example, mono-C₁₋₆ alkylamino group such as methylamino, ethylamino, propylamino, and the like), (xi) a di-lower alkylamino group (for example, di-C₁₋₆ alkylamino group such as dimethylamino, diethylamino, and the like), (xii) a 5- to 7-membered cyclic amino group which may have 1 to 3 hetero atoms selected from, for example, nitrogen atom, oxygen atom, sulfur atom, and the like in addition to carbon atom(s) and one nitrogen atom (for example, pyrrolidino, piperidino, piperazino, morpholino, thiomorpholino, and the like), (xiii) a lower alkyl-carbonylamino group (for example, C₁₋₆ alkyl-carbonylamino group such as acetylamino, propionylamino, butyrylamino, and the like), (xiv) a lower alkylsulfonylamino group (for example, C₁₋₆ alkylsulfonylamino group such as methylsulfonylamino, ethylsulfonylamino, and the like), (xv) a lower alkoxy-carbonyl group (for example, C₁₋₆ alkoxy-carbonyl group such as methoxycarbonyl, ethoxycarbonyl, propoxycarbonyl, and the like), (xvi) carboxyl group, (xvii) a lower alkyl-carbonyl group (for example, C₁₋₆ alkyl-carbonyl group such as methylcarbonyl, ethylcarbonyl, propylcarbonyl, and the like), (xviii) carbamoyl group, (xix) a mono-lower alkylcarbamoyl group (for example, mono-C₁₋₆ alkylcarbamoyl group such as methylcarbamoyl, ethylcarbamoyl, and the like), (xx) a di-lower alkylcarbamoyl group (for example, di-C₁₋₆ alkylcarbamoyl group such as dimethylcarbamoyl, diethylcarbamoyl, and the like), (xxi) a lower alkylsulfonyl group (for example, C₁₋₆ alkylsulfonyl group such as methylsulfonyl, ethylsulfonyl, propylsulfonyl, and the like), (xxii) a lower alkoxy-carbonyl-lower alkyl group (for example, C₁₋₆ alkoxy-carbonyl-C₁₋₆ alkyl group such as methoxycarbonylmethyl, ethoxycarbonylmethyl, tert-butoxycarbonylmethyl, methoxycarbonylethyl, methoxycarbonylmethyl, methoxycarbonyl(dimethyl)methyl, ethoxycarbonyl(dimethyl)methyl, tert-butoxycarbonyl(dimethyl)methyl, and the like), (xxiii) a carboxyl-lower alkyl group (for example, carboxyl-C₁₋₆ alkyl groups such as carboxylmethyl, carboxylethyl, carboxyl(dimethyl)methyl, and the like), (xxiv) a heterocyclic group which may be substituted, (xxv) phenylthio which may be substituted with halogen, (xxvi) phenoxy which may be substituted with halogen, and the like.

Said the "lower alkoxy group" and the "lower alkylthio group" may further have phenyl group as a substituent.

Examples of said the "substituent" and the "hydrocarbon group" of the "hydrocarbon group which may be substituted" include the "substituent" and the "hydrocarbon group" of the "hydrocarbon group which may be substituted" represented by the above R¹, etc.

As the "heterocyclic group" of said "heterocyclic group which may be substituted", a group obtained by eliminating one hydrogen atom from the "heterocyclic ring" of the "heterocyclic group which may be substituted" represented by the above ring B, etc. can be used.

Further, as the "substituent" of the "heterocyclic group which may be substituted", the "substituent" of the "heterocyclic ring which may be substituted" represented by the above ring B (provided that the "heterocyclic ring which may be substituted" is excluded), etc., can be used.

Preferred examples of R^{2c} and R^{3c} include phenyl which may be substituted with C₁₋₄ alkyl (methyl, ethyl, and the like) or C₁₋₄ alkoxy (methoxy, ethoxy, and the like) , C₁₋₄ alkyl (methyl, ethyl, and the like), halogeno (fluoro, chloro, and the like)-C₁₋₄ alkyl (methyl, ethyl, and the like), benzyl, naphthyl, pyridyl, thienyl, furyl, hydrogen atom, etc.

Preferred examples of the "acyl group which may be substituted" represented by the above R¹ include formyl, acetyl, trihalogeno (fluoro, and the like) acetyl, pyridylcarbonyl, thienylcarbonyl, furylcarbonyl, phenacyl, benzoyl, C₁₋₄ alkyl (methyl, and the like)-benzoyl, C₁₋₄ alkoxy (methoxy, and the like)-benzoyl, benzenesulfonyl, naphthylsulfonyl, thienylsulfonyl. and the like, more preferably, -(C=O)-R^{2c} [wherein R^{2c} is phenyl group which may be substituted with a C₁₋₆ alkyl group and a C₁₋₆ alkoxy group or a phenyl -C₁₋₆ alkyl group], etc.

Examples of the "heterocyclic group" of the "heterocyclic group which may be substituted" represented by R¹ include a group obtained by eliminating one hydrogen atom from a monocyclic heterocyclic ring, a dicyclic heterocyclic ring, or a polycyclic heterocyclic ring such as tricyclic or tetracyclic, etc. Said heterocyclic ring, may be either of aromatic or non-aromatic. The hetero atom(s) include, for example, 1 to 6 atoms selected from nitrogen atom, oxygen atom, sulfur atom, and the like. Specifically, as the monocyclic heterocyclic group, a group obtained by eliminating one hydrogen atom from the "heterocyclic ring" of the "heterocyclic ring which may be substituted" represented by the above ring B, etc., can be used. Further, in addition to those, a group obtained by eliminating one hydrogen atom from monocyclic heterocyclic rings such as triazole, thiadiazole, oxadiazole, oxathiadiazole, triazine, tetrazole, etc., can be used. Examples of the dicyclic heterocyclic group include a group obtained by eliminating one hydrogen atom from a dicyclic heterocyclic ring such as indole, dihydroindole, isoindole, dihydroisoindole, benzofuran, dihydrobenzofuran, benzimidazole, benzoxazole, benzisoxazole, benzothiazole, indazole, quinoline, tetrahydroquinoline, isoquinoline, tetrahydroisoquinoline, tetrahydro-1H-1-benzazepin, tetrahydro-lH-2-benzazepin, tetrahydro-1H-3-benzazepin, tetrahydrobenzoxazepin, quinazoline, tetrahydroquinazoline, quinoxaline, tetrahydroquinoxaline, benzodioxane, benzodioxole, benzothiazine, imidazopyridine, and the like. Examples of the polycyclic heterocyclic ring such as tricyclic or tetracyclic include a group obtained by eliminating one hydrogen atom from a polycyclic heterocyclic ring such as acridine, tetrahydroacridine, pyrroloquinoline, pyrroloindole, cyclopentoindole, isoindolobenzazepin, and the like.

In particular, examples of the "heterocyclic group" of said "heterocyclic group which may be substituted" include a group obtained by eliminating one hydrogen atom from the above monocyclic heterocyclic ring or dicyclic heterocyclic ring, etc., and among these, pyridyl group is preferred.

Further, examples of the "substituent" of the "heterocyclic group which may be substituted" include the "substituent" of the "heterocyclic ring which may be substituted" represented by the above ring B (provided that the "heterocyclic ring which may be substituted" is excluded), etc.

Preferred examples of R¹ include (i) a hydrogen atom, (ii) a C₁₋₆ alkyl group, (iii) a halogen atom, nitro, phenyl C₁₋₆ alkyl group which may be substituted with C₁₋₆ alkyl or C₁₋₆ alkoxy-C₁₋₆ alkyl group, or (iv) -(C=O)-R^{2c} [wherein R^{2c} is phenyl group which may be substituted with a C₁₋₆ alkyl group and a C₁₋₆ alkoxy group or a phenyl-C₁₋₆ alkyl group], etc.

More specific examples of a case in which the "aryl group" of the "aryl group which may be substituted" is condensed with a monocyclic heterocyclic ring which may be substituted include, as phenyl group condensed with a monocyclic heterocyclic ring represented by the formula: 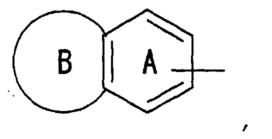 a group obtained by eliminating one hydrogen atom from a dicyclic condensed benzene ring such as 2,3-dihydrobenzofuran; 3,4-dihydro-2H-1-benzothiopyran; 2,3-dihydro-1H-indole; 1,2,3,4-tetrahydroquinoline; 2,3-dihydro-1H-isoindole; 1,2,3,4-tetrahydroisoquinoline; benzazepines such as 2,3,4,5-tetrahydro-1H-1-benzazepine, 2,3,4,5-tetrahydro-1H-2-benzazepine, 2,3,4,5-tetrahydro-1H-3-benzazepine and the like; benzazocines such as 1,2,3,4,5,6-hexahydro-1-benzazocine, 1,2,3,4,5,6-hexahydro-2-benzazocine, 1,2,3,4,5,6-hexahydro-3-benzazocine and the like; benzazonines such as 2,3,4,5,6,7-hexahydro-1H-1-benzazonine, 2,3,4,5,6,7-hexahydro-1H-2-benzazonine; 2,3,4,5,6,7-hexahydro-1H-3-benzazonine, 2,3,4,5,6,7-hexahydro-1H-4-benzazonine and the like; benzoxazoles such as 2,3-dihydrobenzoxazole and the like; benzothiazoles such as 2,3-dihydrobenzothiazole and the like; benzimidazoles such as 2,3-dihydro-1H-benzimidazole and the like; benzoxazines such as 3,4-dihydro-1H-2,1-benzoxazine, 3,4-dihydro-1H-2,3-benzoxazine, 3,4-dihydro-2H-1,2-benzoxazine, 3,4-dihydro-2H-1,4-benzoxazine, 3,4-dihydro-2H-1,3-benzoxazine, 3,4-dihydro-2H-3,1-benzoxazine and the like; benzothiazines such as 3,4-dihydro-1H-2,1-benzothiazine, 3,4-dihydro-1H-2,3-benzothiazine, 3,4-dihydro-2H-1,2-benzothiazine, 3,4-dihydro-2H-1,4-benzothiazine, 3,4-dihydro-2H-1,3-benzothiazine, 3,4-dihydro-2H-3,1-benzothiazine and the like; benzodiazines such as 1,2,3,4-tetrahydrocinnoline, 1,2,3,4-tetrahydrophthaladine, 1,2,3,4-tetrahydroquinazoline, 1,2,3,4-tetrahydroquinoxaline and the like; benzoxathines such as 3,4-dihydro-1,2-benzoxathine, 3,4-dihydro-2,1-benzoxathine, 2,3-dihydro-1,4-benzoxathine, 1,4-dihydro-2,3-benzoxathine, 4H-1,3-benzoxathine, 4H-3,1-benzoxathine and the like; benzodioxines such as 3,4-dihydro-1,2-benzodioxine, 2,3-dihydro-1,4-benzodioxine, 1,4-dihydro-2,3-benzodioxine, 4H-1,3-benzodioxine and the like; benzdithines such as 3,4-dihydro-1,2-benzdithine, 2,3-dihydro-1,4-benzdithine, 1,4-dihydro-2,3-benzdithine, -4H-1,3-benzdithine and the like; benzoxazepines such as 2,3,4,5-tetrabydro-1,2-benzoxazepine, 2,3,4,5-tetrahydro-1,3-benzoxazepine, 2,3,4,5-tetrahydro-1,4-benzoxazepine, 2,3,4,5-tetrahydro-1,5-benzoxazepine, 1,3,4,5-tetrahydro-2,1-benzoxazepine, 1,3,4,5-tetrahydro-2,3-benzoxazepine, 1,3,4,5-tetrahydro-2,4-benzoxazepine, 1,2,4,5-tetrahydro-3,1-benzoxazepine, 1,2,4,5-tetrahydro-3,2-benzoxazepine, 1,2,3,5-tetrahydro-4,1-benzoxazepine and the like; benzothiazepines such as 2,3,4,5-tetrahydro-1,2-benzothiazepine, 2,3,4,5-tetrahydro-1,4-benzothiazepine, 2,3,4,5-tetrahydro-1,5-benzothiazepine, 1,3,4,5-tetrahydro-2,1-benzothiazepine, 1,3,4,5-tetrahydro-2,4-benzoxazepine, 1,2,4,5-tetrahydro-3,1-benzothiazepine, 1,2,4,5-tetrahydro-3,2-benzothiazepine, 1,2,3,5-tetrahydro-4,1-benzothiazepine and the like; benzodiazepines such as 2,3,4,5-tetrahydro-1H-1,2-benzodiazepine, 2,3,4,5-tetrahydro-1H-1,3-benzodiazepine, 2,3,4,5-tetrahydro-1H-1,4-benzodiazepine, 2,3,4,5-tetrahydro-1H-1,5-benzodiazepine, 2,3,4,5-tetrahydro-1H-2,3-benzodiazepine, 2,3,4,5-tetrahydro-1H-2,4-benzodiazepine and the like; benzodioxepines such as 4,5-dihydro-1,3-benzodioxepine, 4,5-dihydro-3H-1,2-benzodioxepine, 2,3-dihydro-5H-1,4-benzodioxepine, 3,4-dihydro-2H-1,5-benzodioxepine, 4,5-dihydro-1H-2,3-benzodioxepine, 1,5-dihydro-2,4-benzodioxepine and the like; benzodithiepines such as 4,5-dihydro-1H-2,3-benzodithiepine, 1,5-dihydro-2,4-benzodithiepine, 3,4-dihydro-2H-1,5-benzodithiepine, 2,3-dihydro-5H-1,4-benzodithiepine and the like; benzoxazocines such as 3,4,5,6-tetrahydro-2H-1,5-benzoxazocine, 3,4,5,6-tetrahydro-2H-1,6-benzoxazocine and the like; benzothiazocines such as 3,4,5,6-tetrahydro-2H-1,5-benzothiazocine, 3,4,5,6-tetrahydro-2H-1,6-benzothiazocine, and the like; benzodiazocines such as 1,2,3,4,5,6-hexahydro-1,6-benzodiazocine and the like; benzoxathiocines such as 2,3,4,5-tetrahydro-1,6-benzoxathiocine and the like; benzodioxocines such as 2,3,4,5-tetrahydro-1,6-benzodioxocine and the like; benzotrioxepines such as 1,3,5-benzotrioxepine, 5H-1,3,4-benzotrioxepine and the like; benzoxathiazepines such as 3,4-dihydro-1H-5,2,1-benzoxathiazepine, 3,4-dihydro-2H-5,1,2-benzoxathiazepine, 4,5-dihydro-3,1,4-benzoxathiazepine, 4,5-dihydro-3H-1,2,5-benzoxathiazepine and the like; benzoxadiazepines such as 2,3,4,5-tetrahydro-1,3,4-benzoxadiazepine and the like; benzthiadiazepines such as 2,3,4,5-tetrahydro-1,3,5-benzthiadiazepine and the like; benzotriazepines such as 2,3,4,5-tetrahydro-1H-1,2,5-benzotriazepine and the like; 4,5-dihydro-1,3,2-benzooxathiepine, 4,5-dihydro-1H-2,3-benzoxathiepine, 3,4-dihydro-2H-1,5-benzoxathiepine, 4,5-dihydro-3H-1,2-benzoxathiepine, 4,5-dihydro-3H-2,1-benzoxathiepine, 2,3-dihydro-5H-1,4-benzoxathiepine, 2,3-dihydro-5H-4,1-benzoxathiepine, and the like; and, in particular, 2,3,4,5-tetrahydro-lH-3-benzazepine, 2,3,4,5-tetrahydro-1H-2-benzazepine, 2,3-dihydro-1H-indole, 2,3,4,5-tetrahydro-1,4-benzoxazepine, and the like.

Preferred examples of a case that the "aryl group" of the "aryl group which may be substituted" is condensed with a monocyclic heterocyclic ring which may be substituted include a group represented by the formula; 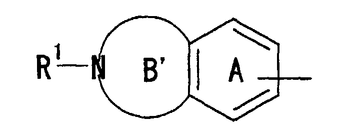 wherein ring B' is a 5- to 9-membered nitrogen-containing heterocyclic ring which may be substituted with oxo group other than R¹, and ring A and R¹ are as defined above, etc.

Examples of the "5- to 9-membered nitrogen-containing heterocyclic ring" of said "5- to 9-membered nitrogen-containing heterocyclic ring may be substituted with oxo group" include a 5- to 9-membered nitrogen-containing heterocyclic ring which may contain 1 to 3 of hetero atoms such as, for example, nitrogen atom, oxygen atom, sulfur atom, and the like, in addition to carbon atom(s) and one nitrogen atom. Preferably, a 5- to 9-membered non-aromatic nitrogen-containing heterocyclic ring (for example, pyrrolidine, piperidine, hexamethyleneimine, heptamethyleneimine, piperazine, homopiperazine, tetrahydrooxazepine, morpholine, thiomorpholine and the like), etc., can be used.

More preferred examples of a case that the "aryl group" of the "aryl group which may be substituted" is condensed with a monocyclic heterocyclic ring which may be substituted include a group represented by the formula: 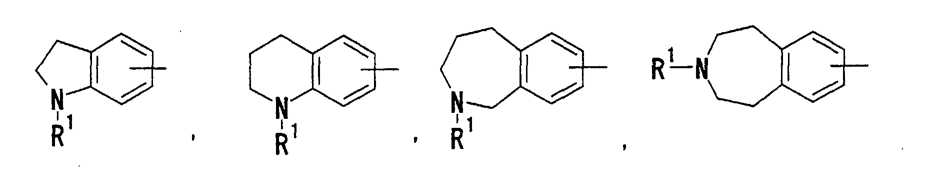 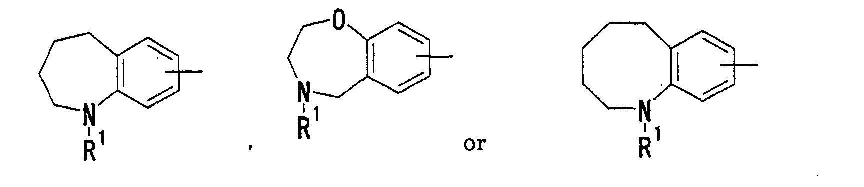 wherein R¹ is as defined above, in addition to a group represented by the formula: 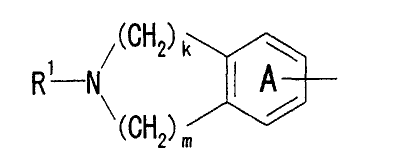 wherein ring A and R¹ are as defined above, and each of k and m is independently an integer of 0 to 5 and 1 < k+m < 5, etc. In particular, preferred examples include a group represented by the formula: 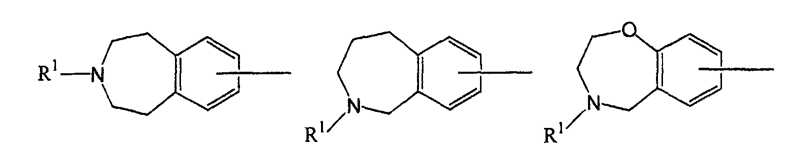 wherein R¹ is as defined above, in addition to a group represented by the formula: 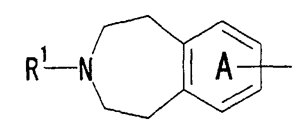 wherein ring A and R¹ are as defined above, etc.

Specific examples of a case that the "aryl group" of the "aryl group which may be substituted" is condensed with a dicyclic heterocyclic ring which may be substituted, or a case that it is condensed with 2 the same or different monocyclic rings (provided that at least one ring is a monocyclic heterocyclic ring) include a group represented by the formula: 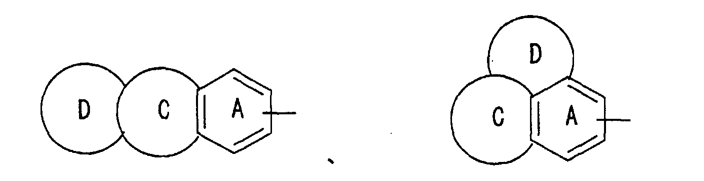 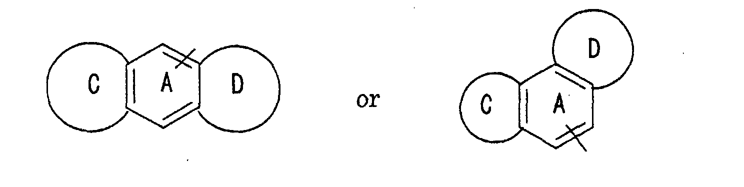 wherein ring A is as defined above, one of ring C and ring D is a heterocyclic ring which may be substituted, and the other is a 5- to 9-membered ring which may be substituted and may contain hetero atom(s).

Examples of the "heterocyclic ring" of the "heterocyclic ring which may be substituted" represented by ring C and ring D include a 4- to 14-membered heterocyclic ring, preferably a 5- to 9-membered heterocyclic ring, and the like. The hetero atom(s) are, for example, 1 to 3 atoms selected from nitrogen atom, oxygen atom, sulfur atom, and the like. Further, it may be aromatic or non-aromatic. Specific examples thereof include pyridine, pyrazine, pyrimidine, imidazole, furan, thiophene, dihydropyridine, diazepine, oxazepine, pyrrolidine, piperidine, hexamethyleneimine, heptamethyleneimine, tetrahydrofuran, piperazine, homopiperazine, tetrahydrooxazepine, morpholine, thiomorpholine, and the like.

The "substituent" of the "heterocyclic ring which may be substituted" are as defined with respect to the "substituent" of the "heterocyclic ring which may be substituted" represented by the above ring B.

Examples of the "5- to 9-membered ring which may contain hetero atom(s)" of the "5- to 9-membered ring which may be substituted and may contain hetero atom(s)" represented by ring C and ring D include a 5- to 9-membered heterocyclic ring (for example, saturated or unsaturated 5-to 9-membered heterocyclic ring such as pyridine, pyrazine, pyrimidine, imidazole, furan, thiophene, dihydropyridine, diazepine, oxazepine, pyrrolidine, piperidine, hexamethyleneimine, heptamethyleneimine, tetrahydrofuran, piperazine, homopiperazine, tetrahydrooxazepine, morpholine, thiomorpholine, and the like), or a 5- to 9-membered carbon ring. Said "5- to 9-membered carbon ring" may be a saturated or unsaturated ring, and examples thereof include benzene, cyclopentane, cyclopentene, cyclohexane, cyclohexene, cyclohexadiene, cycloheptane, cycloheptene, cycloheptadiene and the like. Among these, benzene, cyclohexane, or the like is preferred.

The "substituent" of the "5- to 9-membered ring which may be substituted and may contain hetero atom(s)" is as defined with respect to the "substituent on the arbitrary carbon atom of ring B" of the "heterocyclic ring which may be substituted" represented by the above ring B.

More specific examples of a case that the "aryl group" of the "aryl group which may be substituted" represented by Ar is condensed with a dicyclic heterocyclic ring which may be substituted include:
(1) as phenyl group condensed with a dicyclic heterocyclic ring which is represented by the formula: 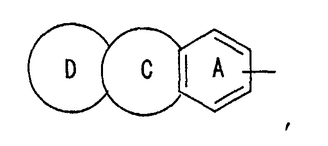 a group obtained by eliminating one hydrogen atom from a tricyclic condensed benzene ring such as carbazole, 1,2,3,4,4a,9a-hexahydrocarbazole, 9,10-dihydroacridine, 1,2,3,4-tetrahydroacridine, 10,11-dihydro-5H-dibenz[b,f]azepine, 5,6,7,12-tetrahydrodibenz[b,g]azocine, 6,11-dihydro-5H-dibenz[b,e]azepine, 6,7-dihydro-5H-dibenz[c,e]azepine, 5,6,11,12-tetrahydrodibenz[b,f]azocine, dibenzofuran, 9H-xanthene, 10,11-dihydrodibenz[b,f]oxepine, 6,11-dihydrodibenz[b,e]oxepine, 6,7-dihydro-5H-dibenz[b,g]oxocine, dibenzothiophene, 9H-thioxanthene, 10,11-dihydrodibenzo[b,f]thiepin, 6,11-dihydrodibenzo[b,e]thiepin, 6,7-dihydro-5H-dibenzo[b,g]thiosine, 10H-phenothiazine, 10H-phenoxazine, 5,10-dihydrophenazine, 10,11-dibenzo[b,f][1,4]thiazepine, 10,11-dihydrodibenz[b,f][1,4]oxazepine, 2,3,5,6,11,11a-hexahydro-lH-pyrrolo[2,1-b][3]benzazepine, 10,11-dihydro-5H-dibenzo[b,e][1,4]diazepine, 5,11-dihydrodibenz[b,e][1,4]oxazepine, 5,11-dihydrodibenzo[b,f][1,4]thiazepine, 10,11-dihydro-5H-dibenzo[b,e][1,4]diazepine, 1,2,3,3a,8,8a-hexahydropyrrolo[2,3-b]indole, and the like;
(2) as a phenyl group condensed with a dicyclic heterocyclic ring which is represented by the formula: 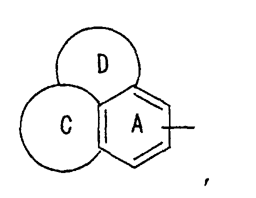 a group obtained by eliminating one hydrogen atom from a tricyclic condensed benzene ring such as 1H,3H-naphtho[1,8-cd][1,2]oxazine, naphtho[1,8-de]-1,3-oxazine, naphtho[1,8-de]-1,2-oxazine, 1,2,2a,3,4,5-hexahydrobenz[cd]indole, 2,3,3a,4,5,6-hexahydro-1H-benzo[de]quinoline, 4H-pyrrolo[3,2,1-ij]quinoline, 1,2,5,6-tetrahydro-4H-pyrrolo[3,2,1-ij]quinoline, 5,6-dihydro-4H-pyrrolo[3,2,1-ij]quinoline, 1H,5H-benzo[ij]quinolidine, azepino[3,2,1-hi]indole, 1,2,4,5,6,7-hexahydroazepino[3,2,1-hi]indole, 1H-pyrido[3,2,1-jk][1]benzazepine, 5,6,7,8-tetrahydro-1H-pyrido[3,2,1-jk][1]benzazepine, 1,2,5,6,7,8-hexahydro-1H-pyrido[3,2,1-jk][1]benzazepine, 2,3-dihydro-1H-benz[de]isoquinoline, 1,2,3,4,4a,5,6,7-octahydronaphtho[1,8-bc]-azepine, 2,3,5,6,7,8-hexahydro-1H-pyrido[3,2,1-jk][1]benzazepine, and the like;
(3) as phenyl group condensed with 2 the same or different monocyclic rings (provided that at least one ring is a monocyclic heterocyclic ring) and represented by the formula: 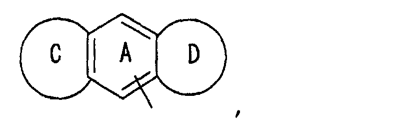 a group obtained by eliminating one hydrogen atom from a tricyclic condensed benzene ring such as 1,2,3,5,6,7-hexahydrobenzo[1,2-b:4,5-b']dipyrrole, 1,2,3,5,6,7-hexahydrocyclopento[f]indole, and the like;
(4) as phenyl group condensed with 2 the same or different monocyclic rings (provided that at least one ring is a monocyclic heterocyclic ring) represented by the formula: 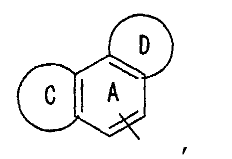 a group obtained by eliminating one hydrogen atom from a tricyclic condensed benzene ring such as 1,2,3,6,7,8-hexahydrocyclopento[e]indole, 2,3,4,7,8,9-hexahydro-1H-cyclopenta[f]quinoline, and the like.

Preferred examples of a case that the "aryl group" of the "aryl group which may be substituted" represented by Ar is condensed with a dicyclic heterocyclic ring which may be substituted include a group represented by the formula: 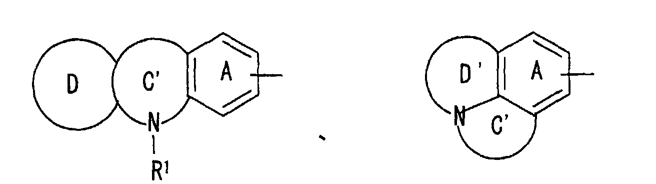 or 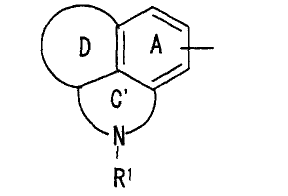 wherein each of ring C' and ring D' is a 5- to 9-membered nitrogen-containing heterocyclic ring which may be substituted with oxo group in addition to R¹, and ring A, ring D and R¹ are as defined above, etc.

Examples of the "5- to 9-membered nitrogen-containing heterocyclic ring" of said "5- to 9-membered nitrogen-containing heterocyclic ring which may be substituted with oxo group" include a 5- to 9-membered nitrogen-containing heterocyclic ring which may contain 1 to 3 of hetero atoms such as, for example, nitrogen atom, oxygen atom, sulfur atom and the like, in addition to carbon atom(s) and one nitrogen atom. Preferred examples thereof include a 5- to 9-membered non-aromatic nitrogen-containing heterocyclic ring (for example, pyrrolidine, piperidine, hexamethyleneimine, heptamethyleneimine, piperazine, homopiperazine, tetrahydrooxazepine, morpholine, thiomorpholine, and the like), etc.

More preferred examples of a case that the "aryl group" of the "aryl group which may be substituted" represented by Ar is condensed with a dicyclic heterocyclic ring which may be substituted include a group represented by the formula: 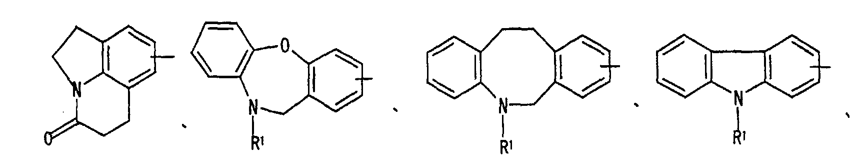 or 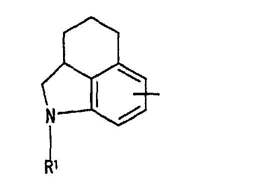 wherein R¹ is as defined above, etc.

Specific examples of a case that "phenyl group" of "phenyl group which may be substituted and may be condensed" is condensed with a tricyclic heterocyclic ring which may be substituted include a group represented by the formula: 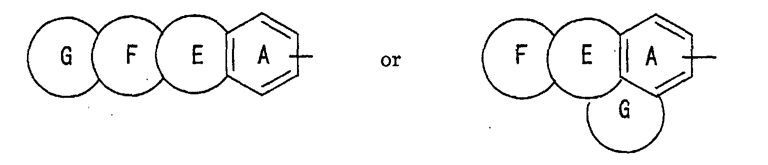 wherein ring A is as defined above, at least one ring among ring E, ring F and ring G is a heterocyclic ring which may be substituted, and the other rings are a 5- to 9-membered ring which may be substituted and may contain hetero atom(s), etc.

The "heterocyclic ring" and the "substituent" of the "heterocyclic ring which may be substituted" represented by ring E, ring F and ring G are as defined with respect to the "heterocyclic ring" and the "substituent" of the "heterocyclic ring which may be substituted" represented by the above ring C and ring D, etc.

The "5- to 9-membered ring which may contain hetero atom(s)" and the "substituent" of the "5- to 9-membered ring which may be substituted and may contain hetero atom(s)" represented by ring E, ring F and ring G are as defined with respect to the "5- to 9-membered ring which may contain hetero atom(s)" and the "substituent" of the "5- to 9-membered ring which may be substituted and may contain hetero atom(s)" represented by the above ring C and ring D, etc.

More specific examples of a case that "phenyl group" of "phenyl group which may be substituted and may be condensed" is condensed with a tricyclic heterocyclic ring which may be substituted include:
(1) as phenyl group condensed with a tricyclic heterocyclic ring which is represented by the formula: 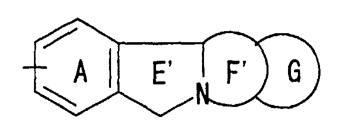 wherein ring E' and ring F' are as defined above, a group obtained by eliminating one hydrogen atom from a tetracyclic condensed benzene ring such as 2H-isoindolo[2,1-e]purine, 1H-pyrazolo[4',3':3,4]pyrido[2,1-a]isoindole, 1H-pyrido[2',3':4,5]imidazo[2,1-a]isoindole, 2H,6H-pyrido[1',2':3,4]imidazo[5,1-a]isoindole, 1H-isoindolo[2,1-a]benzimidazole, 1H-pyrido[3',4':4,5]pyrrolo[2,1-a]isoindole, 2H-pyrido[4',3':4,5]pyrrolo[2,1-a]isoindole, 1H-isoindolo[2,1-a]indole, 2H-isoindolo[1,2-a]isoindole, 1H-cyclopenta[4,5]pyrimido[2,1-a]isoindole, 2H,4H-pyrano[4',3':4,5][1,3]oxazino[2,3-a]isoindole, 2H-isoindolo[2,1-a][3,1]benzoxazine, 7H-isoindolo[1,2-b][1,3]benzoxazine, 2H-pyrido[2',1':3,4]pyrazino[2,1-a]isoindole, pyrido[2',3':4,5]pyrimido[2,1-a]isoindole, pyrido[3',2':5,6]pyrimido[2,1-a]isoindole, 1H-pyrido[1',2':3,4]pyrimido[2,1-a]isoindole, isoindolo[2,1-a]quinazoline, isoindolo[2,1-a]quinoxaline, isoindolo[1,2-a]isoquinoline, isoindolo[2,1-b]isoquinoline, isoindolo[2,1-a]quinoline, 6H-oxazino[3',4':3,4][1,4]diazepino[2,1-a]isoindole, azepino[2',1':3,4]pirazino[2,1-a]isoindole, 2H,6H-pyrido[2',1':3,4][1,4]diazepino[2,1-a]isoindole, 1H-isoindolo[1,2-b][1,3,4]benzotriazepine, 2H- isoindolo[2,1-a][1,3,4]benzotriazepine, isoindolo[2,1-d][1,4]benzoxazepine, 1H-isoindolo[2,1-b][2,4]benzodiazepine, 1H-isoindolo[2,1-c][2,3]benzodiazepine, 2H-isoindolo[1,2-a] [2,4]benzodiazepine, 2H-isoindolo[2,1-d][1,4]benzodiazepine, 5H-indolo[1,2-b][3]benzazepine, 2H-isoindolo[1,2-a] [2]benzazepine, 2H-isoindolo[2,1-b][3]benzazepine, 2H-isoindolo[2,1-b][2]benzazepine, 2H-isoindolo[1,2-b] [1,3,4]benzoxadiazocine, isoindolo[2,1-b][1,2,6]benzotriazocine, 5H-4,8-methano-1H-[1,5]diazacycloundecino[1,11-a]indole, and the like;
(2) as phenyl group condensed with a tricyclic heterocyclic ring which is represented by the formula: 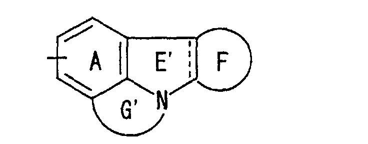 wherein ---- is a single bond or a double bond, and ring E' and ring F' are as defined hereinafter, a group obtained by eliminating one hydrogen atom from tetracyclic condensed benzene rings such as 1H,4H-pyrrolo[3',2':4,5]pyrrolo[3,2,1-ij]quinoline, pyrrolo[3,2,1-jk]carbazole, 1H-furo[2',3':4,5]pyrrolo[3,2,1-ij]quinoline, 1H,4H-cyclopenta[4,5]pyrrolo[1,2,3-de]quinoxaline, 1H,4H-cyclopenta[4,5]pyrrolo[3,2,1-ij]quinoline, pyrido[3',4':4,5]pyrrolo[1,2,3-de]benzoxazine, [1,4]oxazino[2,3,4-jk]carbazole, 1H,3H-[1,3]oxazino[5,4,3-jk]carbazole, pyrido[3',4':4,5]pyrrolo[1,2,3-de][1,4]benzothiazine, 4H-pyrrolo[3,2,1-de]phenanthridine, 4H,5H-pyrido[3,2,1-de]phenanthridine, 1H,4H-3a,6a-diazafluoroanthene, 1-oxa-4,6a-diazafluoroanthene, 4-oxa-2,10b-diazafluoroanthene, 1-thia-4,6a-diazafluoroanthene, 1H-pyrazino[3,2,1-jk]carbazole, 1H-indolo[3,2,1-de][1,5]naphthyridine, benzo[b]pyrano[2,3,4-hi]indolizine, 1H,3H-benzo[b]pyrano[3,4,5,-hi]indolizine, 1H,4H-pyrano[2',3':4,5]pyrrolo[3,2,1-ij]quinoline, 1H,3H-benzo[b]thiopyrano[3,4,5-hi]indolizine, 1H-pyrido[3,2,1-jk]carbazole, 4H-3-oxa-11b-azacyclohepta[jk]fluorene, 2H-azepino[1',2':1,2]pyrimidino[4,5-b]indole, 1H,4H-cyclohepta[4,5]pyrrolo[1,2,3-de]quinoxaline, 5H-pyrido[3',4':4,5]pyrrolo[1,2,3-ef][1,5]benzoxazepine, 4H-pyrido[3',4':4,5]pyrrolo[3,2,1-jk][4,1]benzothiazepine, 5H-pyrido[3',4':4,5]pyrrolo[1,2,3-ef][1,5]benzothiazepine, 5H-pyrido[4',3':4,5]pyrrolo[1,2,3-ef][1,5]benzothiazepine, [1,2,4]triazepino[6,5,4-jk]carbazole, [1,2,4]triazepino[6,7,1-jk]carbazole, [1,2,5]triazepino[3,4,5-jk]carbazole, 5H-[1,4]oxazepino[2,3,4-jk]carbazole, 5H-[1,4]thiazepino[2,3,4-jk]carbazole, [1,4]diazepino[3,2,1-jk]carbazole, [1,4]diazepino[6,7,1-jk]carbazole, azepino[3,2,1-jk]carbazole, 1H-cycloocta[4,5]pyrrolo[1,2,3-de]quinoxaline, 1H-cycloocta[4,5]pyrrolo[3,2,1-ij]quinoline, and the like;
(3) as phenyl group condensed with a tricyclic heterocyclic ring which is represented by the formula: 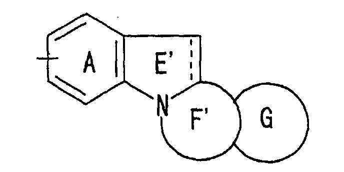 wherein ---- is a single bond or a double bond, and ring E' and ring F' is as defined hereinafter, a group obtained by eliminating one hydrogen atom from tetracyclic condensed benzene rings such as 1H-indolo[1,2-a]benzimidazole, 1H-indolo[1,2-b]indazole, pyrrolo[2',1':3,4]pyrazino[1,2-a]indole, 1H,5H-pyrrolo[1',2':4,5]pyrazino[1,2-a]indole, 2H-pyrido[2',3':3,4]pyrrolo[1,2-a]indole, 1H-pyrrolo [2',3':3,4]pyrido[1,2-a]indole, 1H-indolo[1,2-a]indole, 6H-isoindolo[2,1-a]indole, 6H-indolo[1,2-c][1,3]benzoxazine, 1H-indolo[1,2-b][1,2]benzothiazine, pyrimido[4',5':4,5]pyrimido[1,6-a]indole, pyrazino[2',3':3,4]pyrido[1,2-a]indole, 6H-pyrido[1',2':3,4]pyrimido[1,6-a]indole, indolo[1,2-b]cinnoline, indolo[1,2-a]quinazoline, indolo[1,2-c]quinazoline, indolo[2,1-b]quinazoline, indolo[1,2-a]quinoxaline, indolo[1,2-a][1,8]naphthyridine, indolo[1,2-b]-2,6-naphthyridine, indolo[1,2-b][2,7]naphthyridine, indolo[1,2-h]-1,7-naphthyridine, indolo[1,2-b]isoquinoline, indolo[2,1-a]isoquinoline, indolo[1,2-a]quinoline, 2H,6H-pyrido[2',1':3,4][1,4]diazepino[1,2-a]indole, 1H-indolo[2,1-c][1,4]benzodiazepine, 2H-indolo[1,2-d][1,4]benzodiazepine, 2H-indolo[2,1-a][2,3]benzodiazepine, 2H-indolo[2,1-b][1/3]benzodiazepine, 1H-indolo[1,2-b] [2]benzazepine, 2H-indolo[1,2-a][1]benzazepine, 2H-indolo[2,1-a] [2]benzazepine, indolo[1,2-e][1,5]benzodiazocine, indolo[2,1-b][3]benzazocine, and the like;
(4) as phenyl group condensed with a tricyclic heterocyclic ring which is represented by the formula: 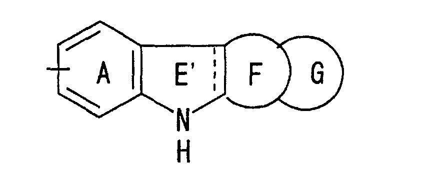 wherein ---- is a single bond or a double bond, and ring E' is as defined hereinafter, a group obtained by eliminating one hydrogen atom from tetracyclic condensed benzene rings such as lH-imidazo[1',2':1,2]pyrido[3,4-b]indole, 1H-imidazo[1',2':1,6]pyrido[4,3-b]indole, 1H-imidazo[1',5':1,2]pyrido[3,4-b]indole, 1H-imidazo[1',5':1,6]pyrido[4,3-b]indole, 1H-pyrido [2',1':2,3]imidazo[4,5-b]indole, imidazo[4,5-a]carbazole, imidazo[4,5-c]carbazole, pyrazolo[3,4-c]carbazole, 2H-pyrazino[1',2':1,5]pyrrolo[2,3-b]indole, 1H-pyrrolo[1',2':1,2]pyrimido[4,5-b]indole, 1H-indolidino[6,7-b]indole, 1H-indolidino[8,7-b]indole, indolo[2,3-b]indole, indolo[3,2-b]indole, pyrrolo[2,3-a]carbazole, pyrrolo[2,3-b]carbazole, pyrrolo[2,3-c]carbazole, pyrrolo[3,2-a]carbazole, pyrrolo[3,2-b]carbazole, pyrrolo[3,2-c]carbazole, pyrrolo[3,4-a]carbazole, pyrrolo[3,4-b]carbazole, pyrrolo[3,4-c]carbazole, 1H-pyrido[3',4':4,5]furo[3,2-b]indole, 1H-furo[3,4-a]carbazole, 1H-furo[3,4-b]carbazole, 1H-furo[3,4-c]carbazole, 2H-furo[2,3-a]carbazole, 2H-furo[2,3-c]carbazole, 2H-furo[3,2-a]carbazole, 2H-furo[3,2-c]carbazole, 1H-pyrido[3',4':4,5]thieno[2,3-b]indole,
   thieno[3',2':5,6]thiopyrano[4,3-b]indole, thieno[3',4':5,6]thiopyrano[4,3-b]indole, 1H-[1]benzothieno[2,3-b]indole, 1H-[1]benzothieno[3,2-b]indole, 1H-thieno[3,4-a]carbazole, 2H-thieno[2,3-b]carbazole, 2H-thieno[3,2-a]carbazole, 2H-thieno[3,2-b]carbazole, cyclopenta[4,5]pyrrolo[2,3-f]quinoxaline,
   cyclopenta[5,6]pyrido[2,3-b]indole, pyrido[2,'3':3,4]cyclopenta[1,2-b]indole, pyrido[2,'3':4,5]cyclopenta[1,2-b]indole,
   pyrido[3',4':3,4]cyclopenta[1,2-b]indole, pyrido[3',4':4,5]cyclopenta[1,2-b]indole, pyrido[4',3':4,5]cyclopenta[1,2-b]indole, 1H-cyclopenta[5,6]pyrano[2,3-b]indole, 1H-cyclopenta[5,6]thiopyrano[4,3-b]indole, cyclopenta[a]carbazole, cyclopenta[c]carbazole, indeno[1,2-b]indole, indeno[2,1-b]indole, [1,2,4]triazino[4',3':1,2]pyrido[3,4-b]indole, [1,3,5]triazino[1',2':1,1]pyrido[3,4-b]indole, 1H-[1, 4]oxazino[4',3':1,2]pyrido[3,4-b]indole, 1H-[1, 4]oxazino[4',3':1,6]pyrido[3,4-b]indole, 4H-[1, 3]oxazino[3',4':1,2]pyrido[3,4-b]indole, indolo[3,2-b][1/4]benzoxazine, 1,3-oxazino[6,5-b]carbazole, 2H-pyrimido[2',1':2,3][1,3]thiazino[5,6-b]indole, 2H-[1,3]thiazino[3',2':1,2]pyrido[3,4-b]indole, 4H-[1,3]thiazino[3',4':1,2]pyrido[3,4-b]indole, indolo[2,3-b][1,4]benzothiazine, indolo[3,2-b][1,4]benzothiazine, indolo[3,2-c][2,1]benzothiazine, 1,4-thiazino[2,3-a]carbazole, [1,4]thiazino[2,3-b]carbazole, 1,4-thiazino[2,3-c]carbazole, 1,4-thiazino[3,2-b]carbazole, 1,4-thiazino[3,2-c]carbazole, lH-indolo[2,3-g]pteridine, 1H-indolo[3,2-g]pteridine, pyrazino[1',2':1,2]pyrido[3,4-b]indole, pyrazino[1',2':1,2]pyrido[4,3-b]indole, 1H-pyrido[2',3':5,6]pyrazino[2,3-b]indole, 1H-pyrido[3',2':5,6]pyrazino[2,3-b]indole, 1H-pyrido[3',4':5,6]pyrazino[2,3-b]indole, pyrido[1',2':1,2]pyrimido[4,5-b]indole, pyrido[1',2':1,2]pyrimido[5,4-b]indole, pyrido[2',1':2;3]pyrimido[4,5-b]indole, pyrimido[1',2':1,2]pyrido[3,4-b]indole, pyrimido[1',2':1,6]pyrido[3,4-b]indole, pyrimido[5',4':5,6]pyrano[2,3-b]indole, pyridazino[4',5':5,6]thiopyrano[4,5-b]indole, 1H-indolo[3,2-c]cinnoline, 1H-indolo[2,3-b]quinoxaline, 1H-pyrazino[2,3-a]carbazole, 1H-pyrazino[2,3-b]carbazole, 1H-pyrazino[2,3-c]carbazole, 1H-pyridazino[3,4-c]carbazole, 1H-pyridozino[4,5-b]carbazole, 1H-pyrimido[4,5-a]carbazole, 1H-pyrimido[4,5-c]carbazole,1H-pyrimido[5,4-a]carbazole, 1H-pyrimido[5,4-b]carbazole, 1H-pyrimido[5,4-c]carbazole, 7H-1,4-dioxino[2',3':5,6][1,2]dioxino[3,4-b]indole, 6H-[1,4]benzodioxino[2,3-b]indole, 6H-[1,4]benzodithiino[2,3-b]indole, 1H-indolo[2,3-b]-1,5-naphthiridine, 1H-indolo[2,3-b]-[1,6]-naphthyridine, 1H-indolo[2,3-b]-[1,8]-naphthyridine, 1H-indolo[2,3-c]-1,5-naphthilidine, 1H-indolo[2,3-c]-[1,6]-naphthyridine, 1H-indolo[2,3-c]-[1,7]-naphthyridine, 1H-indolo[2,3-c]-[1,8]-naphthyridine, 1H-indolo[3,2-b]-1,5-naphthyridine, 1H-indolo[3,2-b]-[1,7]-naphthyridine, 1H-indolo[3,2-b]-[1,8]-naphthyridine, 1H-indolo[3,2-c]-[1,8]-naphthyridine, indolo[2,3-a]quinolidine, indolo[2,3-b]quinolidine, indolo[3,2-a]quinolidine, indolo[3,2-b]quinolidine, pyrano[4',3':5,6]pyrido[3,4-b]indole, pyrido[4',3':4,5]pyrano[3,2-b]indole, pyrido[4',3':5,6]pyrano[2,3-b]indole, pyrido[4',3':5,6]pyrano[3,4-b]indole, 1H-indolo[2,3-c]isoquinoline, 1H-indolo[3,2-c]isoquinoline, 1H-indolo[2,3-c]quinoline, 1H-indolo[3,2-c]quinoline, 1H-pyrido[2,3-a]carbazole, 1H-pyrido[2,3-b]carbazole, 1H-pyrido[2,3-c]carbazole, 1H-pyrido[3,2-a]carbazole, 1H-pyrido[3,2-b]carbazole, 1H-pyrido[3,2-c]carbazole, 1H-pyrido[3,4-a]carbazole, 1H-pyrido[3,4-b]carbazole, 1H-pyrido[3,4-c]carbazole, 1H-pyrido[4,3-a]carbazole, 1H-pyrido[4,3-b]carbazole, 1H-pyrido[4,3-c]carbazole, 1H-quindoline, 1H-quinindoline, 1H-pyrano[3',4':5,6]pyrano[4,3-b]indole, [1]benzopyrano[2,3-b]indole, [1]benzopyrano[3,2-b]indole, [1]benzopyrano[3,4-b]indole, [1]benzopyrano[4,3-b]indole, [2]benzopyrano[4,3-b]indole, pyrano[2,3-a]carbazole, pyrano[2,3-b]carbazole, pyrano[2,3-c]carbazole, pyrano[3,2-a]carbazole, pyrano[3,2-c]carbazole, pyrano[3,4-a]carbazole, 1H-phosphinorino[4,3-b]indole, [1]benzothiopyrano[2,3-b]indole, [1]benzothiopyrano[3,2-b]indole, [1]benzothiopyrano[3,4-b]indole, [1]benzothiopyrano[4,3-b]indole, [2]benzothiopyrano[4,3-b]indole, 1H-benzo[a]carbazole, 1H-benzo[b]carbazole, 1H-benzo[c]carbazole, [1,6,2]oxathiazepino[2',3':1,2]pyrido[3,4-b]indole, 1H-azepino[1',2':1,2]pyrido[3,4-b]indole, 1H-pyrido[1',2':1,2]azepino[4,5-b]indole, 2H-pyrido[1',2':1,2]azepino[3,4-b]indole, 1H-pyrido[3',2':5,6]oxepino[3,2-b]indole, 1H-pyrido[4',3':5,6]oxepino[3,2-b]indole, 2H-pyrido[2',3':5,6]oxepino[2,3-b]indole, 2H-pyrido[2',3':5,6]oxepino[3,2-b]indole, 2H-pyrido[3',4':5,6]oxepino[3,2-b]indole, pyrido[2',3':4,5]cyclohepta[1,2-b]indole, pyrido[3',2':3,4]cyclohepta[1,2-b]indole, pyrido[3',4':4,5]cyclohepta[1,2-b]indole, pyrido[3',4':5,6]cyclohepta[1,2-b]indole, 2H-pyrano[3',2':2,3]azepino[4,5-b]indole, 1H-indolo[3,2-b][1,5]benzoxazepin, 1H-indolo[3,2-d][1,2]benzoxazepin, 1H-indolo[2,3-c][1,5]benzothiazepin, [1,4]diazepino[2,3-a]carbazole, indolo[2,3-b][1,5]benzodiazepin, indolo[2,3-d][1,3]benzodiazepin, indolo[3,2-b][1,4]benzodiazepin, indolo[3,2-b][1,5]benzodiazepin, indolo[3,2-d] [1,3]benzodiazepin, indolo[3,2-d][2,3]benzodiazepin, indolo[2,3-a][3]benzazepin, indolo[2,3-c][1]benzazepin, indolo[2,3-d][1]benzazepin, indolo[2,3-d][2]benzazepin, indolo[3,2-b][1]benzazepin, indolo[3,2-c][1]benzazepin, indolo[3,2-d][1]benzazepin, 1H-indolo[2,1-b][3]benzazepin, 1H-[1]benzoxepino[5,4-b]indole, 1H-[2]benzoxepino[4,3-b]indole, 1H-[1]benzothiepino[4,5-b]indole, 1H-[1]benzothiepino[5,4-b]indole, benzo[3,4]cyclohepta[1,2-b]indole, benzo[4,5]cyclohepta[1,2-b]indole, benzo[5,6]cyclohepta[1,2-b]indole, benzo[6,7]cyclohepta[1,2-b]indole, cyclohepta[b]carbazole, 4H-[1,5]oxazocino[5',4';1,6]pyrido[3,4-b]indole, azocino[1',2':1,2]pyrido[3,4-b]indole, 2,6-methano-2H-azecino[4,3-b]indole, 3,7-methano-3H-azecino[5,4-b]indole, pyrido[1',2':1,8]azocino[5,4-b]indole, pyrido[4',3':6,7]oxocino[2,3-b]indole, pyrido[4',3':6,7]oxocino[4,3-b]indole, 1,5-methano-1H-azecino[3,4-b]indole, 2, 6-methano-1H-azecino[5,4-b]indole, 1H-pyrido[3',4':5,6]cycloocta[1,2-b]indole, 1,4-ethanooxocino[3,4-b]indole, pyrano[3',4':5,6]cycloocta[1,2-b]indole, 1H-indolo[2,3-c][1,2,5,6]benzotetrazocine, 1H-indolo[2,3-c][1, 6]benzodiazocine, 6,13b-methano-13bH-azecino[5,4-b]indole, oxocino[3,2-a]carbazole, 1H-benzo[g]cycloocta[b]indole, 6,3-(iminomethano)-2H-1,4-thiazonino[9,8-b]indole, 1H,3H-[1,4]oxazonino[4',3':1,2]pyrido[3,4-b]indole, 2H-3,6-ethanoazonino[5,4-b]indole, 2H-3,7-methanoazacycloundecino[5,4-b]indole, 1H-6,12b-ethanoazonino[5,4-b]indole, indolo[3,2-e][2]benzazonine, 5,9-methanoazacycloundecino[5,4-b]indole, 3,6-ethano-3H-azecino[5,4-b] indole, 3,7-methano-3H-azacycloundecino[5,4-b]indole, pyrano[4',3':8,9]azecino[5,4-b]indole, 1H-indolo[2,3-c][1,7]benzodiazecine, 1H-indolo[3,2-e] [2]benzazecine, and the like.
   Further, there is mentioned a group obtained by eliminating one hydrogen atom from tetracyclic condensed benzene rings such as benzo[e]pyrrolo[3,2-b]indole, benzo[e]pyrrolo[3,2-g]indole, benzo[e]pyrrolo[3,2,1-hi]indole, benzo[e]pyrrolo[3,4-b]indole, benzo[g]pyrrolo[3,4-b]indole, 1H-benzo[f]pyrrolo[1,2-a]indole, 1H-benzo[g]pyrrolo[1,2-a]indole, 2H-benzo[e]pyrrolo[1,2-a]indole, 1H-benzo[f]pyrrolo[2,1-a]isoindole, 1H-benzo[g]pyrrolo[2,1-a]isoindole, 2H-benzo[e]pyrrolo[2,1-a]isoindole, isoindolo[6,7,1-cde]indole, spiro[cyclohexane-1,5'-[5H]pyrrolo[2,1-a]isoindole], isoindolo[7,1,2-hij]quinoline, 7,11-methanoazocino[1,2-a]indole, 7,11-methanoazocino[2,1-a]isoindole, dibenz[cd,f]indole, dibenz[cd,g]indole, dibenz[d,f]indole, 1H-dibenz[e,g]indole, 1H-dibenz[e,g]isoindole, naphtho[1,2,3-cd]indole, naphtho[1,8-ef]indole, naphtho[1,8-fg]indole, naphtho[3,2,1-cd]indole, 1H-naphtho[1,2-e]indole, 1H-naphtho[1,2-f]indole, 1H-naphtho[1,2-g]indole, 1H-naphtho[2,1-e]indole, 1H-naphtho[2,3-e]indole, 1H-naphtho[1,2-f]isoindole, 1H-naphtho[2,3-e]isoindole, spiro[1H-carbazol-1,1'-cyclohexane], spiro[2H-carbazol-2,1'-cyclohexane], spiro[3H-carbazol-3,1'-cyclohexane], cyclohepta[4,5]pyrrolo[3,2-f]quinoline, cyclohepta[4,5]pyrrolo[3,2-h]quinoline, azepino[4,5-b]benz[e]indole, 1H-azepino[1,2-a]benz[f]indole, 1H-azepino[2,1-a]benz[f]isoindole, benzo[e]cyclohepta[b]indole, benzo[g]cyclohepta[b]indole, and the like; or
(5) as phenyl group condensed with a tricyclic heterocyclic ring which is represented by the formula: 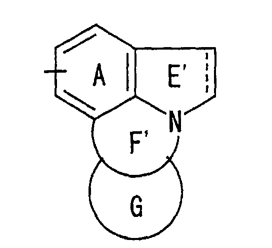 wherein ---- is a single bond or a double bond, and ring E' and ring F' are as defined hereinafter, a group obtained by eliminating one hydrogen atom from tetracyclic condensed benzene rings such as lH-dipyrrolo[2,3-b:3',2',1'-hi]indole, spiro[cyclopentan-1,2'-(1'H)-pyrrolo[3,2,1-hi]indole], spiro[imidazolidin-4,1'-(2'H)-[4H]pyrrolo[3,2,1-ij]quinoline], pyrido[2,3-b]pyrrolo[3,2,1-hi]indole, pyrido[4,3-b]pyrrolo[3,2,1-hi]indole, benzo[de]pyrrolo[3,2,1-ij]quinoline, 3H-pyrrolo[3,2,1-de]acridine, 1H-pyrrolo[3,2,1-de]phenanthridine, spiro[cyclohexan-1,6'-(6H)-pyrrolo[3,2,1-ij]quinoline], 4,9-methanopyrrolo[3,2,1-lm][1]benzazocine, spiro[cycloheptan-1,6'-(6H)-pyrrolo[3,2,1-ij]quinoline], 1H-pyrano[3,4-d]pyrrolo[3,2,1-jk][1]benzazepine, 3H-benzo[b]pyrrolo[3,2,1-jk][4,1]benzoxazepine, 7H-indolo[1,7-ab][4,1]benzoxazepine, benzo[b]pyrrolo[3,2,1-jk][1,4]benzodiazepine, indolo[1,7-ab][1,4]benzodiazepine, indolo[1,7-ab][1]benzazepine, indolo[7,1-ab][3]benzazepine, 1H-cyclohepta[d][3,2,1-jk][1]benzazepine, spiro[azepino[3,2,1-hi]indole-7(4H), 1'-cycloheptane], 4H-5,11-methanopyrrolo[3,2,1-no][1]benzazacycloundecine, spiro[azepino[3,2,1-hi]indole-7(4H),1'-cyclooctane], and the like.

Further, examples of the "phenyl group condensed with a tricyclic heterocyclic ring" include phenyl group condensed with a tricyclic heterocyclic ring exemplified hereinafter, and its dihydro isomer, tetrahydro isomer, hexahydro isomer, octahydro isomer and decahydro isomer, in addition to the above phenyl group condensed with a tricyclic heterocyclic ring including the indole ring or isoindole ring which may be hydrogenated. Specific examples thereof include fluoranthene, acephenanthrylene, aceanthrylene, triphenylene, pyrene, chrysene, naphthacene, pleiadene, benzo[a]anthracene, indeno[1,2-a]indene, cyclopenta[a]phenanthrene, pyrido[1',2':1,2]imidazo[4,5-b]quinoxaline, 1H-2-oxapyrene, spiro[piperidine-4,9'-xanthene], and the like.

Preferred examples of a case that the "phenyl group" of the "phenyl group which may be substituted and may be condensed" is condensed with a tricyclic heterocyclic ring which may be substituted include a group represented by the formula: 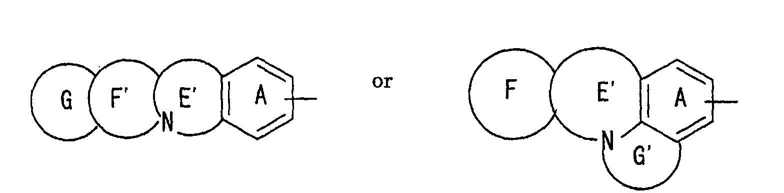 wherein each of ring E', ring F' and ring G' is a 5- to 9-membered nitrogen-containing heterocyclic ring which may be substituted with oxo group in addition to R¹, and ring A, ring F, ring G and R¹ are as defined above, etc.

Among these, , in particular, a group represented by the formula: 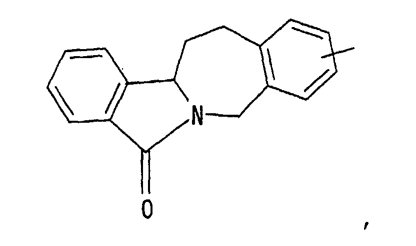 and the like are preferred.

Examples of the "5- to 9-membered nitrogen-containing heterocyclic ring" of the "5- to 9-membered nitrogen-containing heterocyclic ring which may be substituted with oxo group" include the "5- to 9-membered nitrogen-containing heterocyclic ring" represented by the above ring C' and ring D', etc.

Preferred examples of a case that the "aryl group which may be substituted" represented by Ar is condensed with (2) a dicyclic heterocyclic ring which may be substituted, or a case that it is condensed with 2 the same or different monocyclic rings (provided that at least one ring is a monocyclic heterocyclic ring), and a case that it is condensed with (3) a tricyclic heterocyclic ring which may be substituted include a group wherein Ar is represented by the formula: 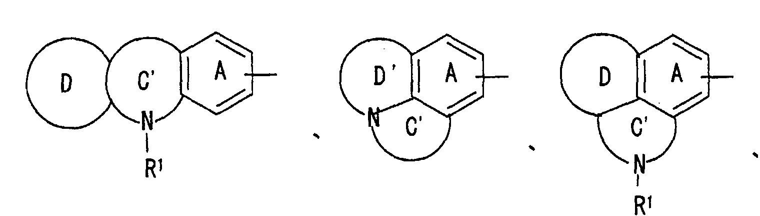 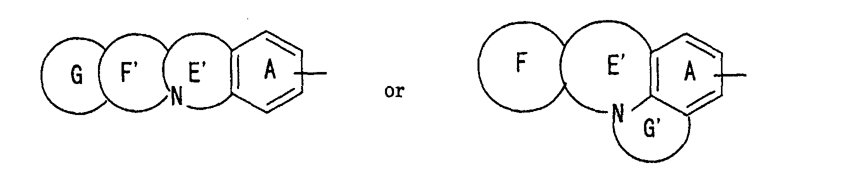 wherein each symbol is as defined above.

In particular, preferred examples of the "aryl group which may be substituted" represented by Ar include a group represented by the formula: 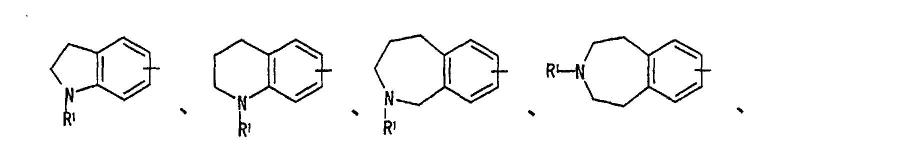 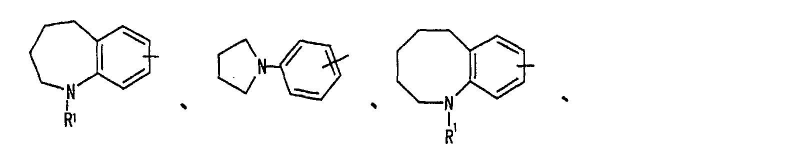 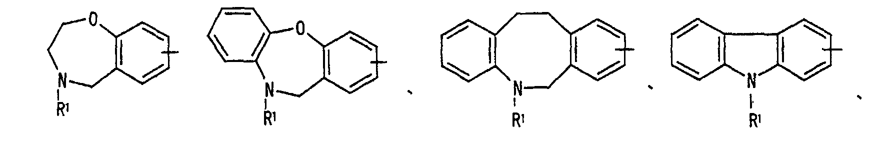 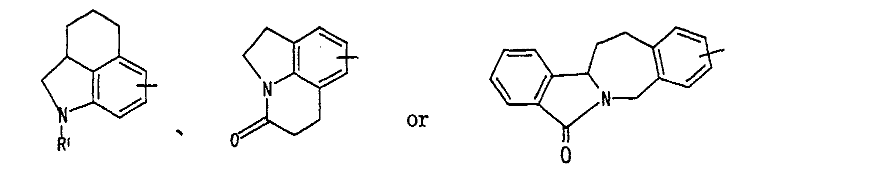 wherein R¹ is as defined above, etc. Among these, particularly preferred one is a group represented by the formula: 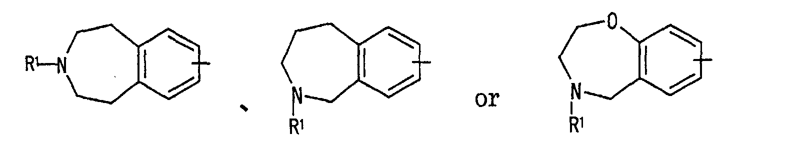 wherein R¹ is as defined above.

In the above formula, n is an integer of 1 to 10. n is preferably an integer of 1 to 6, more preferably an integer of 1 to 5, in particular, further preferably an integer of 2 to 5, and especially preferably 3, 4 or 5.

In the above formula, R is a hydrogen atom or a hydrocarbon group which may be substituted, and may be different in the repetition of n.

The "hydrocarbon group" and the "substituent" of the "hydrocarbon group which may be substituted" represented by R is as defined with respect to the "hydrocarbon group" and the "substituent" of the "hydrocarbon group which may be substituted" represented by the above R¹.

Further, R may be bonded to Ar or the substituent of Ar.

Examples of the compound represented by the formula (Ic) in which R is bonded to Ar or the substituent of Ar include a compound represented by the formula: 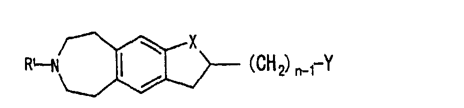 wherein R¹, n, X and Y are as defined above;
a compound represented by the formula: 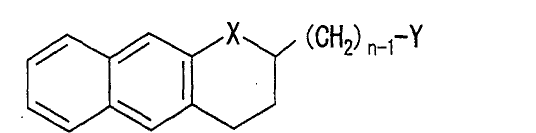 wherein n, X and Y are as defined above;
a compound represented by the formula: 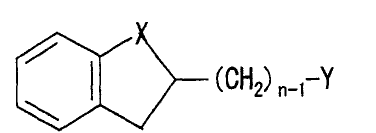 wherein n, X and Y are as defined above; etc.

As R, preferred is a hydrogen atom.

In the above formula, Y is an amino group which may be substituted, or a nitrogen-containing heterocyclic ring (preferably, a nitrogen-containing saturated heterocyclic ring) which may be substituted [Y is preferably amino group which may be substituted]. Further, Y' is an amino group which may be substituted.

Examples of the "amino group which may be substituted" represented by Y and Y' include a group represented by the formula: 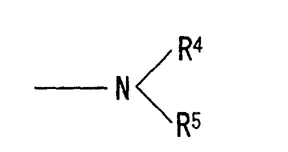 wherein R⁴ and R⁵ are the same or different and are hydrogen atom, a hydrocarbon group which may be substituted, or an acyl group which may be substituted, or R⁴ and R⁵ may be bonded to each other to form a ring, etc.

Examples of the "substituent" and the "hydrocarbon group" of the "hydrocarbon group which may be substituted" represented by R⁴ and R⁵ includes the "substituent" and the "hydrocarbon group" of the "hydrocarbon group which may be substituted" represented by the above R¹, and the like

Preferred examples of the hydrocarbon group which may be substituted represented by R⁴ and R⁵ include (a) a straight or branched chain lower alkyl group (for example, C₁₋₆ alkyl group such as methyl, ethyl, propyl, isopropyl, butyl, isobutyl, tert-butyl, sec-butyl, pentyl, hexyl, and the like) which may have 1 to 3 substituent(s) selected from (i) a halogen atom (,for example, fluoro, chloro, bromo, iodo, and the like), (ii) a lower alkoxy group (for example, C₁₋₆ alkoxy group such as methoxy, ethoxy, n-propoxy, isopropoxy, n-butyloxy, and the like), (iii) hydroxy group, and the like; (b)a lower aralkyl group (for example, phenyl-C₁₋₁₀ alkyl (for example, benzyl, phenylethyl, phenylpropyl, phenylbutyl, phenylpentyl, phenylhexyl, and the like), naphthyl-C₁₋₆ alkyl (for example, α-naphthylmethyl, and the like) or C₇₋₁₆ aralkyl group such as diphenyl-C₁₋₃ alkyl (for example, diphenylmethyl, diphenylethyl, and the like) which may have 1 to 3 substituent(s) selected from (i) a halogen atom (for example, fluoro, chloro, bromo, iodo, and the like), (ii) a lower alkoxy group (for example, C₁₋₆ alkoxy group such as methoxy, ethoxy, n-propoxy, i-propoxy, n-butyloxy, and the like), (iii) hydroxy group, and the like.

More preferred examples thereof include (a) a straight or branched chain unsubstituted lower alkyl group (for example, C₁₋₆ alkyl group such as methyl, ethyl, propyl, isopropyl, butyl, isobutyl, tert-butyl, sec-butyl, pentyl, hexyl, and the like), or (b)an unsubstituted lower aralkyl group (for example, phenyl-C₁₋₁₀ alkyl (for example, benzyl, phenylethyl, phenylpropyl, phenylbutyl, phenylpentyl, phenylhexyl, and the like), naphthyl-C₁₋₆ alkyl (for example, α- naphthylmethyl, and the like) or C₇₋₁₆ aralkyl group such as diphenyl-C₁₋₃ alkyl (for example, diphenylmethyl, diphenylethyl, and the like)), and the like.

Examples of the "acyl group which may be substituted" represented by R⁴ and R⁵ include the "acyl group which may be substituted" represented by the above R¹, etc.

Further, in the "amino group which may be substituted" represented by Y and Y', specific examples of a case that R⁴ and R⁵ are bonded to each other to form a ring, namely, a case that the "amino group which may be substituted" represented by Y and Y' are the "cyclic amino group which may be substituted", include a group represented by the formula: 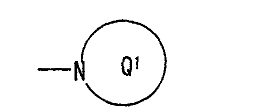 wherein ring Q¹ is a 5- to 9-membered nitrogen-containing heterocyclic group (preferably, a nitrogen-containing saturated heterocyclic group) which may contain 1 to 2 hetero atoms selected from nitrogen atom, oxygen atom, sulfur atom, and the like in addition to carbon atom(s) and one nitrogen atom, etc. More specifically, for example, 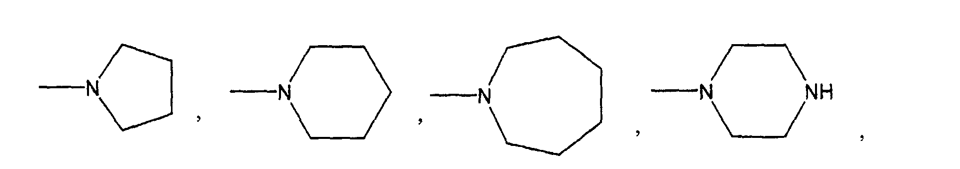 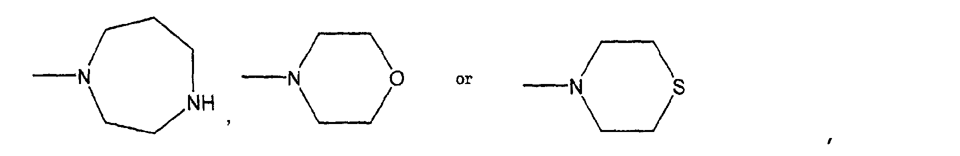 or the like are often used;

Examples of the "substituent" of the "cyclic amino group which may be substituted" as the "amino group which may be substituted" represented by Y and Y' include the "substituent" of the "nitrogen-containing heterocyclic ring which may be substituted" which may be formed together with nitrogen atom adjacent to the above R^{2c} and R^{3c}, and the "hydrocarbon group which may be substituted", the "acyl group which may be substituted", or the "heterocyclic group which may be substituted" represented by the above R^{1c}, etc.

Preferred examples of the "amino group which may be substituted" represented by Y and Y' include a group represented by the formula (1): 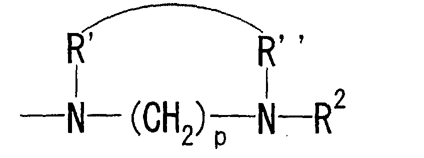 wherein R² is a hydrogen atom, an acyl group which may be substituted, a hydrocarbon group which may be substituted, or a heterocyclic group which may be substituted, p is an integer of 1 to 3, each of R' and R'' is a hydrogen atom or an alkyl group which may be substituted, or R' and R'' may be bonded to each other to form a ring, (2) a piperidino group which may be substituted, etc. Among these, preferably, a group represented by the formula (1a): 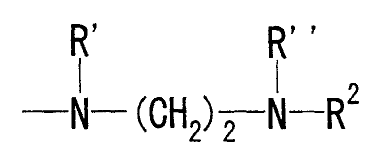 wherein R² is a hydrogen atom, an acyl group which may be substituted, a hydrocarbon group which may be substituted, or a heterocyclic group which may be substituted, and each of R' and R'' is a hydrogen atom or an alkyl group which may be substituted, a group represented by the formula (1b): 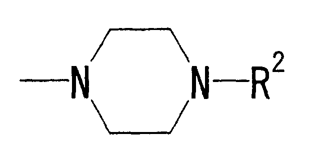 wherein R² is a hydrogen atom, an acyl group which may be substituted, a hydrocarbon group which may be substituted, or a heterocyclic group which may be substituted, etc., are used.

Examples of the "acyl group which may be substituted", the "hydrocarbon group which may be substituted", and the "heterocyclic group which may be substituted" represented by R² include those similar to the "acyl group which may be substituted", the "hydrocarbon group which may be substituted", and the "heterocyclic group which may be substituted" represented by the above R¹.

Examples of the "alkyl group" in the "alkyl group which may be substituted" represented by R' and R'' include a C₁₋₆ alkyl group, and the like. Examples of the "substituent" of said "alkyl group" include those similar to the "substituent" of the "hydrocarbon group which may be substituted" represented by the above R¹.

Further, when R' and R'' are bonded to each other to form a ring, preferred examples thereof include, among the "nitrogen-containing heterocyclic group" exemplified with respect to the above ring Q¹, a 5- to 9-membered nitrogen-containing heterocyclic group (preferably, a nitrogen-containing saturated heterocyclic group) which may contain one hetero atom selected from nitrogen atom, oxygen atom, sulfur atom, and the like, in addition to carbon atom(s) and two nitrogen atoms, and, preferably, the ring is the 5-to 9-membered nitrogen-containing heterocyclic group (preferably, a nitrogen-containing saturated heterocyclic group) which is constituted by carbon atom(s) and two nitrogen atoms. These rings may be further substituted with substituent(s) similar to those of the above ring Q¹.

The piperidino group which may be substituted as Y may have the "acyl group which may be substituted", the "hydrocarbon group which may be substituted", and the "heterocyclic group which may be substituted" represented by the above R¹, and the like, as substituent(s).

Examples of the "nitrogen-containing heterocyclic group" of the "nitrogen-containing heterocyclic group which may be substituted" represented by Y include a 5- to 9-membered nitrogen-containing heterocyclic group (preferably, a nitrogen-containing saturated heterocyclic group) which may contain one to three hetero atoms selected from nitrogen atom, oxygen atom, sulfur atom, and the like, in addition to carbon atom(s) and one nitrogen atom, etc. These nitrogen-containing heterocyclic groups may be a group having a bonding hand at the nitrogen atom composing the ring, or a group having a bonding hand at the carbon atom composing the ring. Examples of the group having a bonding hand at the nitrogen atom composing the ring include a group represented by the formula: 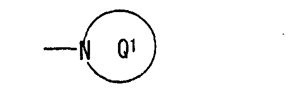 wherein ring Q¹ is a 5- to 9-membered nitrogen-containing heterocyclic group (preferably, a nitrogen-containing saturated heterocyclic group) which may contain 1 to 2 hetero atoms selected from nitrogen atom, oxygen atom, sulfur atom, and the like in addition to carbon atom(s) and one nitrogen atom, etc. More specifically, for example, 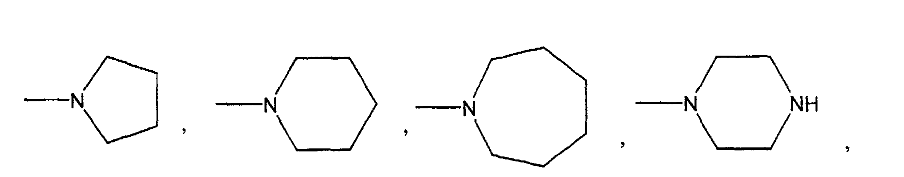 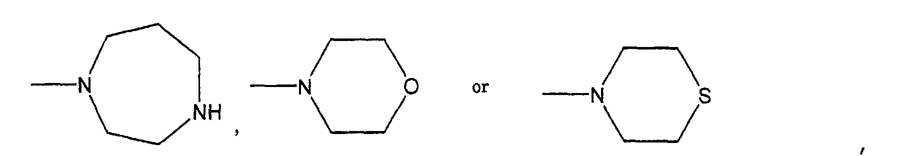 or the like is often used.

Examples of the group having the bonding hand at a carbon atom composing the ring include a group represented by the formula: 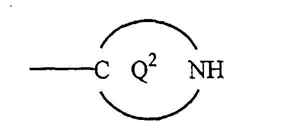 wherein ring Q² represents a 5- to 9-membered nitrogen-containing heterocyclic group (preferably, a nitrogen-containing saturated heterocyclic group) which may contain 1 to 2 hetero atoms selected from nitrogen atom, oxygen atom, sulfur atom, and the like, in addition to carbon atom(s) and one nitrogen atom, etc. More specifically, for example, 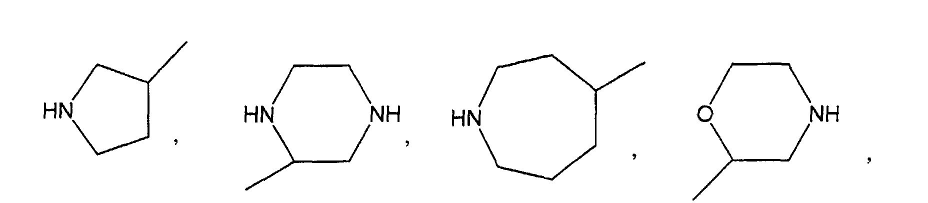 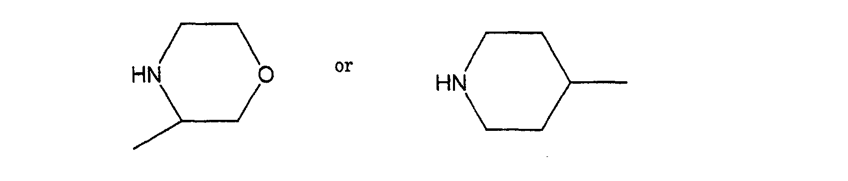 or the like is often used.

Examples of the "substituent" of the "nitrogen-containing heterocyclic group (preferably, a nitrogen-containing saturated heterocyclic group) which may be substituted" represented by Y include the "substituent" of the "nitrogen-containing heterocyclic ring which may be substituted" which may be formed together with the nitrogen atom adjacent to the above R^{2c} and R^{3c}, and the "hydrocarbon group which may be substituted", the "acyl group which may be substituted", or the "heterocyclic group which may be substituted" represented by the above R¹, etc.

Further, when the "cyclic amino group which may be substituted" as the "amino group which may be substituted" represented by Y and Y'; and the "nitrogen-containing heterocyclic group which may be substituted" represented by Y have 2 or more substituents, said substituents may be bonded to each other to form a ring. Specific example of the ring include benzene ring, a 5- to 8-membered (preferably, 5- to 6-membered) aromatic monocyclic heterocyclic ring (for example, pyrrole, oxazole, isoxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, and the like), a ring in which a part or all of the unsaturated bonds of these rings converted to saturated bond(s), and the like.

Furthermore, when the "cyclic amino group which may be substituted" as the "amino group which may be substituted" represented by Y and Y'; and the "nitrogen-containing heterocyclic group which may be substituted" represented by Y have 2 or more substituents on one carbon atom, said substituents may be bonded to each other to form a spiro ring. Specific examples of the case of forming the spiro ring include spiro(1H-inden-1,4'-piperizinyl) ring, and the like.

Preferred examples of the "nitrogen-containing heterocyclic group" of the "nitrogen-containing heterocyclic group which may be substituted" represented by Y include a 4-piperidinyl group, 1-piperidinyl group, 1-piperazinyl group, and the like.

Namely, preferably, Y is a group represented by the formula: 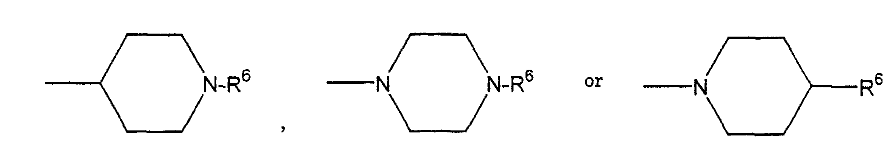 wherein R⁶ is as defined with respect to the above R¹; etc.

More preferably, Y is, for example, a group represented by the formula: 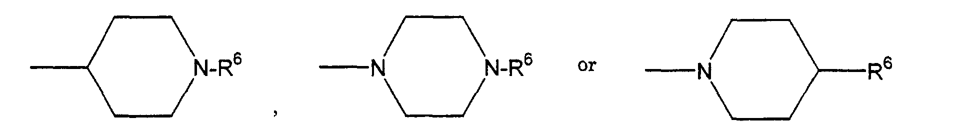 wherein R⁶ is (i) phenyl-C₁₋₆ alkyl which may be substituted with C₁₋₆ alkyl, C₁₋₆ alkoxy, a halogen atom, nitro, mono- or di-C₁₋₆ alkyl-carbamoyloxy, hydroxy, cyano, carboxyl, C₁₋₆ alkoxy-carbonyl, carbamoyl, cyclic aminocarbonyl, amino, C₁₋₆ alkylcarbonylamino, phenylsulfonylamino, C₁₋₆ alkylsulfonylamino, amidino, ureido or a heterocyclic ring (the above C₁₋₆ alkyl, C₁₋₆ alkoxy, carbamoyl, cyclic aminocarbonyl, amino, phenylsulfonylamino, amidino, ureido and heterocyclic ring may be further substituted, and as said "substituent", for example, the "substituent" of the "hydrocarbon group which may be substituted" represented by R¹, and the like are used), (ii) hydrogen atom, (iii) a C₁₋₆ alkyl group which may be substituted with a halogen atom, hydroxy, C₁₋₆ alkoxy, amino, mono- or di-C₁₋₆ alkylamino, carboxyl, cyano or C₁₋₆ alkoxy-carbonyl, or (iv) a C₁₋₆ alkylcarbonyl group which may be substituted with mono- or di-C₁₋₆ alkylamino or C₁₋₆ alkoxy-carbonyl , and preferably, benzyl group which may be substituted with C₁₋₄ alkyl (methyl, and the like), trihalogeno C₁₋₄ alkyl (methyl, and the like), a halogen atom (fluoro, chloro, and the like), nitro, cyano, C₁₋₄ alkoxy (methoxy, and the like), hydroxy, carbamoyl, (4-C₁₋₄ alkyl (methyl, and the like)-1-piperazinyl) carbonyl, aminothiocarbonyl, morpholinocarbonyl, carboxyl, C₁₋₄ alkoxy (methoxy, and the like) carbonyl, C₁₋₄ alkoxy (ethoxy, and the like), C₁₋₄ alkoxy (ethoxy, and the like), carboxyl-C₁₋₄ alkoxy (methoxy, and the like), C₁₋₄ alkoxy (ethoxy, and the like), carboxyl C₁₋₄ alkoxy (methoxy, and the like) -carbonyl-C₁₋₆alkyl (isopropyl, and the like), carboxyl-C₁₋₆ alkyl (isopropyl, and the like), amino, acetylamino, C₁₋₄ alkyl (methyl, and the like) sulfonylamino, (4-C₁₋₄ alkyl (methyl, and the like) phenyl)sulfonylamino, ureido, 3-C₁₋₄ alkyl (methyl, and the like) ureido, amidino, dihydrothiazolyl or dihydroimidazolyl, and the like.

Among these, preferred are those in which R⁶ is benzyl group which may be substituted with C₁₋₄ alkyl (methyl, and the like), trihalogeno (fluoro, and the like) C₁₋₄ alkyl (methyl, and the like), a halogen atom (fluoro, chloro, and the like), nitro, hydroxy, carbamoyl, amino, amidino, or dihydroimidazolyl.

In particular, Y is preferably 1-benzyl-4-piperidinyl group, 4-benzyl-1-piperidinyl group or 4-benzyl-1-piperazinyl group, 1-acetyl-4-piperidinyl group, 1-[(2-methylphenyl)methyl]-4-piperidinyl group, 1-[(3-chlorophenyl)methyl]-4-piperidinyl group, 1-[(2-chlorophenyl)methyl]-4-piperidinyl group, 1-[(3-nitrophenyl)methyl]-4-piperidinyl group, 1-[[3-trifluoromethyl)phenyl]methyl]-4-piperidinyl group, and the like, and 1-benzyl-4-piperidinyl group, 1-acetyl-4-piperidinyl group, 1-[(2-methylphenyl)methyl]-4-piperidinyl group, 1-[(3-chlorophenyl)methyl]-4-piperidinyl group, 1-[(2-chlorophenyl)methyl]-4-piperidinyl group, 1-[(3-nitrophenyl)methyl]-4-piperidinyl group, 1-[[3-(trifluoromethyl)phenyl]methyl]-4-piperidinyl group, and the like are often used.

In the above formula, examples of the "spacer in which the number of atom(s) constituting a linear chain portion is 1 to 4" represented by X include a saturated divalent group or divalent group in which a part of bonds is converted to unsaturated bond(s) such as
(1) -(CH₂)_{f3}- (f3 is an integer of 1 to 4),
(2) -(CH₂)_{g5}-X⁵-(CH₂)_{g6}- (g5 and g6 are the same or different and are an integer of 0 to 3, provided that the sum of g5 and g6 is 1 to 3. X⁵ is NH, O, S, SO or SO₂),
(3) -(CH₂)ₕ₇-X⁵-(CH₂)ₕ₈- X⁶-(CH₂)ₕ₉- (h7, h8 and h9 are the same or different and are an integer of 0 to 2, provided that the sum of h7, h8 and h9 is 0 to 2. Each of X⁵ and X⁶ is NH, O, S, SO or SO₂). However, when h8 is 0, at least one of X⁵ and X⁶ is preferably NH); or the "the divalent group in which the number of atom(s) in a linear chain portion is 1 to 4" such as -CO-, -O-, -NR^{3a}-, -S-, -SO₂-, -SO₂NR^{3a}-, -SO₂NHCONR^{3a}-, -SO₂NHC(=NH)NR^{3a}-, -CS-, -CR^{3a}(R^{3b})-, -C(=CR^{3a}(R^{3b})-, -C(=NR^{3a})-, -CONR^{3a}- (wherein each of R^{3a} and R^{3b} is independently hydrogen atom, cyano group, hydroxy group, amino group, a C₁₋₆ alkyl group or a C₁₋₆ alkoxy group).

X is more preferably -CO-, -O-, -NR^{3a}-, -S-, -SO-, -SO₂-, -SO₂NR^{3a}-, -SO₂NHCONR^{3a}-, -SO₂NHC(=NH)NR^{3a}-, -CS-, -CR^{3a}(R^{3b})-, -C(=CR^{3a}(R^{3b})-, -C(=NR^{3a})-, -CONR^{3a}- (wherein each of R^{3a} and R^{3b} is independently hydrogen atom, cyano group, hydroxy group, amino group, a C₁₋₆ alkyl group or a C₁₋₆ alkoxy group). Among these, -CO-, -O-, -SO₂-, -SO₂NR^{3a}-, -CR^{3a}(R^{3b})-, -CONR^{3a}-, and the like are preferred. In particular, -SO₂NR^{3a}-, -CONR^{3a}-, -CR^{3a}(R^{3b})-, and the like are preferably used.

The divalent group represented by X may be substituted at an arbitrary position (preferably, on a carbon atom), and examples of the substituent include lower (C₁₋₆) alkyl (for example, methyl, ethyl, propyl, isopropyl, butyl, isobutyl, sec-butyl, tert-butyl, pentyl, isopentyl, neopentyl, hexyl, and the like), lower (C₃₋₇) cycloalkyl (for example, cyclopropyl, cyclobutyl, cyclopentyl, cyclohexyl, cycloheptyl, and the like), formyl, lower (C₂₋₇) alkanoyl (for example, acetyl, propionyl, butyryl, and the like), lower (C₁₋₆) lower alkoxy-carbonyl, lower (C₁₋₆) lower alkoxy, hydroxy group, oxo, and the like.

Among the compound represented by the formula (Ic) or a salt thereof, a compound represented by the formula (IIc), 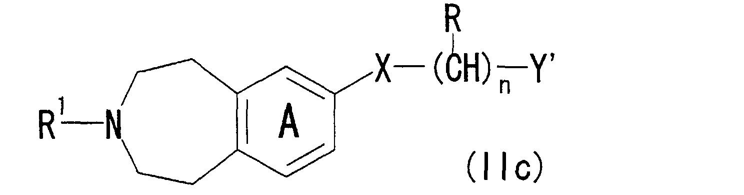 wherein R¹ is a hydrogen atom, a hydrocarbon group which may be substituted or an acyl group which may be substituted, ring A is a benzene ring which may be further substituted, X is a spacer in which the number of atom(s) constituting a linear chain portion is 1 to 4 (provided that -CO- is excluded), n is an integer of 1 to 10, R is a hydrogen atom or a hydrocarbon group which may be substituted, and may be the same or different in the repetition of n, R may be bonded to ring A or a substituent of ring A to form a ring, and Y is an amino group which may be substituted, or a salt thereof is preferably used.

As the salt of the compound having urotensin II receptor antagonistic activity [including the compounds represented by the formulae (Ia), (IIa), (IIa'), (Ib), (Ic) and (IIc)] is used in the present invention, a pharmacologically acceptable salt is preferred, and examples thereof include a salt with an inorganic base, a salt with an organic base, a salt with an inorganic acid, a salt with an organic acid, a salt with a basic or acidic amino acid, and the like.

As the salt with an inorganic base, for example, there are alkali metal salts such as a sodium salt and a potassium salt; alkali earth metal salts such as a calcium salt and a magnesium salt; and an aluminum salt, an ammonium salt, and the like.

As the salt with an organic base, for example, there are trimethylamine, triethylamine, pyridine, picoline, ethanolamine, diethanolamine, triethanolamine, dicyclohexylamine, N,N'-dibenzylethylenediamine, and the like.

As preferred examples of the salt with an inorganic acid, there are salts with hydrochloric acid, hydrobromic acid, nitric acid, sulfuric acid, phosphoric acid, and the like.

As preferred examples of the salt with an organic acid, there are salts with formic acid, acetic acid, trifluoroacetic acid, fumaric acid, oxalic acid, tartaric acid, maleic acid, citric acid, succinic acid, malic acid, methanesulfonate, benzenesulfonic acid, p-toluenesulfonic acid, and the like.

As preferred examples of the salt with a basic amino acid, there are salts with arginine, lysine, ornithine, and the like, and, as preferred examples of the salt with an acidic amino acid, there are salts with aspartic acid, glutamic acid, and the like.

The compound having urotensin II receptor antagonistic activity (including the compounds represented by the formulae (Ia), (IIa), (IIa'), (Ib), (Ic) and (IIc)) used in the present invention may be a hydrate or a non hydrate. Further, when the compound having urotensin II receptor antagonistic activity (including the compounds represented by the formulae (Ia), (IIa), (IIa'), (Ib), (Ic) and (IIc)) used in the present invention exists as a configurational isomer, a diastereomer, a conformer and the like, if desired, each can be isolated by per se known separation and purification procedures. Further, when the compound having urotensin II receptor antagonistic activity (including the compounds represented by the formulae (Ia), (IIa), (IIa'), (Ib), (Ic) and (IIc)) used in the present invention is racemic, it can be separated to a (S) isomer and a (R) isomer by a conventional optical resolution, and any one of the respective optically active isomers and racemates are included in the present invention. The compound having urotensin II receptor antagonistic activity (including the compounds represented by the formulae (Ia), (IIa), (IIa'), (Ib), (Ic) and (IIc) and salts thereof) or a salt thereof used in the present invention [hereinafter, sometimes, referred to as UII receptor antagonist] may be used as a prodrug. Examples of such prodrug may include compounds which may be converted into UII receptor antagonist through, for example, enzyme- or gastric acid-mediated reaction *in vivo* under physiological conditions, i.e., compounds which may be enzymatically oxidized, reduced and/or hydrolyzed to be converted into UII receptor antagonist, and compounds which may be hydrolyzed by gastric acid and the like to be converted into UII receptor antagonist. Examples of prodrug of UII receptor antagonist include compounds wherein amino group of UII receptor antagonist has been acylated, alkylated or phosphorylated (e.g., compounds wherein amino group of UII receptor antagonist has been eicosanoylated, alanylated, pentylaminocarbonylated, (5-methyl-2-oxo-1,3-dioxolene-4-yl) methoxycarbonylated, tetrahydrofuranylated, pyrrolidylmethylated, pivaloyloxymethylated, tert-butylated, etc.); compounds wherein hydroxy group of UII receptor antagonist has been acylated, alkylated, phosphorylated or borated (e.g., compounds wherein hydroxy group of UII receptor antagonist has been acetylated, palmitoylated, propanoylated, pivaloylated, succinylated, fumarylated, alanylated or dimethylamino methylcarbonylated); compounds wherein carboxyl group of UII receptor antagonist has been esterified or amidated (e.g., compounds wherein carboxyl group of UII receptor antagonist has been ethylesterified, phenylesterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolene-4-yl)methyl esterified, cyclohexyloxycarbonylethyl esterified, methylamidated, etc.), and the like. These compounds can be prepared from UII receptor antagonist using per se known method.

Further, prodrugs of UII receptor antagonist may be compounds which may be converted into UII receptor antagonist under physiological conditions as described in "Development of pharmaceuticals (Iyakuhinn no Kaihatsu)", vol. 7, Molecular Design, pp. 163-198, Hirokawa Shoten (1990).

UII receptor antagonist may be labeled with an isotope such as ³H, ¹⁴C, ³⁵S, ¹²⁵I, etc.

UII receptor antagonist of the present invention can be orally or parenterally administrated independently, or by formulating together with pharmacologically acceptable carriers in the form of solid preparations such as tablets, capsules, granules, powders and the like; or liquid preparations such as syrups, injectable preparations, and the like.

Dosage forms for parenteral administration include, for example, injectable preparations, instillations, suppositories, and the like.

As the pharmacologically acceptable carrier, various conventional organic or inorganic carrier substances can be used and such materials are formulated as excipients, lubricants, binders, disintegrants in solid preparations; as solvents, solubilizing aids, suspending agents, isotonicity agents, buffers, soothing agents in liquid preparations, etc. Further, if necessary, pharmaceutical additives such as preservatives, antioxidants, colorants and flavoring agents can be used. Preferred examples of the excipient include lactose, sugar, D-mannitol, starch, crystalline cellulose, light anhydrous silicic acid, and the like. Preferable examples of the lubricant include magnesium stearate, calcium stearate, tarc, colloidal silica, and the like. Preferred examples of the binder include crystalline cellulose, sugar, D-mannitol, dextrine, hydroxypropyl cellulose, hydroxypropylmethyl cellulose, polyvinyl pyrrolidone, and the like. Preferred examples of the disintegrant include starch, carboxymethyl cellulose, calcium carboxymethyl cellulose, sodium cross calmelose, sodium carboxymethylstarch, and the like. Preferred examples of the solvent include injectable water, alcohol, propylene glycol, macrogol, sesame oil, corn oil, and the like. Preferred examples of the solubilizing aid include polyethylene glycol, polypropylene glycol, D-mannitol, benzyl benzoate, ethanol, trisaminomethane, cholesterol, triethanolamine, sodium carbonate, sodium citrate, and the like. Preferred examples of the suspending agent include surfactants such as stearyltriethanolamine, sodium laurylsulfate, laurylaminopropionic acid, lecithin, benzalkonium chloride, benzetonium chloride, glycerin monostearate, and the like; hydrophilic synthetic polymers such as polyvinyl alcohol, polyvinyl pyrrolidone, carboxymethylcellulose sodium, methyl cellulose, hydroxymethyl cellulose, hydroxyethyl cellulose, hydroxypropyl cellulose, and the like; and the like. Preferred example of the isotonicity agent include sodium chloride, glycerin, D-mannitol, and the like. Preferred examples of the buffer include buffers such as phosphate, acetate, carbonate, citrate, and the like; and the like. Preferred examples of the indolent agent include benzyl alcohol, and the like. Preferable examples of the preservative include paraoxybenzoic acid esters, chlorobutanol, benzyl alcohol, phenethyl alcohol, dehydroacetic acid, sorbic acid, and the like. Preferred examples of the antioxidant, for example, sulfite, ascorbic acid, and the like.

Hereinafter, the production process of the compound having urotensin II receptor antagonistic activity or a salt thereof is shown.

The compounds used in each production process below may form salts similar to those of the above compound having urotensin II receptor antagonistic activity, so far as it does not interfere with the reaction.

Further, in each reaction described hereinafter, when the starting compound has amino group, carboxyl group and hydroxy group as a substituent, a protective group generally used in peptide chemistry may be introduced into such a group and, if desired, the objective compound can be obtained by removing the protective group after reaction.

Examples of the protective group for amino group include lower C₁₋₆ alkylcarbonyl which may be substituted (for example, acetyl, propionyl, and the like), formyl, phenylcarbonyl, C₁₋₆ alkyloxycarbonyl (for example, methoxycarbonyl, ethoxycarbonyl, tert-butoxycarbonyl, and the like), phenyloxycarbonyl (for example, benzoxycarbonyl, and the like), C₇₋₁₀ aralkyloxycarbonyl (for example, benzyloxycarbonyl, and the like), trityl, phthaloyl, and the like. Examples of these substituents include a halogen atom (for example, fluorine, chlorine, bromine, iodine, and the like), C₁₋₆ alkylcarbonyl (for example, acetyl, propionyl, butyryl, and the like), nitro group, and the like, and the number of the substituent(s) is about 1 to 3.

Examples of the protective group for carboxyl group include C₁₋₆ alkyl which may be substituted (for example, methyl, ethyl, propyl, isopropyl, butyl, tert-butyl, and the like), phenyl, trityl, silyl, and the like. Examples of these substituents include a halogen atom (for example, fluorine, chlorine, bromine, iodine, and the like), C₁₋₆ alkylcarbonyl (for example, acetyl, propionyl, butyryl, and the like), formyl, a nitro group, and the like, and the number of the substituent(s) is about 1 to 3.

Examples of the protective group for hydroxy group include C₁₋₆ alkyl which may be substituted (for example, methyl, ethyl, propyl, isopropyl, butyl, tert-butyl, and the like), phenyl, C₇₋₁₀ aralkyl (for example, benzyl, and the like), C₁₋₆ alkylcarbonyl (for example, acetyl, propionyl, and the like), formyl, phenyloxycarbonyl, C₇₋₁₀ aralkyloxycarbonyl (for example, benzyloxycarbonyl, and the like), pyranyl, furanyl, silyl, and the like. Examples of these substituents include a halogen atom (for example, fluorine, chlorine, bromine, iodine, and the like), C₁₋₆ alkyl, phenyl, C₇₋₁₀ aralkyl, nitro group, and the like, and the number of the substituent(s) is about 1 to 4.

Further, as a method for introducing and removing the protective group, per se known methods or their modifications [for example, the method described in "Protective Groups in Organic Chemistry" (J.F.W.McOmie et al, Plenam Press Co.)] can be used. As a method for removing the protective group, for example, treatment with an acid, a base, reduction, ultraviolet rays, hydrazine, phenylhydrazine, sodium N-methylthiocarbamate, tetrabutylammonium fluoride, palladium acetate, or the like can be used.

A production process of the compound represented by the formula (Ia) [including the compound represented by the formula (IIa) or (IIa') having a novel structure] or a salt thereof is shown below.

The compound represented by the formula (Ia) or a salt thereof can be produced by a per se known method. Further, the compound represented by the formula (Ia) or a salt thereof can be produced, for example, by the following method, or a method described in "Tetrahedron Letters" Vol. 40, pp. 5643-5646, JP 3-220189 A), JP 48-30280 B, and the like, or a modification thereof.

Among the compound represented by the formula (Ia) [including the compound represented by the formula (IIa) or (IIa') having a novel structure] or a salt thereof, a compound represented by the formula (Iaa) or a salt thereof in which R¹ is an unsubstituted amino group, or a salt thereof can be produced, for example, by the following scheme: 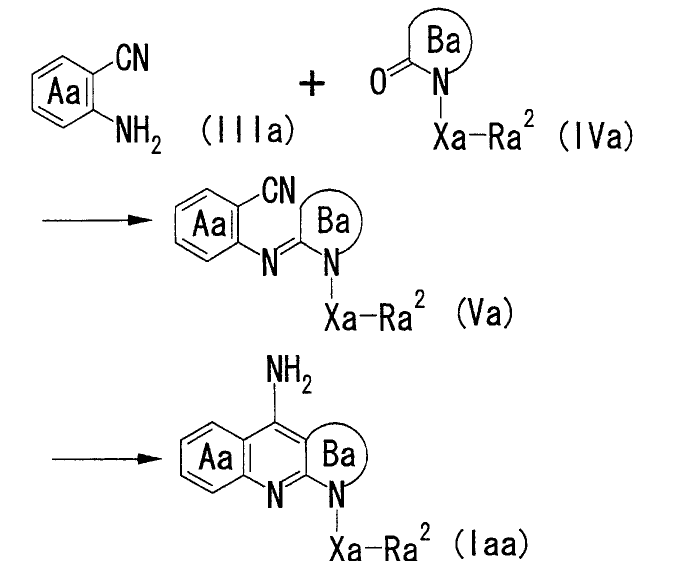 wherein each symbols are as defined above.

In accordance with the method described in JP 3-220189 A, JP 48-30280 B or their modifications, the compound represented by the formula (Iaa) or a salt thereof can be obtained by carrying out the cyclization reaction of a compound represented by the formula (Va) or a salt thereof which is obtained by reacting a compound represented by the formula (IIIa) or a salt thereof with a compound represented by the formula (IVa) or a salt thereof.

The compound represented by the formula (Ia) [including the compound represented by the formula (IIa) or (IIa') having a novel structure] or a salt thereof can be produced, for example, by the following scheme: 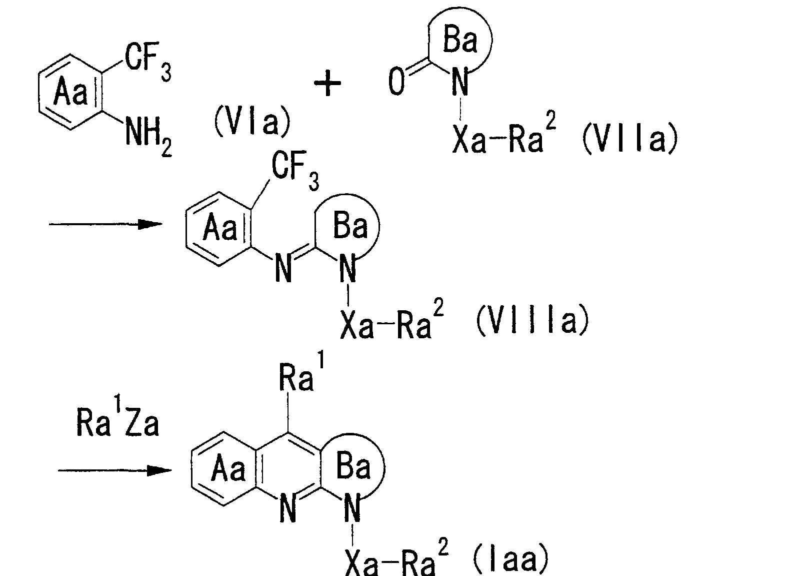 wherein Za is an alkali metal and the other symbols are as defined above.

In accordance with the method described in "Tetrahedron Letters", Vol. 40, pp. 5643-5646 or its modification, and the like, the compound represented by the formula (Ia) or a salt thereof can be obtained by reacting a compound represented by the formula Ra¹Za with a compound represented by the formula (VIIIa) or a salt thereof which is obtained by reacting a compound represented by the formula (VIa) or a salt thereof with a compound represented by the formula (VIIa) or a salt thereof.

Examples of the alkali metal represented by Za include lithium, sodium and the like.

The reaction may be carried out without a solvent or in a solvent. The solvent is not specifically limited so far as it does not influence the reaction, but examples thereof include, ether solvents (for example, diethyl ether, tetrahydrofuran, dioxane, and the like), halogen solvents (for example, dichloromethane, dichloroethane, chloroform, carbon tetrachloride, and the like), hydrocarbon solvents (for example, benzene, toluene, hexane, heptane, and the like), amide solvents (for example, dimethylformamide, dimethylacetamide, N-methylpyrrolidone, and the like), ester solvents (for example, methyl acetate, ethyl acetate, and the like), acetonitrile, dimethyl sulfoxide, and the like. Further, a mixture of two or more thereof may be used.

The compound represented by the formula Ra¹Za is used in an amount of about 0.5 to 20 mole equivalent, and preferably about 0.8 to 10 mole equivalent based on the compound represented by the formula (VIIIa) or a salt thereof. At this time, the reaction temperature is about-80°C to 200°C and preferably about -80°C to 80°C, and the reaction time is about 0.1 to 96 hours and preferably about 0.5 to 72 hours.

Further, among the compound represented by the formula (Ia) or a salt thereof, a compound or a salt thereof in which Ra¹ is not an unsubstituted amino group can be produced by a known method. For example, it can be produced by variously converting in accordance with the following reaction using the compound represented by the formula (Iaa) or a salt thereof synthesized by the above scheme as the starting compound. 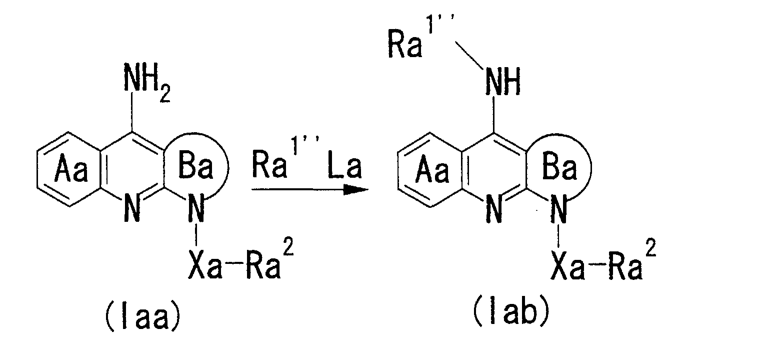 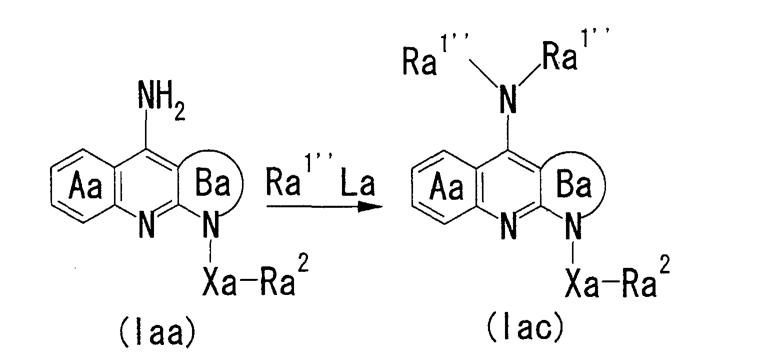 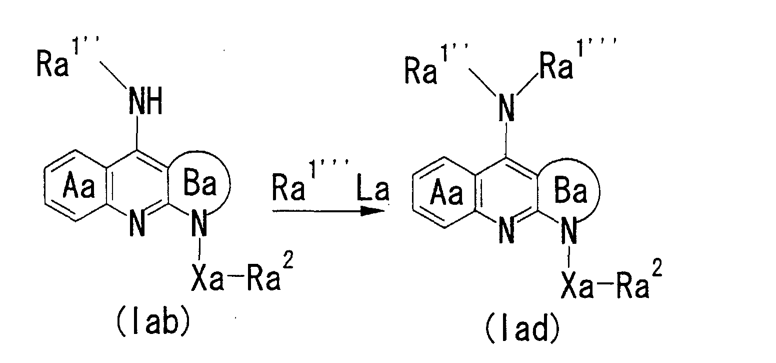 wherein each of Ra₁'' and Ra₁''' is a substituent of amino group (preferably, a lower alkyl group which may be substituted) and La is a leaving group.

Examples of the leaving group represented by La include a halogen atom (for example, chlorine, bromine, iodine, and the like), sulfonic acid esters such as a methanesulfonyl group, a toluenesulfonyl group, etc., and the like.

The reaction may be carried out without a solvent or in a solvent. The solvent is not specifically limited so far as it does not influence the reaction, but examples thereof include ether solvents (for example, diethyl ether, tetrahydrofuran, dioxane, and the like), halogen solvents (for example, dichloromethane, dichloroethane, chloroform, carbon tetrachloride, and the like), hydrocarbon solvents (for example, benzene, toluene, hexane, heptane, and the like), amide solvents (for example, dimethylformamide, dimethylacetamide, N-methylpyrrolidone, and the like), ester solvents (for example, methyl acetate, ethyl acetate, and the like), acetonitrile, dimethyl sulfoxide, and the like. Further, a mixture of 2 or more of these may be used. Further, if necessary, the reaction may be carried out in the presence of a base (for example, triethylamine, 4-(dimethylamino)pyridine, 2-tert-butylimino-2-ethylamino-1,3-dimethylperhydro-1,3,2-diazasulfone, sodium bicarbonate, potassium bicarbonate, sodium carbonate, potassium carbonate, sodium hydride, potassium hydride, and the like), or a phase transfer catalyst (for example, quaternary ammonium salts such as tetrabutylammonium bromide, benzyl triethylammonium chloride, crown ethers such as 18-crown-6, and the like), or a base and a phase transfer catalyst.

The amount of the compound represented by the formula Ra^{1''}La based on the compound represented by the formula (Iaa) or a salt thereof, and the amount of the compound represented by the formula Ra^{1'''}La based on the compound represented by the formula (Iab) or a salt thereof are about 0.5 to 20 mole equivalent, and preferably about 0.8 to 10 mole equivalent. At this time, the reaction temperature is about -20°C to 200°C and preferably about 20°C to 150°C, and the reaction time is about 0.1 to 96 hours and preferably about 0.5 to 72 hours. The amount of the base used is usually about 0.5 to 10 mole equivalent, preferably about 1 to 5 mole equivalent based on the compound represented by the formula (Iaa) or (Iab).

Further, when the substituent in ring A is a halogen atom such as chlorine, bromine, iodine, and the like, it can be easily converted to various functional groups (a substituent of benzene ring represented by ring Aa, and the like) by known substitution reactions (Suzuki coupling reaction, Still reaction, Heck reaction, and the like).

The compound (Ia) thus obtained can be isolated and purified by known separation and purification procedures, for example, such as concentration, vacuum concentration, solvent extraction, crystallization, recrystallization, solvent conversion, chromatography, and the like.

A production process of the compound represented by the formula (Ib) or a salt thereof is shown below.

The compound represented by the formula (Ib) or a salt thereof can be produced, for example, by Scheme 1b. 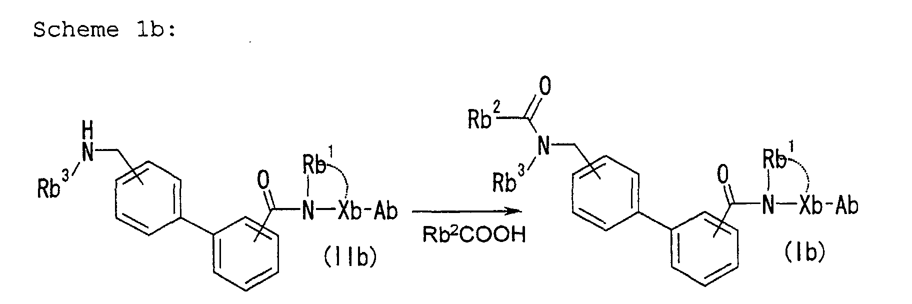 wherein each symbol is as defined above.

The compound represented by the formula (Ib) or a salt thereof can be produced by reacting the compound represented by the formula (IIb), a carboxylic acid represented by Rb²COOH or its reactive derivative or salts thereof in a solvent and, if necessary, in the presence of a base, using a condensing agent. Examples of the reactive derivative of the carboxylic acid include acid anhydride, an active ester (for example, p-nitrophenyl ester, N-hydroxysuccinimide ester, pentafkuorophenyl ester, 1-hydroxybenzotriazole ester, and the like), an acid halide (for example, an acid chloride, an acid bromide, and the like), imidazolide, an anhydride of a mix acid (for example, an anhydride with methyl carbonate, an anhydride with ethyl carbonate, and the like), etc. Specific examples thereof include a compound in which a group represented by the formula -COOH is a group represented by the formula -COQ [wherein Q is a leaving group (for example, a halogen atom (for example, fluorine, chlorine, bromine, iodine, and the like), a methanesulfonyloxy, benzenesulfonyloxy, p-toluenesulfonyloxy, and the like)], etc. Examples of the solvent used, for example, there are mentioned ether solvents (for example, diethyl ether, tetrahydrofuran, dioxane, and the like), hydrocarbon solvents (for example, benzene, toluene, hexane, heptane, and the like), halogen solvents (for example, dichloromethane, dichloroethane, chloroform, carbon tetrachloride, and the like), acetonitrile, N,N-dimethylformamide, and the like.

Examples of the base used include organic bases such as triethylamine, 4-dimethylamino pyridine, N,N-diisopropylethylamine, triethylenediamine, 4-methylmorpholine, and the like; carbonates of an alkali metal or an alkali earth metal (for example, sodium carbonate, potassium carbonate, and the like), bicarbonates of an alkali metal or an alkali earth metal (for example, sodium bicarbonate, potassium bicarbonate, and the like), hydroxides of an alkali metal or an alkali earth metal (for example, sodium hydroxide, potassium hydroxide, and the like), etc. Examples of the condensing agent used include those used in peptide synthesis, and the like. Specific examples thereof include dicyclohexylcarbodiimide, diisopropylcarbodiimide, N-ethyl-N'-3-dimethylaminoropylcarbodiimide and its hydrochloride, benzotriazol-1-yl-tris(dimethylamino)phosphonium hexafluorophosphorilated salt, benzotriazol-1-yl-trispyrrolidinophosphonium hexafluorophosphorilated salt, diethyl cyanophosphorate, diphenylphosphoryl azide, N-hydroxy-5-norbornene-2,3-carboxyimide, and the like. These may be used alone or in combination with 1-hydroxybenzotriazole, 1-hydroxy-7-azabenzotriazole, and the like. At this time, the carboxylic acid represented by the formula Rb2COOH or a salt thereof is used in an amount of 0.5 to 10 mole equivalent, preferably 1 to 5 mole equivalent, per one mole of the compound represented by the formula (IIb). The condensing agent is used in an amount of 0.5 to 10 mole equivalent, preferably 1 to 6 mole equivalent. At this time, the reaction temperature is-50°C to 200°C and preferably -20°C to 100°C, and the reaction time is 0.5 to 96 hours, preferably 0.5 to 72 hours and more preferably 1 to 24 hours.

The compound represented by the formula (Ib) or a salt thereof can be also produced, for example, by Scheme 2b. 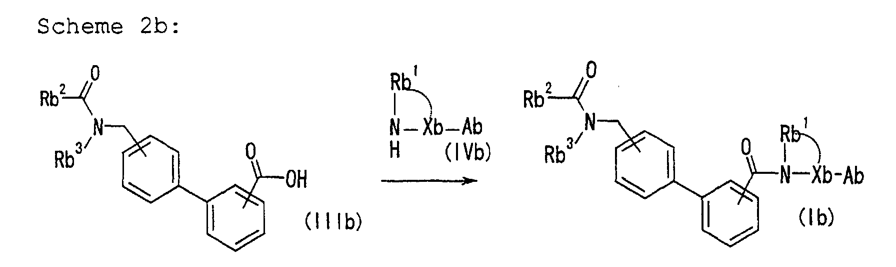 wherein each symbol is as defined above.

The compound represented by the formula (Ib) or a salt thereof can be produced by reacting the compound represented by the formula (IIIb), its reactive derivative or salts thereof, and the compound represented by the formula (IVb) in a solvent and, if necessary, in the presence of a base, using a condensing agent. Examples of the reactive derivative of the compound represented by the formula (IIIb) include acid anhydride, an active ester (for example, p-nitrophenyl ester, N-hydroxysuccinimide ester, pentafkuorophenyl ester, 1-hydroxybenzotriazole ester, and the like), an acid halide (for example, an acid chloride, an acid bromide and the like), imidazolide, an anhydride of a mix acid (for example, an anhydride with methyl carbonate, an anhydride with ethyl carbonate, and the like), etc. Specific examples thereof include a compound in which a group represented by the formula -COOH is a group represented by the formula -COQ [wherein Q is a leaving group (for example, a halogen atom (for example, fluorine, chlorine, bromine, iodine, and the like), a methanesulfonyloxy, benzenesulfonyloxy, p-toluenesulfonyloxy, and the like], etc. Examples of the solvent used include ether solvents (for example, diethyl ether, tetrahydrofuran, dioxane, and the like), hydrocarbon solvents (for example, benzene, toluene, hexane, heptane, and the like), halogen solvents (for example, dichloromethane, dichloroethane, chloroform, carbon tetrachloride, and the like), acetonitrile, N,N-dimethylformamide, and the like.

Examples of the base used include organic bases such as triethylamine, 4-dimethylaminopyridine, N,N-diisopropylethylamine, triethylenediamine, 4-methylmorpholine, and the like; carbonates of an alkali metal or an alkali earth metal (for example, sodium carbonate, potassium carbonate, and the like), bicarbonates of an alkali metal or an alkali earth metal (for example, sodium bicarbonate, potassium bicarbonate, and the like), hydroxides of an alkali metal or an alkali earth metal (for example, sodium hydroxide, potassium hydroxide, and the like), etc. Examples of the condensing agent used include those used peptide synthesis, and the like. Specific examples thereof include dicyclohexylcarbodiimide, diisopropylcarbodiimide, N-ethyl-N'-3-dimethylaminoropylcarbodiimide and its hydrochloride, benzotriazol-1-yl-tris(dimethylamino) phosphonium hexafluorophosphorilated salt, benzotriazol-1-yl-trispyrrolidinophosphonium hexafluorophosphorilated salt, diethyl cyanophosphorate, diphenylphosphoryl azide, and the like. These may be used alone or in combination with 1-hydroxybenzotriazole, 1-hydroxy-7-azabenzotriazole, and the like. At this time, the compound represented by the formula (IVb) or a salt thereof is used in an amount of 0.5 to 10 mole equivalent, preferably 1 to 5 mole equivalent per one mole of the compound represented by the formula (IIIb). The condensing agent is used in an amount of 0.5 to 10 mole equivalent, and preferably 1 to 6 mole equivalent. The reaction temperature at this time is -50°C to 200°C and preferably -20°C to 100°C, and the reaction time is 0.5 to 96 hours, preferably 0.5 to 72 hours and more preferably 1 to 24 hours.

The compound represented by the formula (IIb) or a salt thereof can be also produced, for example, by Scheme 3b. 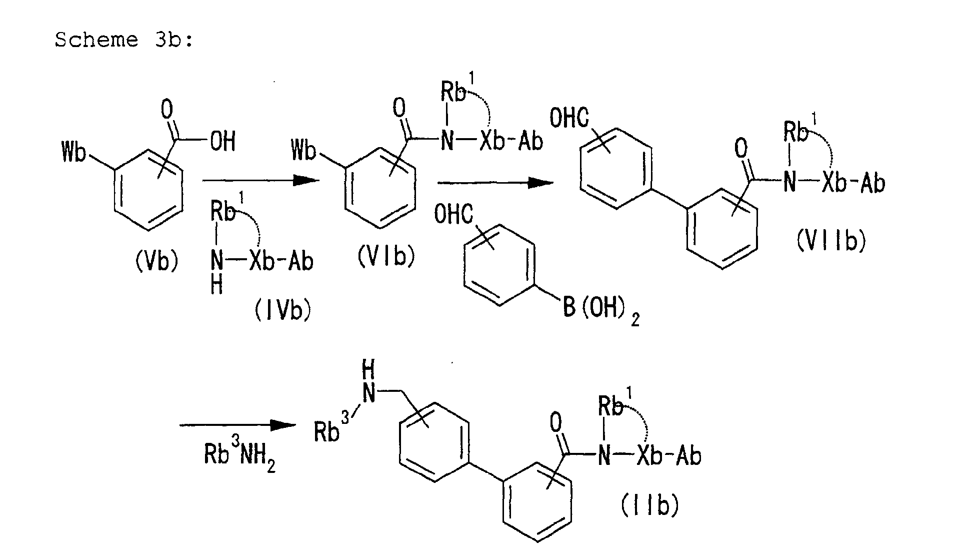 wherein Wb is a halogen atom (for example, fluorine, chlorine, bromine, iodine, and the like) or trifluoromethanesulfonyloxy group, and the other symbols are as defined above.

The compound represented by the formula (VIb) or a salt thereof can be produced by reacting the compound represented by the formula (Vb), its reactive derivative or salts thereof, and the compound represented by the formula (IVb). The reaction is carried out under conditions similar to those of the condensation reaction exemplified in the above Scheme 2b.

The compound represented by the formula (VIIb) or a salt thereof can be produced by reacting the compound represented by the formula (VIb) or a salt thereof, with formylbenzeneboric acid or its ester or anhydride, in a solvent under basic conditions in the presence of a transition metal catalyst. Examples of the solvent used include water, alcohol solvents (for example, methanol, ethanol, n-propanol, isopropanol, and the like), ether solvents (for example, diethyl ether, tetrahydrofuran, 1,2-dimethoxyethane, and the like), hydrocarbon solvents (for example, benzene, toluene, hexane, heptane, and the like), N,N-dimethylformamide, and the like. These solvents may be used alone, or a mixture of 2 or more kinds thereof at an appropriate proportion. Examples of the base used include carbonates of an alkali metal or an alkali earth metal (for example, sodium carbonate, potassium carbonate, and the like), bicarbonates of an alkali metal or an alkali earth metal (for example, sodium bicarbonate, potassium bicarbonate, and the like), hydroxides of an alkali metal or an alkali earth metal (for example, sodium hydroxide, potassium hydroxide, and the like), triethylamine, 4-dimethylaminopyridine, N,N-diisopropylethylamine, triethylenediamine, 4-methylmorpholine, etc. Examples of the transition metal catalyst used include palladium catalysts [for example, tetrakis(triphenylphosphine) palladium, 1,1-bis(diphenylphosphino)ferocenedichloro palladium, dichlorobis(triphenylphosphine) palladium, and the like], etc. At this time, the formylbenzeneboric acid or its ester or anhydride is used in an amount of 0.5 to 10 mole equivalent, preferably 1 to 5 mole equivalent per one mole of the compound represented by the formula (VIb) or a salt thereof. The transition metal catalyst is used in an mount of 0.01 to 1 mole equivalent, preferably 0.05 to 0.2 mole equivalent. At this time, the reaction temperature is 0°C to 200°C and preferably 50°C to 100°C, and the reaction time is 0.5 to 48 hours, and preferably 1 to 24 hours.

The compound represented by the formula (IIb) or a salt thereof can be produced under conditions of a reductive amination reaction using the compound represented by the formula (VIIb) or a salt thereof, and amine represented by the formula Rb³NH₂ or a salt thereof. The reductive amination reaction can be carried out by reacting the compound represented by the formula (VIIb) or a salt thereof, and an amine represented by the formula Rb³NH₂ or a salt thereof in the presence of a metal hydride complex compound (for example, sodium borohydride, sodium cyanoborohydride, sodium triacetoxyborohydride, and the like) in a solvent such as an ether solvent (for example, diethyl ether, tetrahydrofuran, dioxane, and the like), a hydrocarbon solvent (for example, benzene, toluene, hexane, heptane, and the like), a halogen solvent (for example, dichloromethane, dichloroethane, chloroform, carbon tetrachloride, and the like), an alcohol solvent (for example, methanol, ethanol, n-propanol, isopropanol, and the like), acetonitrile, N,N-dimethylformamide, acetic acid, and the like, or a mixed solvent thereof. At this time, the amine represented by the formula Rb³NH₂ or a salt thereof is used in an amount of 0.5 to 10 mole equivalent, preferably 1 to 5 mole equivalent per one mole of the compound represented by the formula (VIIb) or a salt thereof. The metal hydride complex compound is used in an amount of 0.5 to 10 mole equivalent, preferably 1 to 5 mole equivalent. At this time, the reaction temperature is 0°C to 200°C and preferably 20°C to 100°C, and the reaction time is 0.5 to 96 hours, and preferably 1 to 24 hours.

The compound represented by the formula (IIb) or a salt thereof can be also produced, for example, by Scheme 4b. 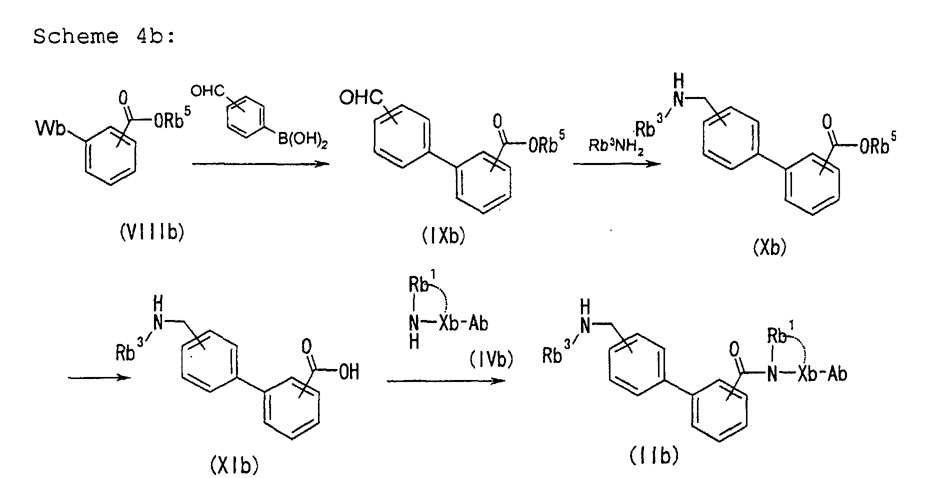 wherein Rb⁵ is C₁₋₆ alkyl which may be substituted (for example, methyl, ethyl, propyl, isopropyl, butyl, tert-butyl, phenyl, trityl, silyl, and the like), and the other symbols are as defined above.

The compound represented by the formula (IXb) or a salt thereof can be produced by reacting the compound represented by the formula (VIIIb) or a salt thereof, with formylbenzeneboric acid or its ester body or anhydride, in a solvent under basic conditions in the presence of a transition metal catalyst. The reaction is carried out under conditions similar to those exemplified with respect to the reaction of the compound represented by the formula (VIb) or a salt thereof to the compound represented by the formula (VIIb) or a salt thereof shown in the above Scheme 3b, etc.

The compound represented by the formula (Xb) or a salt thereof can produce by the reaction of the compound represented by the formula (IXb) or a salt thereof and the amine represented by the formula Rb³NH₂ or a salt thereof under conditions of a reductive amination reaction. The reaction is carried out under conditions similar to those exemplified with respect to the reaction from the compound represented by the formula (VIIb) or a salt thereof to the compound represented by the formula (IIb) or a salt thereof shown in the above scheme 3b, etc.

The compound represented by the formula (XIb) or a salt thereof can be produced by treating the compound represented by the formula (Xb) or a salt thereof with an acid or a base. Namely, the compound represented by the formula (Xb) or a salt thereof can be produced by treatment at 0°C to 150°C, and preferably 20°C to 50°C in a solvent such as water, an ether solvent (for example, diethyl ether, tetrahydrofuran, dioxane, and the like), an alcohol solvent (for example, methanol, ethanol, n-propanol, isopropanol, and the like) or a mixed solvent thereof, with a mineral acid (for example, nitric acid, hydrochloric acid, hydrobromic acid, iodic acid, sulfuric acid, and the like) or a hydroxide of an alkali metal (for example, sodium hydroxide, potassium hydroxide, lithium hydroxide, and the like). The strength of the acid or base is preferably about 0.1 to 10, and the reaction time is 1 to 72 hours.

The compound represented by the formula (IIb) or a salt thereof can be produced by reacting the compound represented by the formula (XIb), its reactive derivative or a salt thereof, with the compound represented by the formula (IVb) or a salt thereof. The reaction is carried out under conditions similar to those of the condensation reaction exemplified in the above Scheme 2b.

The compound represented by the formula (IIIb) or a salt thereof can be also produced, for example, by Scheme 5b. 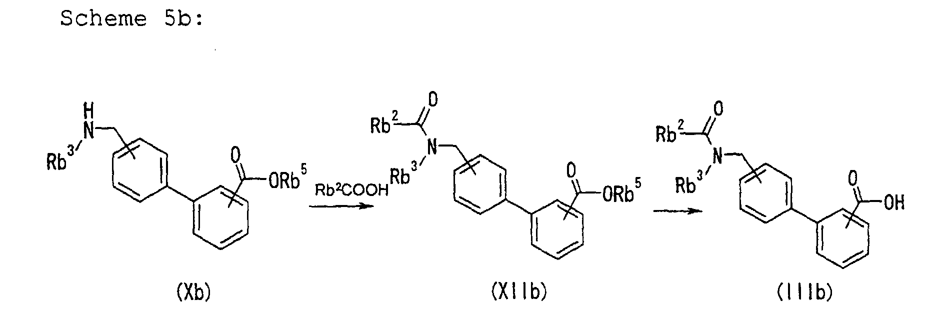 wherein each symbols are as defined above.

The compound represented by the formula (XIIb) or a salt thereof can be produced by reacting the compound represented by the formula (Xb) or a salt thereof whose production process is exemplified in the above Scheme 4b, with the carboxylic acid represented by the formula Rb²COOH, its reactive derivative or a salt thereof, using a condensing agent, in a solvent and, if necessary, in the presence of a base. The reaction is carried out under conditions similar to those of the condensation reaction exemplified in the above Scheme 1b.

The compound represented by the formula (IIIb) or a salt thereof can be produced by treating the compound represented by the formula (XIIb) or a salt thereof with an acid or a base. The reaction is carried out under conditions similar to those exemplified with respect to the reaction from the compound represented by the formula (Xb) or a salt thereof to the compound represented by the formula (XIb) or a salt thereof shown in the above Scheme 4b, etc.

The compound (Ib) thus obtained can be isolated and purified by known separation and purification procedures, for example, such as concentration, vacuum concentration, solvent extraction, crystallization, recrystallization solvent conversion, chromatography, and the like.

A production process of the compound represented by the formula (Ic) [including the compound represented by the formula (IIc) having a novel structure] or a salt thereof is shown below.

The compound represented by the formula (Ic) or a salt thereof can be produced by a known method. Further, the compound represented by the formula (Ic) or a salt thereof can be produced, for example, by the following method, or methods described in EP 487071 A, EP 560235 A, WO98/4659, WO00/23437, and the like, or modifications thereof.

When the compound (Ic) of present invention and the compound (the starting compound or the synthesis intermediate) in each process in the production of the compound (Ic) are free compounds, they can be converted into their salt according to a conventional method, and when they form their salts, they can be also converted into free compounds or other salts.

Further, the compound (Ic) of the present invention and the starting compound or synthesis intermediate may be an optical isomer, a stereo isomer, a positional isomer or a rotational isomer, or a mixture thereof, and these are also included in the compound (Ic) of the present invention and the starting compounds or synthesis intermediates. For example, the compound (Ic) may be a racemate, or an optical isomer which is resolved from the racemate. Further, these can be isolated and purified in accordance with known separation procedures.

The optical isomer can be produced in accordance with known procedures. Specifically, the optical isomer can be produced by using optically active starting compounds or synthesis intermediates, or by optically resolving the racemate of an end compound in accordance with a conventional method. As an optical resolution method, known methods such as a fractional recrystallization method, an optically active column method, a diastereomer method, and the like can be employed. A stereo isomer, a positional isomer or a rotational isomer can be also produced by employing known methods.

The following each reaction can be carried out without using a solvent or using an appropriate solvent, if necessary. Said solvent is not specifically limited so far as it does not influence with the reaction and, in general, any one of those which can be used for a chemical reaction can be used. Examples thereof include organic solvents such as hydrocarbon solvents (for example, hexane, toluene, and the like), ether solvents (for example, ethyl ether, tetrahydrofuran, dioxane, dimethoxyethane, and the like), amide solvents (for example, formamide, N,N-dimethylformamide, N,N-dimethylacetamide, hexamethylphosphorictriamide, and the like), urea solvents (for example, 1,3-dimethyl-2-imidazolidinone, and the like), sulfoxide solvents (for example, dimethylsulfoxide, and the like), alcohol solvents (for example, methanol, ethanol, isopropanol, tert-butanol, and the like), nitrile solvents (for example, acetonitrile, propionitrile, and the like), pyridine, etc., or water, and the like. The amount of said solvent used is usually about 0.5 ml to about 100 ml, preferably about 3 ml to about 30 ml per 1 mmol of the compound. The reaction temperature varies depending on the kind of the solvent used, but is usually about -30°C to about 180°C, preferably about 0°C to about 120°C. The reaction time varies depending on the reaction temperature, but is usually about 0.5 hours to about 72 hours, preferably about 1 hours to about 24 hours. The reaction is usually carried out at normal pressure, but may be carried out under pressured conditions of about 1 atm to about 100 atm, if necessary.

The compound obtained in the following each step is isolated and purified by a known procedure, for example, concentration, liquid conversion, solvent conversion, solvent extraction, fractionation, distillation, crystallization, recrystallization, chromatography, preparative high performance liquid chromatography, etc., and is provided for the next reaction, but the reaction mixture as such may be used as a starting material without isolation and purification.

In the following explanation, a "condensation reaction" can be carried out in the presence of a base, if necessary. Examples of said base include inorganic bases such as sodium carbonate, sodium bicarbonate, potassium carbonate, lithium carbonate, sodium hydroxide, potassium hydroxide, potassium hydride, sodium hydride, sodium methoxide, potassium tert-butoxide, etc.; and organic bases such as pyridine, lutidine, collidine, triethylamine, etc. The amount of said base used is usually an equal mol amount to an excessive amount, preferably about 1 mol equivalent to about 5 mol equivalent based on the compound. Further, in the present reaction, the reaction may be accelerated in the presence of a catalyst amount of an iodine compound such as sodium iodide, potassium iodide, 4-dimethylaminopyridine, etc.

Among compounds (Ic) of the present invention, the known compounds can be produced by the synthetic method described below. Further, they can be also produced by methods described in JP 6-166676 A, JP 11-310532 A, EP-A-487071, EP-A-560235, WO98/46590, WO00/23437, and the like, or their modifications.

On the other hand, the novel compounds in the present invention, for example, the compound represented by the formula (IIc) or a salt thereof, can be produced by the synthetic method described below.

1-1) Among compounds (IIc), the compound (IIca) in which -X- is -O- or a salt thereof can be produced according to the following reaction formula.

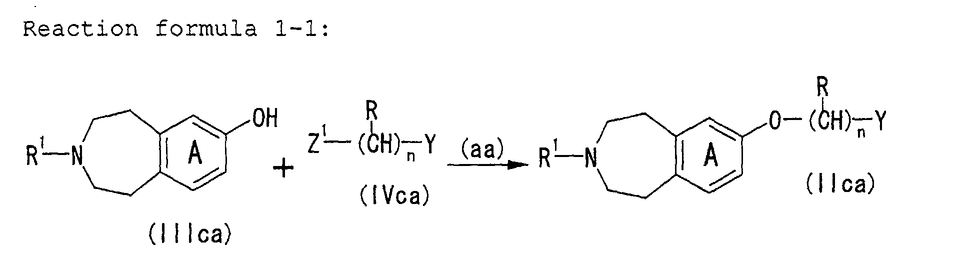

In the step (aa), the compound (IIca) can be produced by a condensation reaction of a compound represented by the formula (IIIca) (wherein each symbol is as defined above) (hereinafter, sometimes, abbreviated as compound (IIIca)), with a compound represented by the formula (IVca) (wherein Z¹ is a leaving group and the other symbols are as defined above) (hereinafter, sometimes, abbreviated as compound (IVca)).

Examples of the leaving group represented by Z¹ include a halogen atom (for example, chloro, bromo, iodo, and the like), a C₁₋₆ alkylsufonyloxy group (for example, methanesulfonyloxy, ethanesulfonyloxy, trifluoromethanesulfonyloxy, and the like), a C₆₋₁₀ arylsufonyloxy group (for example, benzenesulfonyloxy, p-toluenesulfonyloxy, and the like), etc. In particular, for example, a halogen atom (for example, bromo, iodo and the like), and the like are preferably used.

In the condensation reaction of compound (IIIca) with compound (IVca), preferred examples of the solvent to be used include alcohol solvents (for example, ethanol, and the like), or nitrile solvents (for example, acetonitrile, and the like). The reaction temperature varies depending on the kind of the solvent used, but is preferably about 0°C to about 120°C. The reaction time varies depending on the reaction temperature, but is preferably about 1 hours to about 24 hours. As the base, for example, sodium carbonate, potassium carbonate, triethylamine, and the like are preferably used. The amount of said base used is preferably about one equivalent to about 3 equivalent based on compound (IVca). Further, this reaction may be accelerated in the presence of a catalyst amount of an iodine compound (for example, sodium iodide, potassium iodide, and the like, or 4-dimethylaminopyridine, and the like based on compound (IVca), if necessary. Specifically, this reaction can be carried out in a solvent, for example, N,N-dimethylformamide, etc., in the presence of, for example, potassium carbonate, sodium hydride, and the like as a base. The amount of said base used is preferably about one equivalent to about 3 equivalent based on compound (IVca).

Compound (IVca) can be produced by a known method or its modification.

Further, the starting compound (IIIca) or a salt thereof of the step (aa) can be produced, for example, according to the method described in WO00/23437.

1-2) Among compounds (IIc), compound (IIcb) in which -X- is -NR^{3a}- or a salt thereof can be produced according to the following reaction formula.

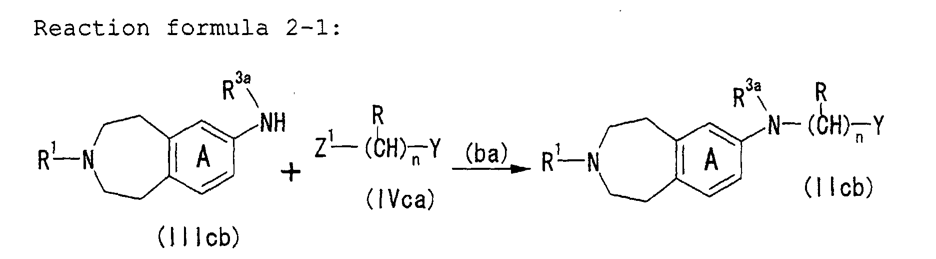

In the step (ba), compound (IIcb) can be produced by the condensation reaction of a compound represented by the formula (IIIcb) [wherein each symbol is as defined above] (hereinafter, sometimes, abbreviated as compound (IIIcb)), with a compound represented by the formula (IVca).

The condensation reaction of compound (IIIcb) with compound (IVca) can be carried out in a solvent, for example, such as N,N-dimethylformamide, etc., in the presence of, for example, potassium carbonate, sodium hydride, and the like as a base. The amount of said base used is preferably about one equivalent to about 3 equivalent based on compound (IVca).

Further, the starting compound (IIIcb) or a salt thereof of the step (ba) can be produced by the following reaction formula 2-2. Namely, compound (IIIcb) can be produced by successively carrying out
the step (bb): a nitration reaction of a compound represented by the formula (Vcb) (hereinafter, sometimes, abbreviated as compound (Vcb)) (wherein each symbol is as defined above),
the step (bc): a reduction reaction of a compound represented by the formula (VIcb) (hereinafter, sometimes, abbreviated as compound (VIcb)) (wherein each symbol is as defined above), and
the step (bd): a condensation reaction of a compound represented by the formula (VIIIcb) (hereinafter, sometimes, abbreviated as compound (VIIIcb)) (wherein each symbol is as defined above), with a compound represented by the formula (IXcb) (hereinafter, sometimes, abbreviated as the compound (IXcb)) (wherein each symbol is as defined above).

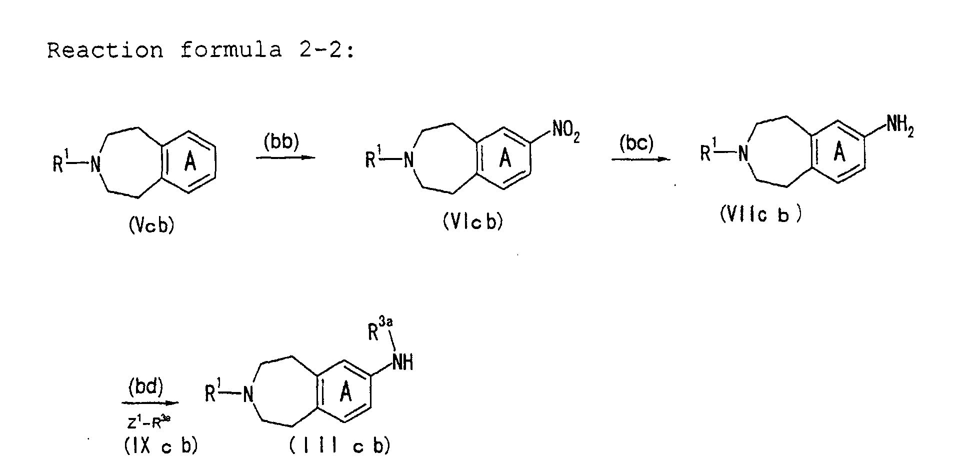

Compound (VIcb)) can be produced by carrying out nitration of compound (Vcb) in the step (bb).

This reaction can be carried out by known methods (for example, methods described in "Synthesis", pp. 217-238 (1977), "Chemistry of the Nitro and Nitroso Groups", pp. 1-48, Wiley (1970), and the like) or their modifications, using an appropriate nitration reagent (for example, nitric acid, nitric acid-sulfuric acid, nitroniumtrifluoroborate, and the like).

Compound (Vcb)) can be produced by known methods, or their modifications. For example, it can be produced in accordance with known methods described in J. Org. Chem., Vol. 34, pp. 2235 (1969); J. Org. Chem., Vol. 54, pp. 5574 (1989); Tetrahedron Lett., Vol. 35, pp. 3023 (1977); Bull. Chem. Soc. Jpn., Vol. 56, pp. 2300 (1983); Indian, J. Chem., Vol. 2, pp. 211 (1964); Indian J. Chem., Vol. 12, pp. 247 (1974); Bull. Chem. Soc. Jpn., Vol. 43, pp. 1824 (1970); Chem. Pharm. Bull., Vol. 20, pp. 1328 (1972); Chem. Pharm. Bull., Vol. 27, pp. 1982 (1979); Helv. Chem. Acta., Vol. 46, pp. 1696, (1963); Synthesis, pp. 541 (1979); U.S. Patent 3,682,962; U.S. Patent 3,991,126; Ger. Offen. 2,314,392; Ger. Patent 1,545,805; J. Chem. Soc., pp. 1381 (1949); Can. J. Chem., Vol. 42, pp. 2904 (1964); J. Org. Chem., Vol. 28, pp. 3058 (1963); J. Am. Chem. Soc., Vol. 76, pp. 3194 (1954), Vol. 87, pp. 1397 (1965), Vol. 88, pp. 4061 (1966); JP 49-41539 A, and the like, or their modifications.

In the step (bc), compound (VIIIcb) can be produced by a reduction reaction of compound (VIcb).

This reaction can be carried out using an appropriate reduction reaction (for example, a catalytic reduction using a transition metal catalyst, a reduction reaction using a metal such as tin, etc., in an acidic solvent). Specifically, it can be carried out in accordance with known methods or their modifications described in Organic Synthesis Coll., Vol. 5, pp. 829-833 (1973); Organic Synthesis Coll., Vol. 1, pp. 455 (1941); J. Am. Chem. Soc., Vol. 66, pp. 1781 (1954), and the like.

In the step (bd), compound (IIIcb) can be produced by a condensation reaction of compound (VIIcb) with compound (IXcb).

The condensation reaction of compound (VIIcb) with compound (IXcb) can be carried out, for example, in the same manner as that of compound (IIIca) with compound (IVca).

Further, compound (IIIcb) can be also produced, for example, by a method according to reductive alkylation (for example, methods described in J. Am. Chem. Soc., Vol.87, pp. 2767 (1965), Organic Synthesis Coll., Vol. 4, pp. 283-285 (1963), and the like), or a method according to Michael addition (for example, methods described in Helv. Chem. Acta., Vol. 43, pp. 1898, (1960), J. Org. Chem., Vol. 39, pp. 2044 (1974), Synthesis, Vol. 5, 375 (1981), and the like), or their modifications, using compound (VIIcc) as a starting material.

1-3) Among compounds (IIc), compound (IIca) in which -X- is -NR^{3a}CO- or a salt thereof can be produced by the following reaction formula 3.

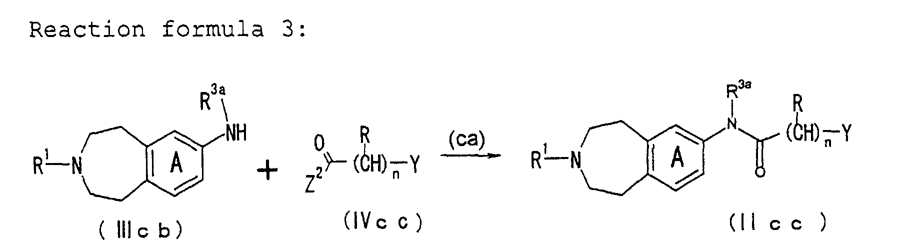

In the step (ca), compound (IIcc) can be produced by an amidation reaction of compound (IIIcb) and a compound represented by the formula (IVcc) (hereinafter, sometimes, abbreviated as compound (IVcc)) (wherein Z² is a leaving group and the other symbols are as defined above).

Examples of the leaving group represented by Z² include a halogen atom (for example, chloro, bromo, iodo, and the like), a C₁₋₆ alkyloxy group (for example, methoxy, ethoxy, benzyloxy, and the like), a C₆₋₁₀ aryloxy group (for example, phenoxy, p-nitrophenoxy, and the like), hydroxy group, etc. In particular, for example, a halogen atom (for example, chloro, and the like), hydroxy group, and the like are preferably used.

The amidation reaction of compound (IIIcb) and compound (IVcc) can also be carried out using an appropriate condensing agent and a base. When Z² is hydroxy group, this amidation reaction can be carried out using an appropriate condensing agent, for example, that used in peptide chemistry field, in particular, carbodiimides such as dicyclohexyl carbodiimide, 1-ethyl-3-(3-dimethylaminopropyl)carbodiimide, etc., phosphonic acids such as diphenylphosphoryl azide, diethyl cyanophosphorate, etc., phosgene equivalents such as 1,1'-carbonylbis-1H-imidazole, etc. The amount of said condensing agent is usually about 1 equivalent to about 5 equivalent, preferably, about 1 equivalent to about 1.5 equivalent per 1 mmol of the compound (IIIcb).

Further, when Z² is a halogen atom, it is preferable to carry out the reaction using an appropriate base, for example, sodium carbonate, potassium carbonate, triethylamine, and the like. The amount of said base is usually about 1 equivalent to about 10 equivalent, preferably, about 1 equivalent to about 2 equivalent based on the compound (IIIcb).

1-4) Among compounds (IIc), compound (IIcd) in which -X- is -S-, -SO-, or -SO₂- or a salt thereof can be produced by the following reaction formula 4-1.

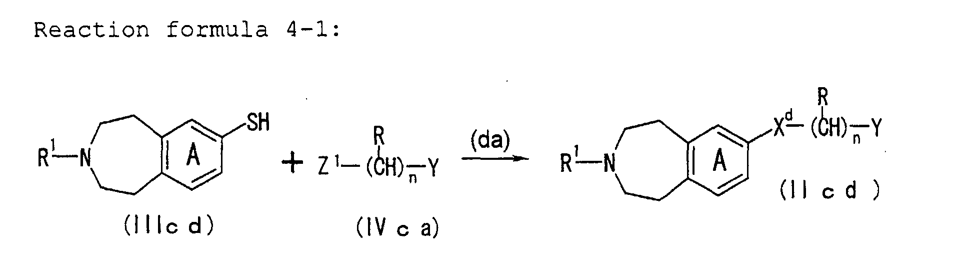

In the step (da), compound (IIcd) can be produced by carrying out a condensation reaction of compound (IIIcd) and compound (IVca), and successively carrying out oxidation reaction, if necessary. [In the formula, X^{d} is -S-, -SO-, or -SO₂-, and the other symbols are as defined above].

The condensation reaction of compound (IIIcb) with compound (IVca) can be carried out in a solvent, for example, such as N,N-dimethylformamide, etc., in the presence of, for example, potassium carbonate, sodium hydride, and the like as a base. The amount of said base used is preferably about one equivalent to about 3 equivalent based on compound (IVca).

Compound (IIcd) in which X^{d} is -S- can be introduced to compound (IIcd) in which X^{d} is -SO- or -SO₂- by carrying out oxidation reaction, if necessary.

As the oxidizing agent, any oxidizing agent of sulfide can be used, but for example, meta-chloroperbenzoic acid, peracetic acid, hydrogen peroxide, alkali metal periodate, and the like are preferably used. In particular, meta-chloroperbenzoic acid, hydrogen peroxide, and the like are preferably used. The amount of said oxidizing agent used is preferably about one equivalent to about 1.1 equivalent based on compound (IIcd), in particular, when S is oxidized to SO. Further, it is preferred to use about 2 to 2.5 equivalent based on the compound (IVcd), in particular, when S is oxidized to SO₂. As the solvent of this reaction, for example, dichloromethane, chloroform, acetic acid, ethyl acetate, and the like are preferred.

Starting compound (IIIcd) or a salt thereof of the step (da) can be produced by the reaction formula 4-2 below. Namely, compound (IIIcd) can be produced by
the step (db): a chlorosufonylation reaction of compound (Vcb), and
the step (dc): a reduction reaction of a compound represented by the formula (VIcd) (hereinafter, sometimes, abbreviated as compound (VIcd)) (wherein each symbol is as defined above).

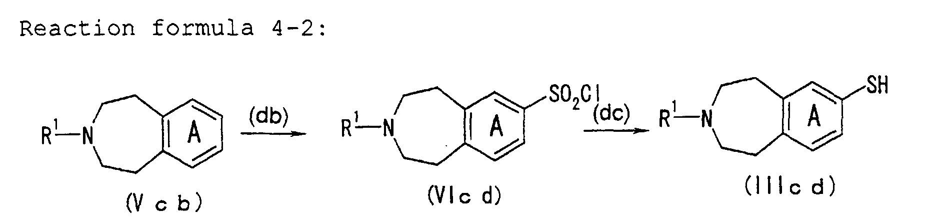

In the step (db), compound (VIcd) can be produced by carrying out a chlorosufonylation reaction of compound (Vcb).

Examples of the reagent of this chlorosufonylation reaction include chlorosulfonic acid, sulfuryl chloride, sulfur dioxide-cupric chloride, and the like. In particular, chlorosulfonic acid, and the like are preferred. The amount of said chlorosufonylation reagent is about one equivalent to an excessive amount. This reaction can be carried out without a solvent or by using a solvent. As the solvent used for a case of using a solvent, preferably, there are, for example, dichloromethane, 1,2-dichloroethane, carbon disulfide, and the like. The reaction without a solvent is preferred in particular. The reaction temperature is preferably about -20°C to about 100°C.

Further, chlorosufonyl group is introduced to any position which can be reacted, but, for example, when ring A is unsubstituted, the chlorosufonylation of the 7-position is mainly carried out. However, a compound in which the chlorosufonylation of the 6-position is carried out can be prepared and isolated.

In the step (dc), compound (IIIcd) can be produced by reducing compound (VIcd).

This reduction reaction can be carried out under appropriate reduction conditions, for example, combinations of a metal and an acid such as zinc-acetic acid and tin-hydrochloric acid, etc., a catalytic hydrogenation using a transition metal catalyst, or metal hydrides such as lithium aluminumhydride, and the like. In particular, the reduction reaction using zinc-acetic acid is preferred.

1-5) Among compounds (IIc), compound (IIce) in which -X- is -SO₂NR^{3a}- or a salt thereof can be produced by the following reaction formula 5.

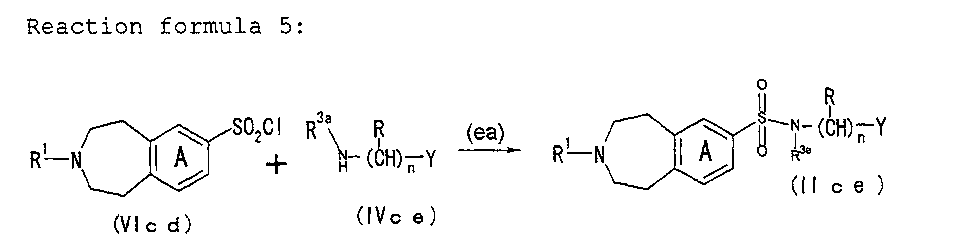

In the step (ea), compound (IIce) can be produced by carrying out a condensation reaction of compound (VIcd) and a compound represented by the formula (IVce) (hereinafter, sometimes abbreviated as the compound (IVce)) (wherein each symbol is as defined above).

The condensation reaction of the compound (VIcd) with the compound (IVce) can be carried in the same manner as, for example, the amidation reaction of the compound (IIIcb) with the compound (IVcc).

The compound (IVce) or a salt thereof can be produced by a known method or its corresponding method. For example, it can be produced by methods described in J. Med. Chem., Vol. 33, pp. 1880 (1990), and the like or their corresponding methods.

1-6) Among compounds (IIc), compound (IIcf) in which -X- is -SO₂NHCONR^{3a}- or a salt thereof can be produced by the following reaction formula 6.

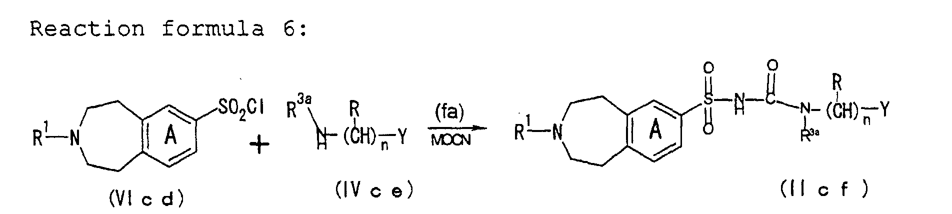

In the step (fa), compound (IIcf) can be produced by acting an alkali metal isocyanate (MOCN, wherein M is an alkali metal) with compound (VIcd), and then reacting compound (IVce). This reaction can be carried out, for example, according to methods described in EP 759431 A, JP 7-118267 A, and the like or their modifications.

The reaction of compound (VIcd) with an alkali metal isocyanate is carried out in the presence of a base, if necessary. As the base used, pyridine, triethylamine, and the like are preferred in particular. The amount of said base is preferably about 1 equivalent to about 5 equivalent based on compound (VIcd). As the reaction solvent, acetonitrile, and the like are preferred in particular. As the alkali metal, for example, potassium, and the like are preferably used.

1-7) Among compounds (IIc), compound (IIcg) in which -X- is -SO₂NHC(=NH)NR^{3a}- or a salt thereof can be produced by the following reaction formula 7.

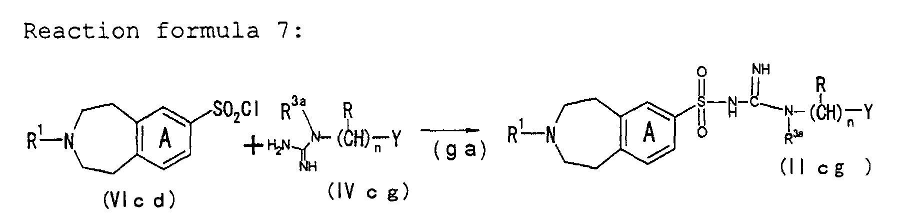

In the step (ga), compound (IIcg) can be produced by a condensation reaction of compound (VIcd) and a compound represented by the formula (IVcg) (hereinafter, sometimes, abbreviated as compound (IVcg)) (wherein each symbol is as defined above).

The condensation reaction of compound (VIcd) with compound (IVcg) can be carried in the same manner as, for example, the amidation reaction of compound (IIIcb) with compound (IVcc).

Compound (IVcg) can be produced by a known method or its modification, using compound (IVce). For example, compound (IVcg) can be produced by a method of reacting S-methylisothiourea with compound (IVce) (for example, a method described in J. Org. Chem., Vol. 13, pp. 924 (1948), and the like), a method of reacting cyanamide with compound (IVce) (for example, Helv. Chem. Acta., Vol. 29, pp. 324, (1946), and the like), and a method of reacting 1,3-bis(tert-butoxycarbonyl)-2-methyl-2-thiopseudourea with compound (IVce) (for example, Tetrahedron Lett., Vol. 33, pp. 6541-6542 (1992), J. Org. Chem., Vol. 52, pp. 1700-1703 (1987), and the like), etc.

1-8) Among compounds (IIc), compound (IIch) in which -X- is -CR^{3a}(R^{3b})- or a salt thereof can be produced by the following reaction formula 8.

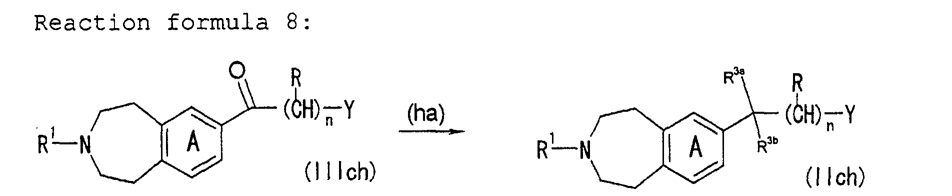

In the step (ha), compound (IIch) can be produced by reacting a compound represented by the formula (IIIch) (hereinafter, sometimes, abbreviated as compound (IIIch)) (wherein each symbol is as defined above), with an appropriate reagent, to convert the carbonyl group.

As the reagent used for the conversion reaction of the carbonyl group, there are reducing agents such as, for example, sodium borohydride, lithium aluminumhydride, triethylsilane, etc., organometallic reagents such as, for example, alkyllithium, alkylmagnesiumhydride, additionally nucleophilic reacting agent such as, for example, hydrogen cyanide, etc.

Specifically, the conversion of the carbonyl group to -CH(OH)- and -CH₂- can be carried out under suitable conditions (for example, combinations of triethylsilane-trifluoroacetic acid, lithium aluminumhydride-aluminum chloride, zinc-hydrochloric acid, and the like), using reducing agents such as sodium borohydride, lithium aluminumhydride, triethylsilane, and the like.

This reaction can be carried out by methods described in, for example, "Reduction with Complex Metal Hydrides", Interscience, New York (1956), Chem. Soc. Rev., Vol. 5, pp. 23 (1976); Synthesis, pp. 633 (1974); J. Am. Chem. Soc., Vol. 91, pp. 2967 (1969); J. Org. Chem., Vol. 29, pp. 121 (1964); Org. Reactions, Vol. 1, pp. 155 (1942); Angew. Chem., Vol. 71, pp. 726 (1956); Synthesis, pp. 633 (1974); J. Am. Chem. Soc., Vol. 80, pp. 2896 (1958); Org. Reactions; Vol. 4, pp. 378 (1948); J. Am. Chem. Soc., Vol. 108, pp. 3385 (1986); and the like, or their modifications.

Further, the conversion of the carbonyl group to -CR^{3c}(OH)- (wherein R^{3c} is a C₁₋₆ alkyl group) can be carried out by methods described in, for example, "Grignard Reactions of Nonmetallic Substances", Prentice-Hall: Englewood Cliffs, NJ, 1954, pp. 138-528; "Organolithium Methods", Academic Press: New York, 1988, pp. 67-75, and the like, or their modifications, using organometallic reagents such as, alkyllithium, alkylmagnesiumhydride, and the like.

Furthermore, the conversion of the carbonyl group can be carried out by methods described in Advanced Organic Chemistry, 5^{th} ed., Wiley-Interscience: New York 1992, pp. 879-981, and the like, or their modifications.

Compound (IIIch) can be produced by known methods or their modifications, for example, methods described in JP 5-140149 A, JP 6-206875 A, J. Med. Chem., Vol. 37, pp. 2292 (1994), and the like, or their modifications.

1-9) Among compounds (IIc), compound (IIci) in which -X- is -C(=CR^{3a}(R^{3b}))- or a salt thereof can be produced by the following reaction formula 9.

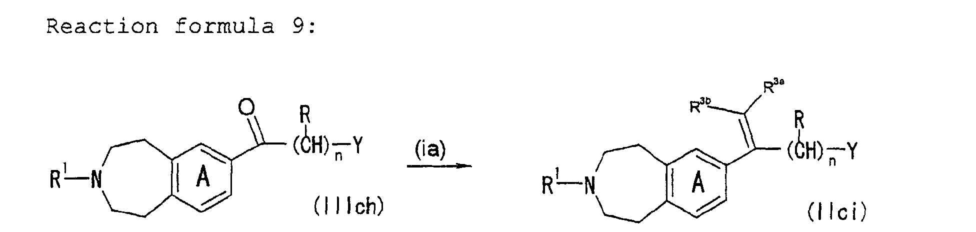

In the step (ia), compound (IIci) can be produced by reacting compound (IIIch) with an appropriate reagent to convert the carbonyl group.

As the conversion reaction of the carbonyl group, there are mentioned Wittig reaction, Horner-Wadsworth-Emmons reaction, Peterson olefination reaction, Knoevenagel reaction, and the like, and as the reagent, conventional reagents to be used for those reactions are used.

This reaction can be carried out by methods described in, for example, Advanced Organic Chemistry, 5^{th} ed., Wiley-Interscience: New York 1992, pp. 879-981; Organic Synthesis Coll., Vol. 5, pp. 751 (1973); Organic Synthesis Coll., Vol. 5, pp. 509 (1973); Synthesis 384 (1984); Org. Reactions, Vol. 15, pp. 204 (1967); and the like, or their modifications.

1-10) Among compounds (IIc), compound (IIcj) in which -X- is -C(=NR^{3a})- or a salt thereof can be produced by the following reaction formula 10.

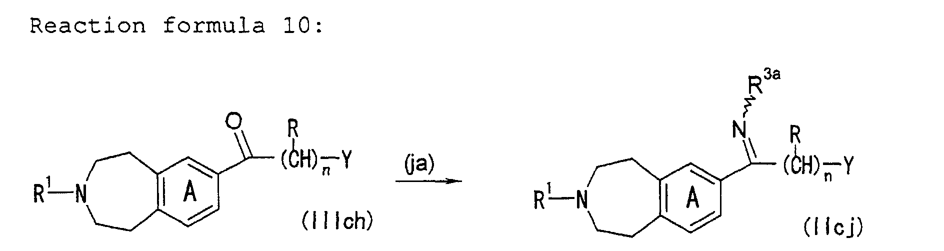

In the step (ja), compound (IIcj) can be produced by reacting compound (IIIch) with an appropriate reagent, to convert the carbonyl group.

As the reagent used for conversion of the carbonyl group, for example, there are mentioned hydrazine which may be substituted, hydroxylamine which may be substituted, and the like. As said substituent, a C₁₋₆ alkyl group, and the like are used.

This reaction can be carried out by methods described in, for example, Advanced Organic Chemistry, 5^{th} ed., Wiley-Interscience: New York 1992, pp. 904-907; Organic Functional Group Preparations, Vol. III, Academic (1983); Rodd's Chemistry of Carbon Compounds, Vol. 1, part C, Elsevier Publishing Co. (1965); and the like, or their modifications.

1-11) Among compounds (IIc), compound (IIck) in which -X- is -CS- or a salt thereof can be produced by the following reaction formula 11.

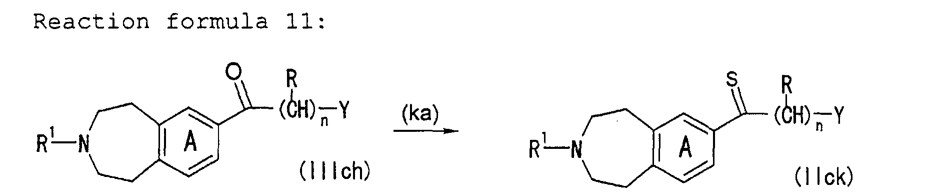

In the step (ka), compound (IIck) can be produced by reacting compound (IIIch) with an appropriate reagent to convert the carbonyl group to the thiocarbonyl group.

As the reagent used for conversion of the carbonyl group to the thiocarbonyl group, for example, there are mentioned conventional sulfidation reagents such as Lawesson reagent, phosphorous pentasulfide, hydrogen sulfide-hydrochloric acid, etc.

This reaction can be carried out by methods described in Synthesis, Vol. 7, pp. 543 (1991); J. Am. Chem. Soc., Vol. 106, pp. 934; J. Am. Chem. Soc., Vol. 68, pp. 769 (1946); and the like, or their modifications.

1-12) Among compounds (IIc), compound (IIcm) in which -X- is -CONR^{3a}- or a salt thereof can be produced by the following reaction formula 12-1.

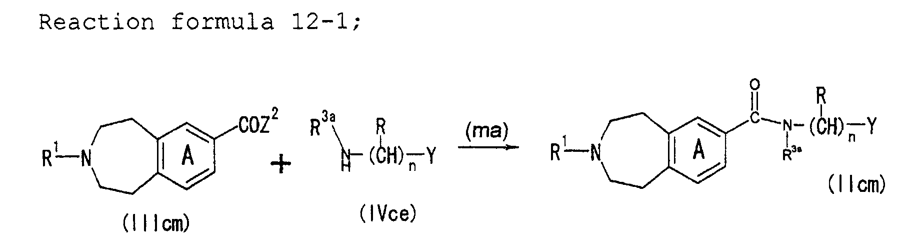

In the step (ma), compound (IIcm) can be produced by carrying out a condensation reaction of a compound represented by the formula (IIIcm) (hereinafter, sometimes, abbreviated as compound (IIIcm)) (wherein each symbol is as defined above), with compound (VIce).

The condensation reaction of compound (IIIcm) with compound (IVce) can be carried in the same manner as, for example, the amidation reaction of compound (IIIcb) with compound (IVcc).

Further, the starting compound (IIIcm) of the step (ma) can be produced by the reaction formula 12-2 below. Namely, compound (IIIcm) can be produced by successively carrying out the step (mb): an acetylation reaction of compound (Vcb), and the step (mc): an oxidation reaction and functional conversion, if necessary, of a compound represented by the formula (VIcm) (hereinafter, sometimes, abbreviated as compound (VIcm)) (wherein each symbol is as defined above).

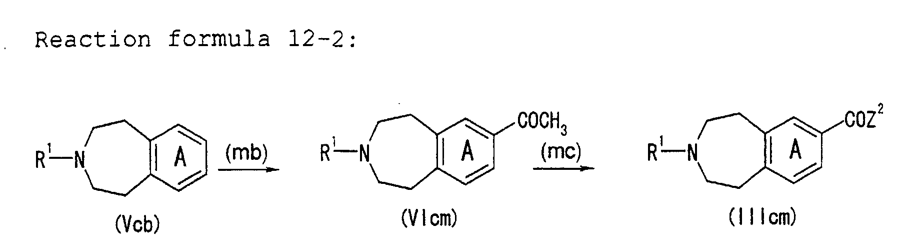

In the step (mb), compound (VIcm) can be produced by carrying out the acetylation of compound (Vcb).

This reaction can be carried out under conditions of a normal Friedel Crafts reaction. As the reagent of an acetylation, acetyl chloride, acetic anhydride and the like are used. Specifically, it can be produced by methods described in, for example, JP 5-140149 A, JP 6-206875 A, J. Med. Chem., Vol. 37, pp. 2292 (1994), and the like, or their modifications.

In the step (mc), compound (IIIcm), in particular, the compound in which Z² is hydroxyl group can be produced by oxidizing compound (VIcm).

As the oxidizing agent used in this reaction, for example, there are mentioned a hypochlorite, a hypobromite, a halogen simple body (for example, bromine, iodine, and the like) in the presence of an appropriate base (for example, sodium hydroxide, and the like), etc. Specifically, this reaction can be carried out by methods described in Organic Synthesis Coll., Vol. 2, pp. 428 (1943), J. Am. Chem. Soc., Vol. 66, pp. 894 (1944), and the like, or their modifications.

Further, if desired, it can be converted to compound (IIIcm) in which Z² is a halogen atom (for example, chloro, bromo, iodo, and the like) or a C₁₋₆ alkyloxy group (for example, methoxy, ethoxy, benzyloxy, and the like), or a C₆₋₁₀ aryl oxy group (for example, phenoxy, p-nitrophenoxy, and the like), by carrying out a functional conversion of compound (IIIcm) in which Z² is hydroxy group.

The functional conversion can be carried out according to methods described in, for example, Advanced Organic Chemistry, 5^{th} ed., Wiley-Interscience: New York 1992, pp. 393-396, 437-438, Comprehensive Organic Transformations, VCH Publishers Inc. (1989), and the like, or their modifications.

Compounds (IIc) thus obtained can be isolated and purified by known separation and purification procedures, for example, such as concentration, vacuum concentration, solvent extraction, crystallization, recrystallization, solvent conversion, chromatography, and the like.

Since the UII receptor antagonist used in the present invention has a potent effect of inhibiting amyloid β40 secretion, it can be used for a prophylactic or therapeutic agent for various central nervous system disorders resulted from or related to amyloid β protein, particularly, increase in amyloid β40 secretion. Particularly, it is useful for a prophylactic or therapeutic agent for (1) cerebrovascular amyloid angiopathy which is observed to be complicated with Alzheimer's disease brain and the like, (2) neurodegenerative diseases (for example, senile dementia, Alzheimer's disease, Down's disease, Parkinson's disease, Creutzfeldt-Jakob disease, amyotrophic spinal cord lateral sclerosis, diabetic neuropathy, and the like), (3) neuropathy at cerebrovascular disorder (for example, cerebral infarction, cerebral hemorrhage, cerebral circulation disorder accompanied with encepharoartery sclerosis, and the like), head injury and myelo injury, sequelae of encephalitis or cerebral paralysis, (4) dysmnesia (for example, senile dementia, amnesia, and the like), or (5) mental disease (for example, depression, anxiety disorder, panic disorder, shizophrenia, and the like), and the like.

The UII receptor antagonist used in the present invention can be used in combination with, for example, an anti-Alzheimer's disease drug (for example, acetylcholinesterase inhibitor such as Donepezil, Tacrine and the like, vaccine therapy and the like), an antiparkinson drug (for example, Carbidopa + Levodopa, Pergolide, Ropinirole, Cabergolin, Pramipexole, Entacapron, Lazabemide and the like), therapeutic agent for amyotrophic spinal cord lateral sclerosis (for example, Riluzole, Mecasermin, Gabapentin and the like), an antidepressant (for example, Fluoxetine, Sartraline, Peroxetine, Venlafaxine, Nefazodone, Reboxetine, Imipramine hydrochloride, Duloxetine and the like), therapeutic agent for shizophrenia (for example, Olanzapine, Risperidone, Quetiapine, Iloperidone and the like), and the like in the prevention and treatment of said diseases.

The UII receptor antagonist used in the present invention has weak toxicity, and is superior in intracerebral transition.

Accordingly, the UII receptor antagonist is useful for a prophylactic or therapeutic agent for central nervous system diseases in mammals (for example, rat, mouse, guinea pig, rabbit, sheep, horse, pig, cow, monkey, human, and the like)

The UII receptor antagonist used in the present invention can be formulated in accordance with known procedures, and can be orally or parenterally administered safely (for example, local, rectal, intravenous administration and the like) as UII receptor antagonist as it is, or as a pharmaceutical composition, for example, tablets (including sugar-coating tablet, film-coating tablet, etc.), powders, granules, capsules (including soft capsule, etc.), liquid preparations, injectable preparations, suppositories, sustained-releases, agent and the like, by appropriately mixing with an appropriate amount the antagonist with a pharmacologically acceptable carrier in a production step of the composition.

The amount of the UII receptor antagonist in the pharmaceutical composition of the present invention is usually about 0.1 to 100% by weight based on the total weight of the preparation. The dose varies depending on a particular subject administered, administration route, disease, and the like, but for example, is about 0.01 to 500mg, preferably about 0.1 to 100mg, and more preferably about 1 to 100mg per once as an active ingredient (the compound having urotensin II receptor antagonistic activity) to an adult (about 60 kg), as the oral preparation for a therapeutic agent for Alzheimer's disease, and can be administrated once to several times per day.

The pharmacologically acceptable carrier to be used for the production of the composition of the present invention may be various conventional organic or inorganic carrier substances for pharmaceutical preparations and examples thereof include excipients, lubricants, binders, disintegrants, etc., for solid preparations; solvents, solubilizing aids, suspending agents, isotonicity agents, buffers, soothing agents, etc., for liquid preparations. Further, if necessary, additives such as preservatives, antioxidants, colorants, sweetening agents, absorbing agents, wetting agents, etc., can be used.

Examples of the excipient include lactose, sugar, D-mannitol, starch, corn starch, crystalline cellulose, soft anhydrous silicic acid, and the like.

Examples of the lubricant include magnesium stearate, calcium stearate, tarc, colloid silica, and the like.

Examples of the binder include crystalline cellulose, sugar, D-mannitol, dextrine, hydroxypropyl cellulose, hydroxypropylmethyl cellulose, polyvinyl pyrrolidone, starch, sucrose, gelatin, methylcellulose, sodium carboxymethyl cellulose, and the like.

Examples of the disintegrant include starch, carboxymethyl cellulose, calcium carboxymethyl cellulose, sodium cross calmelose, sodium carboxymethylstarch, L-hydroxypropyl cellulose, and the like.

Examples of the solvent include water for injection, alcohol, propylene glycol, macrogol, sesame oil, corn oil, and the like.

Examples of the solubilizing aid include polyethylene glycol, polypropylene glycol, D-mannitol, benzyl benzoate, ethanol, trisaminomethane, cholesterol, triethanolamine, sodium carbonate, sodium citrate, and the like.

Examples of the suspending agent include surfactants such as stearyltriethanolamine, sodium laurylsulfate, laurylaminopropionic acid, lecithin, benzalkonium chloride, benzetonium chloride, glycerin monostearate, etc.; hydrophilic polymers, for example, such as polyvinyl alcohol, polyvinyl pyrrolidone, sodium carboxymethyl cellulose , methyl cellulose, hydroxymethyl cellulose, hydroxyethyl cellulose, hydroxypropyl cellulose, etc.; and the like.

Examples of the isotonicity agent include glucose, D-sorbitol, sodium chloride, glycerin, D-mannitol, and the like.

Examples of the buffer include phosphate, acetate, carbonate, citrate; and the like.

Examples of the soothing agent include benzyl alcohol, and the like.

Examples of the preservative include paraoxybenzoate esters, chlorobutanol, benzyl alcohol, phenethyl alcohol, dehydroacetic acid, sorbic acid, and the like.

Examples of the antioxidant include bisulfate, ascorbic acid, and the like.

### Examples

The following Experimental Examples, Synthesis Examples and Preparation Examples further illustrate the present invention in detail but they are mere examples and are not to be construed to limit the present invention.

### Experimental Example 1

Test compound 1 was added to IMR32 cells at various concentrations (µM) 10 minutes before addition of 10 nM of urotensin II. Then, urotensin II was added in the presence or absence of test compound 1, the mixture was cultured for 24 hours, and the amount of amyloid β protein in the supernatant of the culture was determined by enzyme immunoassay (Biochemistry, Vol.34, No.32, pp. 10272-10278, (1995)).

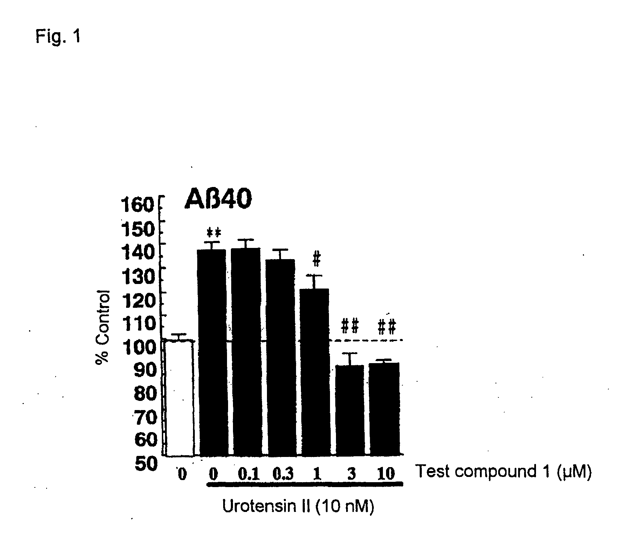

The results are shown in Fig. 1.

As seen from Fig. 1, it was found that test compound 1 concentration-dependently suppressed increase in the selective secretion of amyloid β40 by urotensin II to the level without addition of urotensin II. (**: significant increase at ≤1% with respect to the group without addition of urotensin II, and #, ##: significant suppression at ≤1% and ≤5%, respectively, with respect to the group with addition of urotensin II alone).

Test compound 1 was purchased from ASINEX Co.

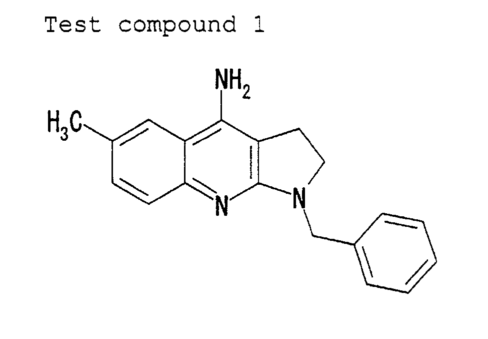

### Experimental Example 2

Test compound 2 as described hereinafter (the compound of Synthesis Example 5) was used and added at a concentration of 1 nM, and the amount of amyloid β protein was determined in the same manner as that of Experimental Example 1.

Namely, IMR32 cells were used and test compound 2 was added at a concentration of 1 nM 10 minutes before addition of 10 nM of urotensin II. After addition of urotensin II, the mixture was cultured for 24 hours and the amount of amyloid β protein in the supernatant of the culture was determined by enzyme immunoassay.

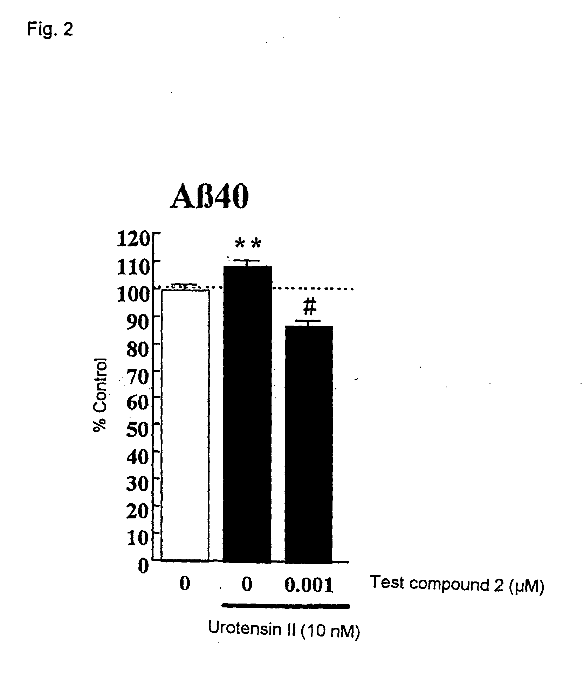

The results are shown in Fig. 2.

As seen from Fig. 2, it was found that test compound 2 completely suppressed increase of amyloid β40 by the urotensin II at a concentration of 1 nM. (**: significant increase at ≤1%, and #, ##: significant inhibition at ≤1%, respectively, with respect to the group with addition of urotensin II alone).

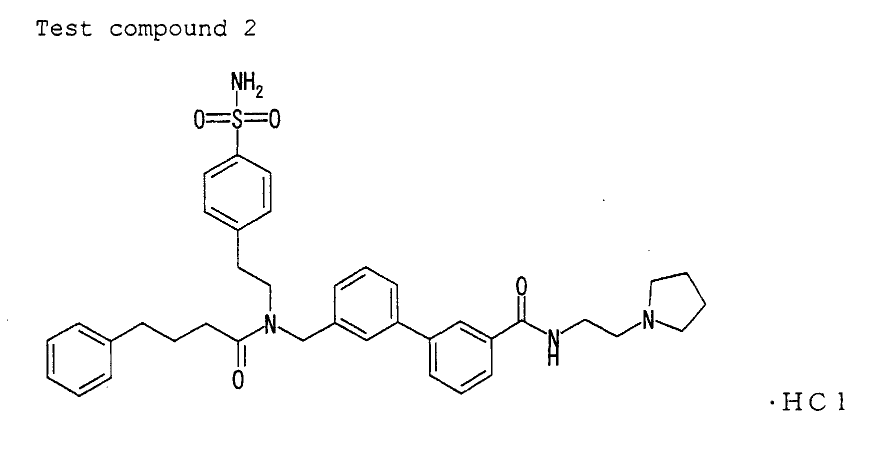

### Synthesis Example 1

### 1-Benzyl-6-bromo-2,3-dihydro-1H-pyrrolo[2,3-b]quinolin-4-ylamine

N-Benzylpyrrolidone (1.8 g, 10.4 mmol) was dissolved in 4ml of chloroform, phosphorous oxychloride (1.8 g, 11.7 mmol) was added thereto, and the mixture was stirred at room temperature for 30 minutes. 4-Bromo-2-cyanoaniline (2.0 g, 10 mmol) was added thereto and the mixture was refluxed by heating. The reaction mixture was poured on ice water, and neutralized with 20% sodium hydroxide aqueous solution. After extraction by chloroform, the organic layer was dried over anhydrous sodium sulfate. After concentration under reduced pressure, the residue was dissolved in 100 ml of nitrobenzene, 2 g of zinc chloride was added and the mixture was heated at 160°C for 3 hours. 20% sodium hydroxide aqueous solution was added to the reaction solution, and it was extracted with ethyl acetate. After the organic layer was dried over anhydrous sodium sulfate, it was concentrated under reduced pressure, and the residue was subjected to silica gel column chromatography (silica gel 50 g, ethyl acetate/hexane=1/2). The objective fraction was concentrated under reduced pressure, ethanol was added to the residue, and a precipitate was collected by filtration. The precipitate was washed with ethanol, and then dried under reduced pressure to obtain the title compound (1.2 g, 3.4 mmol).
¹H-NMR (DMSO-d₆) δ: 2.86 (2H, t, J=8.0Hz), 3.41 (2H, t, J=8.0Hz), 4.59 (2H, s), 7.24-7.33 (6H, m), 7.42 (1H, dd, J=9.2, 2.2Hz), 8.12 (1H, d, J=2.2Hz).
Mass (ESI+); 354 (M+H), 356

### Synthesis Example 2

### 3'-{({2-[4-(Aminosulfonyl)phenyl]ethyl}[(E)-3-phenyl-2-propenoyl]amino)methyl}-N-[2-(1-pyrrolidinyl)ethyl][1,1'-biphenyl]-3-carboxamide

1) 3-Bromo-N-[2-(1-pyrrolidinyl)ethyl]phenyl-carboxamide
   1-(2-Aminoethyl)pyrrolidine (4.34 g), diethyl cyanophosphate (5.57 ml) and triethylamine (10.4 ml) were added to a solution of 3-bromobenzoic acid (5.00 g) in N,N-dimethylformamide solution (DMF; 60 ml), and the mixture was stirred at room temperature for 16 hours. The reaction mixture was diluted with water, and extracted with diethyl ether. The extract was dried over anhydrous sodium sulfate, and the solvent was distilled off under reduced pressure. Hexane was added to the residue to effect crystallization, and the title compound (6.31 g) was obtained.
   ¹H-NMR (CDCl₃) δ: 1.70-1.90 (4H, m), 2.50-2.60 (4H, m), 2.70 (2H, t, J=6.0 Hz), 3.45-3.60 (2H, m), 6.86 (1H, s), 7.30 (1H, t, J=8.0 Hz), 7.60 (1H, dm, J=8.0 Hz), 7.70 (1H, dm, 8.0 Hz), 7.93 (1H, t, J=1.6 Hz).
2) 3'-Formyl-N-[2-(1-pyrrolidinyl)ethyl][1,1'-biphenyl]-3-carboxamide
   Palladium tetrakistriphenylphosphine (735 mg) and 2M sodium carbonate aqueous solution (21.2 ml) were added to a solution of 3-bromo-N-[2-(1-pyrrolidinyl) ethyl]phenylcarboxamide (6.31 g) in toluene (50 ml), a solution of 3-formylboric acid (3.49 g) in ethanol (15 ml) was further added, and the mixture was stirred at 90°C for 15 hours. After the reaction mixture was diluted with water, it was extracted with diethyl ether. The extract was washed with saturated saline, and then dried over anhydrous magnesium sulfate. The solvent was distilled off under reduced pressure to obtain the title compound (6.83 g).
   ¹H-NMR (CDCl₃) δ: 1.95-2.35 (4H, m), 2.95 (2H, m), 3.30-3.50 (2H, m), 3.80-3.40 (4H, m), 7.40-7.60 (2H, m), 7.76 (1H, dm, J=8.0Hz), 7.85 (1H, dm, J=8.0Hz), 8.00 (1H, dm, 8.0Hz), 8.09 (1H, dm, J=8.0Hz), 8.25 (1H, bs), 8.40 (1H, bs), 8.41 (1H, m), 10.10 (1H, s).
3) 3'-[{2[4-(Aminosufonyl)phenyl]ethyl}aminomethyl]-N-[2-(1-pyrrolidinyl)ethyl][1,1'-biphenyl]-3-carboxamide
   4-(2-Aminoethyl)benzenesulfoneamide (2.37 g) and molecular sieves 3A (4.0 g) were added to a solution of 3'-formyl-N-[2-(1-pyrrolidinyl)ethyl][1,1'-biphenyl]-3-carboxamide (3.81 g) in methanol (50ml), and the mixture was stirred at room temperature for 1.5 hours. After the reaction mixture was diluted with tetrahydrofuran (THF), molecular sieve was filtered, and the filtrate was concentrated under reduced pressure. The residue was dissolved in methanol-THF (1:1) mixed solvent (100 ml), and sodium borohydride (0.89 g) was added. After the reaction mixture was stirred at room temperature for 5 hours, the solvent was distilled off under reduced pressure. After the residue was diluted with water, it was extracted with ethyl acetate. The extract was washed with saturated saline, then dried over anhydrous magnesium sulfate, and the solvent was distilled off under reduced pressure. Hexane was added to the residue to effect crystallization, and the objective compound (3.71 g) was obtained.
   ¹H-NMR (CDCl₃) δ: 1.75-1.85 (4H, m), 2.55-2.65 (4H, m), 2.78 (2H, t, J=6.0Hz), 2.85-3.00 (4H, m), 3.60-3.65 (2H, m), 3.87 (2H, s), 7.05-7.15 (1H, m), 7.20-7.60 (6H, m), 7.65-7.85 3H, m), 7.84 (2H, d, J=8.4Hz), 8.05 (1H, s).
4) 3'-{({2-[4-(Aminosulfonyl)phenyl]ethyl}[(E)-3-phenyl-2-propenoyl]amino)methyl}-N-[2-(1-pyrrolidinyl)ethyl][1,1'-biphenyl]-3-carboxamide
   3'-[{2[4-(Aminosufonyl)phenyl]ethyl}aminomethyl]-N-[2-(1-pyrrolidinyl)ethyl][1,1'-biphenyl]-3-carboxamide (506 mg), trans-cinnamic acid (163 mg), 1-ethyl-3-(3-dimethylaminopropyl)carbodiimide hydrochlorate (EDCI·HCl; 211 mg) and 1-hydroxybenzotriazole (NOBT; 149 mg) were dissolved in a mixed solvent of dichloromethane (15 ml) and DMF (7 ml), and the mixture was stirred at room temperature for 18 hours. After the solvent was distilled off under reduced pressure, water was added to the residue, and it was extracted with ethyl acetate. The extract was rinsed with saturated saline, then dried over anhydrous magnesium sulfate, and the solvent was distilled off under reduced pressure. The residue was purified with silica gel column chromatography (dichloromethane/methanol=98/2) to obtain the objective compound (284 mg).
   ¹H-NMR (CDCl₃) δ: 1.73 (4H, m), 2.52 (4H, m), 2.69 (2H, t, J=6.0Hz), 2.85-3.00 (2H, m), 3.50-3.60 (2H, m), 3.66 (2H, t, J=7.0Hz), 4.60 (2H,s), 6.57 (1H, d, J=15.6Hz), 6.85 (1H, d, J=15.6Hz), 7.10-7.90 (16H, m), 8.05 (1H, s).
   MS (APCI+): 637 (M+H)

### Synthesis Example 3

### 3'-{({2-[4-(Aminosulfonyl)phenyl]ethyl}[(E)-3-phenyl-2-propenoyl]amino)methyl}-N-[2-(1-pyrrolidinyl)ethyl][1,1'-biphenyl]-3-carboxamide hydrochloride

3'-{({2-[4-(Aminosulfonyl)phenyl]ethyl}[(E)-3-phenyl-2-propenoyl]amino)methyl}-N-[2-(1-pyrrolidinyl)ethyl][1,1'-biphenyl]-3-carboxamide (200 mg) was treated with a solution of 4 N-hydrogen chloride in ethyl acetate to obtain the objective compound (198 mg).
¹H-NMR (DMSO-d₆) δ: 1.80-2.10 (4H, m), 2.90-3.10 (4H, m), 3.30-3.50 (2H, m), 3.55-3.90 (6H, m), 4.73 (2H,s), 7.05-8.00 (18H, m), 8.25 (1H, s), 9.03 (1H, m).
Elemental analysis (molecular formula:
C₃₇H₄₀N₄O₄S·HCl·1.5H₂O):
Calcd, C: 63.46; H: 6.33; N: 8.00; Cl: 5.08
Found, C: 63.65; H: 6.51; N: 7.86; Cl: 5.25

### Synthesis Example 4

### 3'-{({2-[4-(Aminosulfonyl)phenyl]ethyl}[4-phenylbutanoyl]amino)methyl}-N-[2-(1-pyrrolidinyl)ethyl][1,1'-biphenyl]-3-carboxamide

The objective compound (277 mg) was obtained in the same manner as Synthesis Example 2.
¹H-NMR (DMSO-d₆) δ: 1.75-1.85 (8H, m), 2.20-2.40 (2H, m), 2.45-2.60 (2H, m), 2.60-2.95 (4H, m), 3.20-3.60 (6H, m), 4.62 (2H,s), 7.05-7.95 (18H, m), 8.13 (1H, s), 8.71 (1H, m). MS (ESI+): 653 (M+H)

### Synthesis Example 5

### 3'-{({2-[4-(Aminosulfonyl)phenyl]ethyl}[4-phenylbutanoyl]amino)methyl}-N-[2-(1-pyrrolidinyl)ethyl][1,1'-biphenyl]-3-carboxamide hydrochloride

The compound of Synthesis Example 4 was obtained in the same manner as Synthesis Example 2, and the objective compound (185 mg) was obtained in the same manner as Synthesis Example 3.
¹H-NMR (DMSO-d₆) δ: 1.75-2.10 (8H, m), 2.25-2.45 (2H, m), 2.45-2.60 (2H, m), 2.80-2.90 (2H, m), 2.95-3.10 (2H, m), 3.20-3.50 (2H, m), 3.50-3.75 (4H, m), 4.61 (2H,s), 7.05-8.00 (18H, m), 8.23 (1H, s), 9.02 (1H, m).
Elemental analysis (molecular formula C₃₈H₄₄N₄O₄S·HCl·H₂O):
Calculated value, C: 64.53; H: 6.70; N: 7.92; Cl: 5.01
Experimental value, C: 64.39; H: 6.82; N: 7.86; Cl: 5.20

### Synthesis Example 6

### N-[2-(4-Benzylpiperazin-1-yl)ethyl]-2,3,4,5-tetrahydro-1H-3-benzazepine-7-carboxamide trihydrochloride

1) 1,2,4,5-Tetrahydro-3H-3-benzazepine-3-carboaldehyde
   Acetic anhydride (18 ml) was added to formic acid (54 ml), and the mixture was stirred at room temperature for one hour. A solution of 2,3,4,5-tetrahydro-1H-3-benzazepine (9.5 g) in ethyl acetate (5 ml) was added dropwise to the mixture under ice cooling. After stirring at room temperature for 30 minutes, the solvent was concentrated under reduced pressure. Ethyl acetate and an aqueous saturated sodium bicarbonate solution were added to the residue, and then the mixture was extracted with ethyl acetate. The extract was washed with saturated saline, then dried over anhydrous magnesium sulfate, and the solvent was distilled off under reduced pressure to obtain the title compound (9.37 g).
   ¹H-NMR (CDCl₃) δ: 2.85-3.00 (4H, m), 3.45-3.50 (2H, m), 3.64-3.70 (2H, m), 7.10-7.20 (4H, m), 8.15 (1H, s)
2) 7-Acetyl-1,2,4,5-tetrahydro-3H-3-benzazepine-3-carboaldehyde
   Aluminum chloride (12.0 g) was added to a solution of 1,2,4,5-tetrahydro-3H-3-benzazepine-3-carboaldehyde (4.50 g) and acetyl chloride (2.01 ml) in dichloroethane (25 ml). After the reaction mixture was stirred at room temperature for 15 hours, it was poured in ice water, and was extracted with ethyl acetate. The extract was rinsed with saturated saline, then dried over anhydrous magnesium sulfate, and the solvent was concentrated under reduced pressure. The residue was purified with silica gel column chromatography (ethyl acetate) to obtain the title compound (3.26 g).
   ¹H-NMR (CDCl₃) δ: 2.60 (3H, s), 2.90-3.05 (4H, m), 3.45-3.55 (2H, m), 3.65-3.75 (2H, m), 7.20-7.30 (1H, m), 7.50-7.80 (2H, m), 8.16 (1H, s)
3) 3-Formyl-2,3,4,5-tetrahydro-1H-3-benzazepine-7-carboxylic acid
   An aqueous solution (70ml) of sodium hydroxide (4.78g) was added to a solution of 7-acetyl-1,2,4,5-tetrahydro-3H-3-benzazepine-3-carboaldehyde (3.24 g) in dioxane (50 ml), and then bromine (2.31 ml) was added dropwise under ice cooling. After the reaction mixture was stirred under ice cooling for 30 minutes, acetone was added to stop the reaction. After the solvent was concentrated under reduced pressure, the water layer was extracted with ethyl acetate, and 5 N hydrochloric acid was added to the extract. The precipitated crystals were collected by filtration, and successively washed with water and ether to obtain the title compound (2.11 g).
   ¹H-NMR (DMSO-d₆) δ: 2.85-3.00 (4H, m), 3.45-3.60 (4H, m), 7.32 (1H, dd, J=2.2, 7.6Hz), 7.72-7.80 (2H, m), 8.12 (1H, s)
4) 2,3,4,5-Tetrahydro-1H-3-benzazepine-7-carboxylic acid
   A solution of 3-formyl-2,3,4,5-tetrahydro-1H-3-benzazepine-7-carboxylic acid (1.0 g) in concentrated hydrochloric acid (50 ml) was stirred at 100°C for 12 hours. After the solvent was concentrated under reduced pressure, the obtained solid was collected by filtration, and successively washed with water and ether to obtain the title compound (990 mg).
   ¹H-NMR (CDCl₃) δ: 3.18 (4H, m), 3.46 (4H, m), 7.33 (1H, d, J=7.8Hz), 7.76 (1H, d, J=7.8Hz), 7.78 (1H, s)
5) 3-(Tert-butoxycarbonyl)-2,3,4,5-tetrahydro-1H-3-benzazepine-7-carboxylic acid
   2,3,4,5-Tetrahydro-1H-3-benzazepine-7-carboxylic acid (300 mg) was dissolved in 1 N sodium hydroxide aqueous solution (2.64 ml), water (2.5 ml) and tetrahydrofuran (2.5 ml), then di-tert-butyl dicarbonate (0.33 ml) was added, and the mixture was stirred at room temperature for 2 hours. After tetrahydrofuran was concentrated under reduced pressure, the water layer was made acidic by 5% potassium hydrogensulfate aqueous solution, and it was extracted with ethyl acetate. The extract was washed with saturated saline, then dried over anhydrous magnesium sulfate, and the solvent was concentrated under reduced pressure to obtain the title compound (344 mg).
   ¹H-NMR (CDCl₃) δ: 1.49 (9H, s), 2.95-3.00 (4H, m), 3.55-3.60 (4H, m), 7.23 (1H, d, J=8.4Hz), 7.86 (1H, s), 7.89 (1H, d, J=8.4Hz)
6) tert-Butyl 7-({[2-(4-benzylpiperazin-1-yl)ethyl]amino}carbonyl)-1,2,4,5-tetrahydro-3H-3-benzazepine-3-carboxylate
   Diethyl cyanophosphate (0.086 ml) was added to a solution of 3-(tert-butoxycarbonyl)-2,3,4,5-tetrahydro-1H-3-benzazepine-7-carboxylic acid (150 mg), 2-(4-benzylpiperazin-1-yl)ethylamine (124 mg) and triethylamine (0.079 ml) in DMF (5ml). After the reaction mixture was stirred at room temperature for 15 hours, it was diluted with water. After it was extracted with ethyl acetate, the extract was washed with saturated saline, then dried over anhydrous magnesium sulfate, and the solvent was concentrated under reduced pressure. The residue was purified with silica gel column chromatography (n-hexane/ethyl acetate=1/2) to obtain the title compound (199 mg).
   ¹H-NMR (CDCl₃) δ: 1.49 (9H, s), 2.50-2.65 (8H, m), 2.59 (2H, t, J=6.0Hz), 2.90-3.00 (4H, m), 3.53 (2H, s), 3.45-3.60 (6H, m), 6.81 (1H, m), 7.15-7.35 (6H, m), 7.45-7.60 (2H, m)
   MS (ESI+): 493 (M+H)
7) N-[2-(4-Benzylpiperazin-1-yl)ethyl]-2,3,4,5-tetrahydro-1H-3-benzazepine-7-carboxamide trihydrochloride
   tert-Butyl 7-({[2-(4-benzylpiperazin-1-yl)ethyl]amino}carbonyl)-1,2,4,5-tetrahydro-3H-3-benzazepine-3-carboxylate (199 mg) was treated with a solution of 1 N hydrogen chloride in ethyl acetate to obtain the objective compound (126 mg).
   ¹H-NMR (DMSO-d₆) δ: 3.00-4.00 (20H, m), 4.35 (2H, m), 7.30 (1H, d, J=7.8Hz), 7.40-7.50 (3H, m), 7.60-7.70 (2H, m), 7.70-7.80 (2H, m), 8.84 (1H, m)
   MS (ESI+): 393 (M+H)

The compounds of Synthesis Examples 7 to 9 were prepared in the same manner as Synthesis Example 6.

### Synthesis Example 7

### N-[2-(4-Benzhydrylpiperazin-1-yl)ethyl]-2,3,4,5-tetrahydro-1H-3-benzazepine-7-carboxamide trihydrochloride

Yield: 238 mg
¹H-NMR (DMSO-d₆) δ: 3.00-4.00 (21H, m), 7.25-7.40 (8H, m), 7.60-7.90 (5H, m), 8.89 (1H, m)
MS (APCI +): 469 (M+H)

### Synthesis Example 8

### N-[2-[4-(4-Chlorobenzyl)piperazin-1-yl)ethyl]-2,3,4,5-tetrahydro-1H-3-benzazepine-7-carboxamide trihydrochloride

Yield: 198 mg
¹H-NMR (DMSO-d₆) δ: 3.00-4.00 (20H, m), 4.31 (2H, m), 7.30 (1H, d, J=7.8Hz), 7.45-7.80 (6H, m), 8.85 (1H, m)
MS (APCII+): 427 (M+H)

### Synthesis Example 9

### N-(2-{4-[Bis(4-fluorophenyl)methyl]piperazin-1-yl}ethyl)-2,3,4,5-tetrahydro-1H-3-benzazepine-7-carboxamide trihydrochloride

Yield: 148 mg
¹H-NMR (DMSO-d₆) δ: 3.00-3.45 (16H, m), 3.50-3.80 (5H, m), 7.15-7.40 (5H, m), 7.50-8.00 (6H, m), 8.90 (1H, m) MS (APCI+): 505 (M+H)

### Preparation Example

| 1. Capsule | | |
|---|---|---|
| (1) | Compound obtained in Synthesis Example 6 | 40 mg |
| (2) | Lactose | 70 mg |
| (3) | Microcrystalline cellulose | 9 mg |
| (4) | Magnesium stearate | 1 mg |
| | 1 capsule | 120 mg |

(1), (2) and (3) as well as 1/2 of (4) were kneaded, and granulated. To this granule is added the remaining (4), and the whole is capsulated into a gelatin capsule.

| 2.Tablet | | |
|---|---|---|
| (1) | Compound obtained in Synthesis Example 6 | 40 mg |
| (2) | Lactose | 58 mg |
| (3) | Corn starch | 18 mg |
| (4) | Microcrystalline cellulose | 3.5 mg |
| (5) | Magnesium stearate | 0.5 mg |
| | 1 tablet | 120 mg |

(1), (2), (3), 2/3 of (4) and 1/2 of (5) are kneaded, and granulated. Then, the remaining (4) and (5) are added to the granule and the mixture is formulated into tablets by compression molding.

### Industrial applicability

Since the compound having urotensin II receptor antagonistic activity or a salt thereof has a superior amyloid β40 secretion inhibitory activity, it is useful for a prophylactic or therapeutic agent for (1) cerebrovascular amyloid angiopathy which is observed to be complicated with Alzheimer's disease brain, and the like, (2) neurodegenerative diseases (for example, senile dementia, Alzheimer's disease, Down's disease, Parkinson's disease, Creutzfeldt-Jakob disease, amyotrophic spinal cord lateral sclerosis, diabetic neuropathy, and the like), (3) neuropathy at cerebrovascular disorder (for example, cerebral infarction, cerebral hemorrhage, cerebral circulation disorder accompanied with encepharoartery sclerosis, and the like), head injury and myelo injury, sequelae of encephalitis or head paralysis, (4) dysmnesia (for example, senile dementia, amnesia, and the like), or (5) mental disease (for example, depression, anxiety disorder, panic disorder, shizophrenia, and the like), and the like.

## Claims

1. A prophylactic or therapeutic agent for central nervous system diseases which comprises a compound having urotensin II receptor antagonistic activity or a salt thereof.

2. The agent according to claim 1, which is an amyloid β40 secretion inhibitor.

3. The agent according to claim 1, which is a prophylactic or therapeutic agent for (1) neurodegenerative diseases, (2) neuropathy at cerebrovascular disorder, cephal injury or myelo injury, sequelae of encephalitis or cerebral paralysis, (3) dysmnesia, or (4) mental diseases.

4. The agent according to claim 1, wherein the compound having urotensin II receptor antagonistic activity or a salt thereof is a non-peptide compound or a salt thereof.

5. The agent according to claim 1, wherein the compound having urotensin II receptor antagonistic activity is a quinoline derivative.

6. The agent according to claim 1, wherein the compound having urotensin II receptor antagonistic activity is a 4-aminoquinoline derivative.

7. The agent according to claim 1, wherein the compound having urotensin II receptor antagonistic activity or a salt thereof is a compound represented by the formula (Ia): 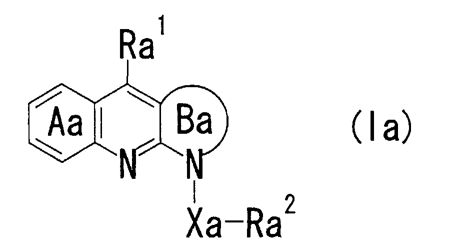 wherein Aa is a benzene ring which may be substituted, Ba is a 5- to 8-membered ring which may be substituted, Xa is a divalent group in which the number of atom(s) in a linear chain portion is 1 to 4, Ra¹ is an amino group which may be substituted, and Ra² is a cyclic group which may be substituted, or a salt thereof.

8. The agent according to claim 7, wherein Aa is substituted with a hydrocarbon group which may be substituted.

9. The agent according to claim 7, wherein Aa is substituted with a C₁₋₄ alkyl group which may be substituted.

10. The agent according to claim 1, wherein the compound having urotensin II receptor antagonistic activity or a salt thereof is a compound represented by the formula (IIa): 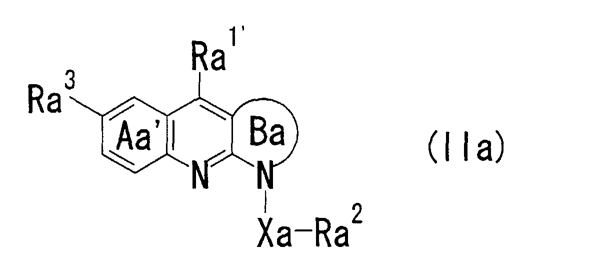 wherein Aa' is a benzene ring which may be further substituted in addition to the substituent Ra³, Ba is a 5-to 8-membered ring which may be substituted, Xa is a divalent group in which the number of atom(s) in a linear chain portion is 1 to 4, Ra^{1'} is a substituted amino group, Ra² is a cyclic group which may be substituted, Ra³ is a hydrocarbon group which may be substituted, a heterocyclic group which may be substituted, nitro group, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted), or a salt thereof.

11. The agent according to claim 1, wherein the compound having urotensin II receptor antagonistic activity or a salt thereof is a compound represented by the formula (IIa'): 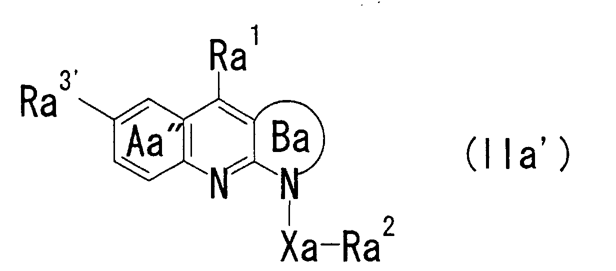 wherein Aa'' is a benzene ring which may be further substituted in addition to the substituent Ra^{3'}, Ba is a 5-to 8-membered ring which may be substituted, Xa is a divalent group in which the number of atom(s) in a linear chain portion is 1 to 4, Ra¹ is an amino group which may be substituted, Ra² is a cyclic group which may be substituted, Ra^{3'} is a hydrocarbon group which may be substituted, a heterocyclic group which may be substituted, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted), or a salt thereof.

12. The agent according to claim 11, wherein R^{3'} is a hydrocarbon group which may be substituted.

13. The agent according to claim 12, wherein R^{3'} is alkyl.

14. The agent according to claim 12, wherein Ra¹ is amino.

15. The agent according to claim 1, wherein the compound having urotensin II receptor antagonistic activity or a salt thereof is a compound represented by the formula (Ib): 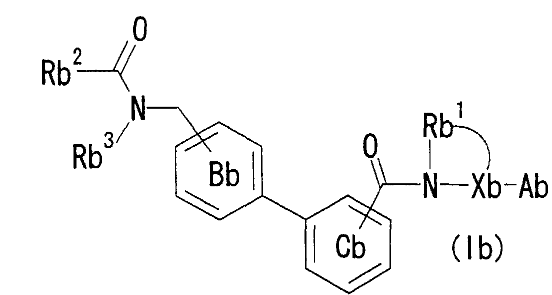 wherein Rb¹ is a hydrogen atom or a hydrocarbon group which may be substituted, Xb is a spacer in which the number of atom(s) constituting a linear chain portion is 1 to 8, Rb¹ and Xb may be bonded to form a ring, Ab is an amino group which may be substituted or a nitrogen-containing heterocyclic group which may be substituted, each of Rb² and Rb³ is a hydrocarbon group which may be substituted, and each of ring Bb and ring Cb is a benzene ring which may be further substituted (provided that 4'-[[(methoxyacetyl)methylamino]methyl]-N-[4-methoxy-3-(4-methyl-1-piperazinyl)phenyl]-2'-methyl-[1,1'-biphenyl]-4-carboxamide is excluded), or a salt thereof.

16. The agent according to claim 15, wherein Xb is a chain spacer.

17. The agent according to claim 1, wherein the compound having urotensin II receptor antagonistic activity or a salt thereof is a compound represented by the formula (Ic): 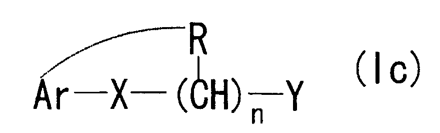 wherein Ar is an aryl group which may be substituted, X is a spacer in which the number of atom(s) constituting a linear chain portion is 1 to 4, n is an integer of 1 to 10, R is a hydrogen atom or a hydrocarbon group which may be substituted, and may be the same or different in the repetition of n, R may be bonded to Ar or the substituent of Ar to form a ring, and Y is an amino group which may be substituted or a nitrogen-containing heterocyclic group which may be substituted, or a salt thereof.

18. The agent according to claim 17, wherein X is a spacer other than -CO-.

19. The agent according to claim 1, wherein the compound having urotensin II receptor antagonistic activity or a salt thereof is a compound represented by the formula (IIc): 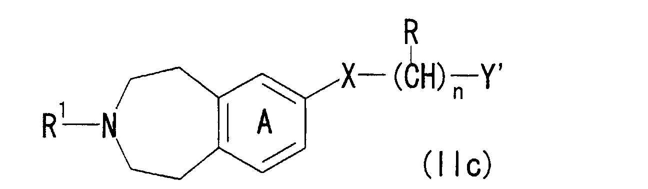 wherein R¹ is a hydrogen atom or a hydrocarbon group which may be substituted or an acyl group which may be substituted, ring A is a benzene ring which may be further substituted, X is a chain spacer in which the number of atom(s) constituting a linear chain portion is 1 to 4 (provided that -CO- is excluded), n is an integer of 1 to 10, R is a hydrogen atom or a hydrocarbon group which may be substituted, and may be the same or different in the repetition of n, R may be bonded to ring A or the substituent of ring A to form a ring, and Y is an amino group which may be substituted, or a salt thereof.

20. A method for preventing or treating central nervous system diseases in a mammal which comprises administering an effective amount of a compound having urotensin II receptor antagonistic activity or a salt thereof to the mammal in need of the prevention or treatment of central nervous system diseases.

21. The method according to claim 20, wherein the compound having urotensin II receptor antagonistic activity or a salt thereof is a compound having amyloid β40 secretion inhibitory activity or a salt thereof.

22. The method according to claim 20, wherein (1) neurodegenerative diseases, (2) neuropathy at cerebrovascular disorder, cephal injury and myelo injury, sequelae of encephalitis or cerebral paralysis, (3) dysmnesia, or (4) mental diseases are prevented or treated.

23. The method according to claim 20, wherein the compound having urotensin II receptor antagonistic activity or a salt thereof is a non-peptide compound or a salt thereof.

24. The method according to claim 20, wherein the compound having urotensin II receptor antagonistic activity is a quinoline derivative.

25. The method according to claim 20, wherein the compound having urotensin II receptor antagonistic activity is a 4-aminoquinoline derivative.

26. The method according to claim 20, wherein the compound having urotensin II receptor antagonistic activity or a salt thereof is a compound represented by the formula (Ia): 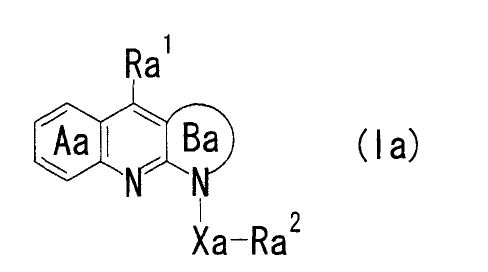 wherein Aa is a benzene ring which may be substituted, Ba is a 5- to 8-membered ring which may be substituted, Xa is a divalent group in which the number of atom(s) in a linear chain portion is 1 to 4, Ra¹ is an amino group which may be substituted, and Ra² is a cyclic group which may be substituted, or a salt thereof.

27. The method according to claim 26, wherein Aa is substituted with a hydrocarbon group which may be substituted.

28. The method according to claim 20, wherein the compound having urotensin II receptor antagonistic activity or a salt thereof is a compound represented by the formula (IIa): 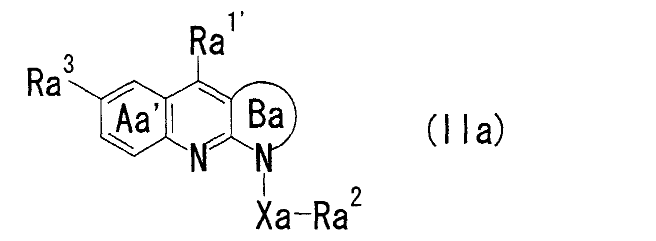 wherein Aa' is a benzene ring which may be further substituted in addition to a substituent Ra³, Ba is a 5- to 8-membered ring which may be substituted, Xa is a divalent group in which the number of atom(s) in a linear chain portion is 1 to 4, Ra^{1'} is a substituted amino group, Ra² is a cyclic group which may be substituted, Ra³ is a hydrocarbon group which may be substituted, a heterocyclic group which may be substituted, nitro group, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted), or a salt thereof.

29. The method according to claim 20, wherein the compound having urotensin II receptor antagonistic activity or a salt thereof is a compound represented by the formula (IIa'): 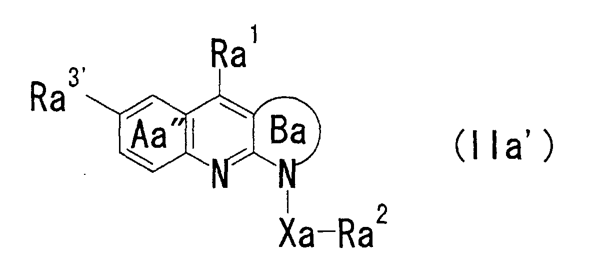 wherein Aa'' is a benzene ring which may be further substituted in addition to the substituent Ra^{3'}, Ba is a 5-to 8-membered ring which may be substituted, Xa is a divalent group in which the number of atom(s) in a linear chain portion is 1 to 4, Ra¹ is an amino group which may be substituted, Ra² is a cyclic group which may be substituted, Ra^{3'} is a hydrocarbon group which may be substituted, a heterocyclic group which may be substituted, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted), or a salt thereof.

30. The method according to claim 29, wherein R^{3'} is a hydrocarbon group which may be substituted.

31. The method according to claim 30, wherein R^{3'} is alkyl.

32. The method according to claim 30, wherein Ra¹ is amino.

33. The method according to claim 20, wherein the compound having urotensin II receptor antagonistic activity or a salt thereof is a compound represented by the formula (Ib) : 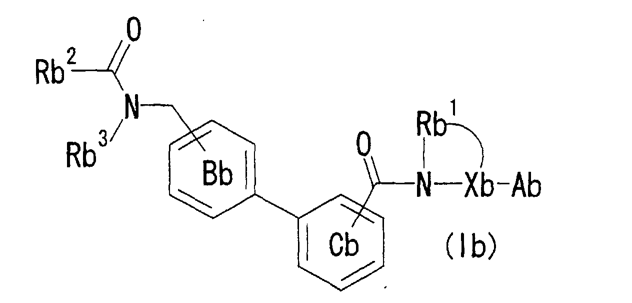 wherein Rb¹ is a hydrogen atom or a hydrocarbon group which may be substituted, Xb is a spacer in which the number of atom(s) constituting a linear chain portion is 1 to 8, Rb¹ and Xb may be bonded to form a ring, Ab is an amino group which may be substituted or a nitrogen-containing heterocyclic group which may be substituted, each of Rb² and Rb³ is a hydrocarbon group which may be substituted, and each of ring Bb and ring Cb is a benzene ring which may be further substituted (provided that 4'-[[(methoxyacetyl)methylamino]methyl]-N-[4-methoxy-3-(4-methyl-1-piperazinyl)phenyl]-2'-methyl-[1,1'-biphenyl]-4-carboxamide is excluded)], or a salt thereof,

34. The method according to claim 33, wherein Xb is a chain spacer.

35. The method according to claim 20, wherein the compound having urotensin II receptor antagonistic activity or a salt thereof is a compound represented by the formula (Ic): 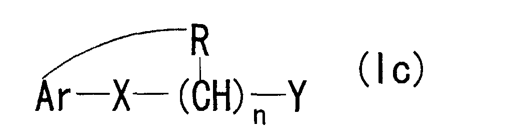 wherein Ar is an aryl group which may be substituted, X is a spacer in which the number of atom(s) constituting a linear chain portion is 1 to 4, n is integer of 1 to 10, R is a hydrogen atom or a hydrocarbon group which may be substituted, and may be the same or different in the repetition of n, R may be bonded to Ar or the substituent of Ar to form a ring, and Y is an amino group which may be substituted or a nitrogen-containing heterocyclic group which may be substituted, or a salt thereof.

36. The method according to claim 35, wherein X is a spacer other than -CO-.

37. The method according to claim 20, wherein the compound having urotensin II receptor antagonistic activity or a salt thereof is a compound represented by the formula (IIc): 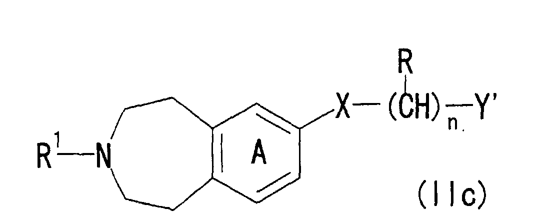 wherein R¹ is a hydrogen atom or a hydrocarbon group which may be substituted or an acyl group which may be substituted, ring A indicates benzene ring which may be further substituted, X is a chain spacer in which the number of atom(s) constituting a linear chain portion is 1 to 4 (provided that -CO- is excluded), n is an integer of 1 to 10, R is a hydrogen atom or a hydrocarbon group which may be substituted, and may be the same or different in the repetition of n, R may be bonded to ring A or the substituent of ring A to form a ring, and Y is an amino group which may be substituted, or a salt thereof.

38. Use of a compound having urotensin II receptor antagonistic activity or a salt thereof for manufacturing a prophylactic or therapeutic agent for central nervous system diseases.

39. The use according to claim 38, wherein the compound having urotensin II receptor antagonistic activity or a salt thereof is a compound having amyloid β40 secretion inhibitory activity or a salt thereof.

40. The use according to claim 38 for manufacturing a prophylactic or therapeutic agent of (1) neurodegenerative diseases, (2) neuropathy at cerebrovascular disorder, cephal injury and myelo injury, sequelae of encephalitis or cerebral paralysis, (3) dysmnesia, or (4) mental diseases.

41. The use according to claim 38, wherein the compound having urotensin II receptor antagonistic activity or a salt thereof is a non-peptide compound or a salt thereof.

42. The use according to claim 38, wherein the compound having urotensin II receptor antagonistic activity is a quinoline derivative.

43. The use according to claim 38, wherein the compound having urotensin II receptor antagonistic activity is a 4-aminoquinoline derivative.

44. The use according to claim 38, wherein the compound having urotensin II receptor antagonistic activity or a salt thereof is a compound represented by the formula (Ia): 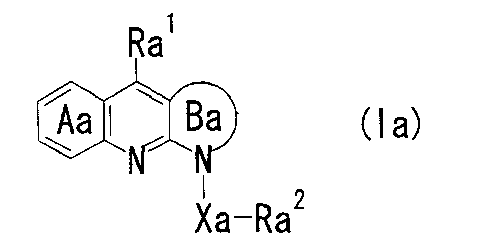 wherein Aa is a benzene ring which may be substituted, Ba is a 5- to 8-membered ring which may be substituted, Xa is a divalent group in which the number of atom(s) in a linear chain portion is 1 to 4, Ra¹ is an amino group which may be substituted, and Ra² is a cyclic group which may be substituted, or a salt thereof.

45. The use according to claim 44, wherein Aa is substituted with a hydrocarbon group which may be substituted.

46. The use according to claim 38, wherein the compound having urotensin II receptor antagonistic activity or a salt thereof is a compound represented by the formula (IIa): 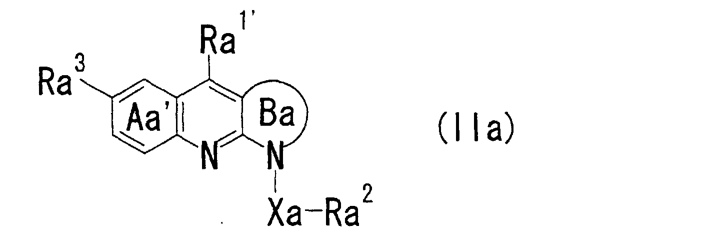 wherein Aa' is a benzene ring which may be further substituted in addition to the substituent Ra³, Ba is a 5-to 8-membered ring which may be substituted, Xa is a divalent group in which the number of atom(s) in a linear chain portion is 1 to 4, Ra^{1'} is a substituted amino group, Ra² is a cyclic group which may be substituted, Ra³ is a hydrocarbon group which may be substituted, a heterocyclic group which may be substituted, nitro group, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted), or a salt thereof.

47. The use according to claim 38, wherein the compound having urotensin II receptor antagonistic activity or a salt thereof is a compound represented by the formula (IIa'): 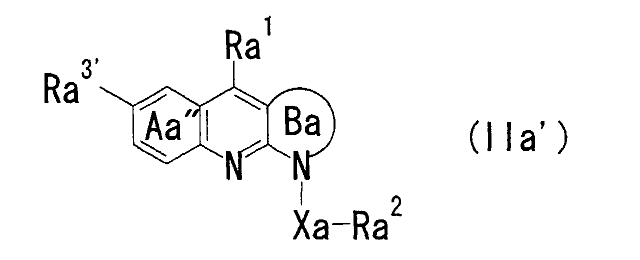 wherein Aa'' is a benzene ring which may be further substituted in addition to the substituent Ra^{3'}, Ba is a 5-to 8-membered ring which may be substituted, Xa is a divalent group in which the number of atom(s) in a linear chain portion is 1 to 4, Ra¹ is an amino group which may be substituted, Ra² is a cyclic group which may be substituted, Ra^{3'} is a hydrocarbon group which may be substituted, a heterocyclic group which may be substituted, a halogen atom, amino group which may be substituted, or a group represented by Ra⁴-Ya- (wherein Ya is oxygen atom or sulfur atom which may be oxidized, and Ra⁴ is a hydrocarbon group which may be substituted, or a heterocyclic group which may be substituted), or a salt thereof.

48. The use according to claim 47, wherein R^{3'} is a hydrocarbon group which may be substituted.

49. The use according to claim 47, wherein R^{3'} is alkyl.

50. The use according to claim 47, wherein Ra¹ is amino.

51. The use according to claim 38, wherein the compound having urotensin II receptor antagonistic activity or a salt thereof is a compound represented by the formula (Ib) : 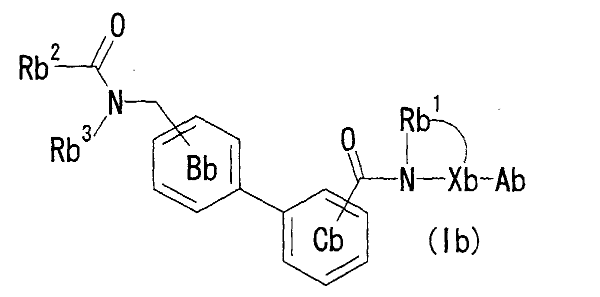 wherein Rb¹ is a hydrogen atom or a hydrocarbon group which may be substituted, Xb is a spacer in which the number of atom(s) constituting a linear chain portion is 1 to 8, Rb¹ and Xb may be bonded to form a ring, Ab is an amino group which may be substituted or a nitrogen-containing heterocyclic group which may be substituted, each of Rb² and Rb³ is a hydrocarbon group which may be substituted, and each of ring Bb and ring Cb is a benzene ring which may be further substituted, (provided that 4'-[[(methoxyacetyl)methylamino]methyl]-N-[4-methoxy-3-(4-methyl-1-piperazinyl)phenyl]-2'-methyl-[1,1'-biphenyl]-4-carboxamide is excluded), or a salt thereof.

52. The use according to claim 51, wherein Xb is a chain spacer.

53. The use according to claim 38, wherein the compound having urotensin II receptor antagonistic activity or a salt thereof is a compound represented by the formula (Ic): 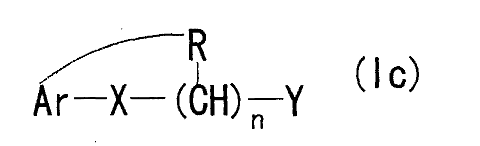 wherein Ar is an aryl group which may be substituted, X is a spacer in which the number of atom(s) constituting a linear chain portion is 1 to 4, n vindicates an integer of 1 to 10, R is a hydrogen atom or a hydrocarbon group which may be substituted, and may be the same or different in the repetition of n, R may be bonded to Ar or the substituent of Ar to form a ring, and Y is an amino group which may be substituted or a nitrogen-containing heterocyclic group which may be substituted, or a salt thereof.

54. The use according to claim 53, wherein X is a spacer other than -CO-.

55. The use according to claim 38, wherein the compound having urotensin II receptor antagonistic activity or a salt thereof is a compound represented by the formula (IIc): 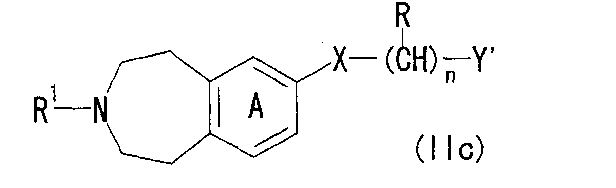 wherein R¹ is a hydrogen atom or a hydrocarbon group which may be substituted or an acyl group which may be substituted, ring A is a benzene ring which may be further substituted, X is a chain spacer in which the number of atom(s) constituting a linear chain portion is 1 to 4 (provided that -CO- is excluded), n indicates an integer of 1 to 10, R is a hydrogen atom or a hydrocarbon group which may be substituted, and may be the same or different in the repetition of n, R may be bonded to ring A or the substituent of ring A to form a ring, and Y is an amino group which may be substituted, or a salt thereof.
